(19) **Europäisches Patentamt / European Patent Office / Office européen des brevets**

(11) **EP 4 473 288 B1**

(12) **EUROPEAN PATENT SPECIFICATION**

(45) Date of publication and mention of the grant of the patent:
**29.10.2025 Bulletin 2025/44**

(21) Application number: **23708082.5**

(22) Date of filing: **31.01.2023**

(51) International Patent Classification (IPC):
**G01N 15/1031** (2024.01)   **G01N 15/1429** (2024.01)
**G01N 15/14** (2024.01)   **G06N 3/02** (2006.01)
**G06N 3/045** (2023.01)   **G06N 3/09** (2023.01)
**G06N 5/01** (2023.01)   **G06N 20/20** (2019.01)
**G01N 15/10** (2024.01)

(52) Cooperative Patent Classification (CPC):
**G01N 15/1429; G01N 15/1031; G01N 15/1459; G01N 15/147; G06N 3/045; G06N 3/09; G06N 5/01; G06N 20/20;** G01N 2015/1006

(86) International application number:
**PCT/US2023/012003**

(87) International publication number:
**WO 2023/147177 (03.08.2023 Gazette 2023/31)**

(54) **MACHINE LEARNING TECHNIQUES FOR CYTOMETRY**

MASCHINENLERNVERFAHREN FÜR ZYTOMETRIE

TECHNIQUES D'APPRENTISSAGE AUTOMATIQUE POUR CYTOMÉTRIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.01.2022 US 202263304990 P**

(43) Date of publication of application:
**11.12.2024 Bulletin 2024/50**

(73) Proprietor: **BostonGene Corporation**
**Waltham, MA 02453 (US)**

(72) Inventors:
- **ZAITSEV, Aleksandr**
  **Yerevan, 42/2 (AM)**
- **FASTOVETS, Dmitrii**
  **Yerevan, 0070 (AM)**
- **BOBE, Anatoly**
  **Yerevan, 0070 (AM)**
- **GOLDBERG, Michael, F.**
  **Brookline, MA 02445 (US)**
- **ATAULLAKHANOV, Ravshan**
  **Moscow, 123104 (RU)**
- **KAMYSHEVA, Anna**
  **Yerevan, 0070 (AM)**
- **VORONINA, Mariia**
  **Promorsky Krai Arsenyev, 692337 (RU)**
- **KOMAROVA, Mariia**
  **Fryazino (RU)**
- **KRAUZ, Ilya**
  **Yerevan, 0070 (AM)**
- **KILINA, Anastasiia**
  **Yerevan, 0070 (AM)**
- **PICHUGIN, Aleksei**
  **Moscow, 119607 (RU)**
- **USHAKOVA, Ekaterina**
  **Moscow, 127543 (RU)**
- **DYIKANOV, Daniiar**
  **Waltham, Massachusetts 02453 (US)**

(74) Representative: **Mewburn Ellis LLP**
**Aurora Building**
**Counterslip**
**Bristol BS1 6BX (GB)**

(56) References cited:
**WO-A1-2020/081582     WO-A1-2021/247868**
**US-A1- 2020 340 909**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

RELATED APPLICATIONS

This application claims benefit under 35 U.S.C. § 119(e) of the filing date of U.S. provisional patent application serial number 63/304,990, filed January 31, 2022, entitled "MACHINE LEARNING TECHNIQUES FOR CYTOMETRY", Attorney Docket No. B1462.70030US00.

BACKGROUND

Cytometry is a laboratory technique used for analyzing single cells or particles in a biological sample. Cytometry is used in a variety of applications such as immunology and molecular biology. Cytometry may be used to measure characteristics of individual cells or particles. Two types of cytometry include flow cytometry and mass cytometry.

Flow cytometry measures the intensity produced by fluorescently labelled markers that are used to label cells in the biological sample. For example, a cell labelled with a marker, or a particular combination of markers may be processed by a flow cytometry platform, which measures fluorescence intensities of the markers for each cell. The measured intensities, or "marker values", of those markers may later be used to determine a type for each cell.

Mass cytometry measures an intensity of heavy metal ion tags used to label cells in the biological sample. For example, a cell labelled with a marker, or a particular combination of markers may be processed by a mass cytometry platform, which measures the relative intensity or abundance of the markers for each cell. The intensities, or "marker values", of those markers may later be used to determine a type for each cell.

When obtaining cytometry data for a biological sample, the biological sample may be partitioned into multiple sub-samples. Each sub-sample may be processed using a different "panel" of markers. A panel of markers is the set of markers used to label cells in the biological sample or sub-sample. Since different markers bind to different cell types or subtypes, using different panels of markers to obtain cytometry data allows for the identification of different cell types in the biological sample.

WO 2021/247868 A1 relates to a computer-implemented method that includes storing image data that includes an input image of a blood sample within a blood monitoring device.

WO 2020/081582 A1 relates to methods for applying artificial neural networks to flow cytometry data generated from biological samples to diagnose and characterize cancer in a subject.

SUMMARY

Provided herein is a method for identifying types of cells present in biological samples using flow cytometry or mass cytometry and multiple machine learning models according to claim 1, the method comprising: using at least one computer hardware processor to perform: obtaining cytometry data for a biological sample previously obtained from a subject, the biological sample comprising a plurality of cells, the cytometry data including cytometry measurements obtained during respective cytometry events, the cytometry events corresponding to particular objects in the biological sample being measured by a flow cytometry platform or a mass cytometry platform, the cytometry events including a subset of events corresponding to cells in the biological sample being measured by the cytometry platform; and identifying types of cells in the plurality of cells using the multiple machine learning models to obtain a respective plurality of cell types, the multiple machine learning models including a first machine learning model and a second machine learning model different from the first machine learning model, the identifying comprising, for each particular event in the subset of events, obtaining, from the cytometry data, cytometry measurements corresponding to the particular event; determining an event type for the particular event by processing the cytometry measurements corresponding to the particular event using the first machine learning model, the event type indicating whether the particular event corresponds to a cell being measured by the cytometry platform, debris being measured by the cytometry platform, or a bead being measured by the cytometry platform; and when the determined event type indicates that the particular event corresponds to the cell being measured by the cytometry platform, determining a type of the cell by processing the cytometry measurements corresponding to the particular event using the second machine learning model.

Some embodiments provide for at least one non-transitory computer-readable storage medium according to claim 14 storing processor-executable instructions that, when executed by at least one computer hardware processor, cause the at least one computer hardware processor to perform a method according to any one of claims 1-13, the method comprising: obtaining cytometry data for a biological sample previously obtained from a subject, the biological sample comprising a plurality of cells, the cytometry data including cytometry measurements obtained during respective cytometry events, the cytometry events corresponding to particular objects in the biological sample being measured by a flow cytometry platform or a mass cytometry platform, the cytometry events including a subset of events corresponding to cells in the biological sample being measured by the cytometry platform; and identifying types of cells in the plurality of cells using the multiple machine learning models to obtain a respective plurality of cell types, the multiple machine learning models including a first machine learning model and a second machine learning model different from the first machine learning model, the identifying comprising, for each particular event in the subset of events, obtaining, from the cytometry data, cytometry measurements corresponding to the particular event; determining an event type for the particular event by processing the cytometry measurements corresponding to the particular event using the first machine learning model, the event type indicating whether the particular event corresponds to a cell being measured by the cytometry platform, debris being measured by the cytometry platform, or a bead being measured by the cytometry platform; and when the determined event type indicates that the particular event corresponds to the cell being measured by the cytometry platform, determining a type of the cell by processing the cytometry measurements corresponding to the particular event using the second machine learning model.

Some embodiments provide for a system according to claim 15 comprising: at least one computer hardware processor; and at least one non-transitory computer-readable storage medium storing processor-executable instructions that, when executed by the at least one computer hardware processor, cause the at least one computer hardware processor to perform a method according to any one of claims 1-13, the method comprising: obtaining cytometry data for a biological sample previously obtained from a subject, the biological sample comprising a plurality of cells, the cytometry data including cytometry measurements obtained during respective cytometry events, the cytometry events corresponding to particular objects in the biological sample being measured by a flow cytometry platform or a mass cytometry platform, the cytometry events including a subset of events corresponding to cells in the biological sample being measured by the cytometry platform; and identifying types of cells in the plurality of cells using the multiple machine learning models to obtain a respective plurality of cell types, the multiple machine learning models including a first machine learning model and a second machine learning model different from the first machine learning model, the identifying comprising, for each particular event in the subset of events, obtaining, from the cytometry data, cytometry measurements corresponding to the particular event; determining an event type for the particular event by processing the cytometry measurements corresponding to the particular event using the first machine learning model, the event type indicating whether the particular event corresponds to a cell being measured by the cytometry platform, debris being measured by the cytometry platform, or a bead being measured by the cytometry platform; and when the determined event type indicates that the particular event corresponds to the cell being measured by the cytometry platform, determining a type of the cell by processing the cytometry measurements corresponding to the particular event using the second machine learning model.

In some embodiments, the subset of events comprises at least 10,000 events.

In some embodiments, the subset of events comprises at least 100,000 events.

In some embodiments, the first machine learning model comprises a first multiclass classifier, and the second machine learning model comprises a second multiclass classifier.

In some embodiments, the first machine learning model comprises a first decision tree classifier, a first gradient boosted decision tree classifier, or a first neural network, and the second machine learning model comprises a second decision tree classifier, a second gradient boosted decision tree classifier, or a second neural network.

Some embodiments further comprise: determining cell composition percentages of different types of cells in the biological sample based on the identified plurality of cell types.

In some embodiments, determining the cell composition percentages comprises: determining a first cell composition percentage for a first type of cell by determining a ratio between a number of cells in the plurality of cells identified as being of the first type and a total number of the cells in the plurality of cells.

In some embodiments, the subject has, is suspected of having, or is at risk of having cancer, and the method further comprises: identifying a treatment for the subject based on the determined cell composition percentages.

In some embodiments, identifying the treatment for the subject based on the determined cell composition percentages comprises: identifying ipilimumab for the subject when a cell composition percentage of peripheral blood mononuclear cells (PBMCs) is below a threshold.

In some embodiments, identifying the treatment for the subject based on the determined cell composition percentages comprises: determining a ratio between a cell composition percentage of CD8+PD-1+ cells and a cell composition percentage of CD4+PD-1; and identifying immune checkpoint blockade therapy for the subject when the determined ratio is above a threshold.

Some embodiments further comprise: comparing a cell composition percentage of the determined cell composition percentages to a range of cell composition percentages associated with a patient cohort; and identifying the subject as a member of the patient cohort based on a result of the comparing.

In some embodiments, the patient cohort comprises a healthy cohort, a cohort of patients with a disease, or a cohort of patients who have received a treatment.

Some embodiments further comprise: comparing a cell composition percentage of the determined cell composition percentages to a range of cell composition percentages associated with a study, wherein the study evaluates effectiveness of one or more treatments in treating a disease; and identifying a treatment for the subject based on a result of the comparing.

In some embodiments, the subset of events corresponding to the cells in the biological sample being measured by the cytometry platform comprises a first subset of events, and the cytometry events further include: a second subset of events corresponding to beads in the biological sample being measured by the cytometry platform, and a third subset of events corresponding to debris in the biological sample being measured by the cytometry platform.

In some embodiments, the flow cytometry measurements corresponding to the particular event comprise fluorescence intensity values for at least some of a plurality of markers.

In some embodiments, the plurality of events includes a first plurality of events and a second plurality of events, and the cytometry data comprises first cytometry data for the first plurality of events and second cytometry data for the second plurality of events, the first cytometry data comprising measurements obtained for first markers of a plurality of markers during each of at least some of the first plurality of events and the second cytometry data comprising measurements obtained for second markers of the plurality of markers during each of at least some of the second plurality of events, wherein the first markers of the plurality of markers and the second markers of the plurality of markers are different.

In some embodiments, the first cytometry data comprises data from a first panel, and the second cytometry data comprises data from a second panel different from the first panel.

BRIEF DESCRIPTION OF DRAWINGS

FIG. 1A is a diagram depicting an illustrative technique for determining a respective type for one or more events based on cytometry data, according to some embodiments of the technology described herein.

FIG. 1B is a table showing example cytometry data for multiple events corresponding to cells and/or particles in biological sample, according to some embodiments of the technology described herein.

FIG. 1C is a table showing example cytometry data, including example marker values, for multiple events, according to some embodiments of the technology described herein.

FIG. 1D is a block diagram of a system 150 including example computing device 108 and software 112, according to some embodiments of the technology described herein.

FIG. 1E is a block diagram of a system 180 for processing cytometry data 132-1 for a cell to determine one or more types for the cell, according to some embodiments of the technology described herein.

FIG. 2A is a flowchart of an illustrative process 200 for determining a respective type for one or more cells using cytometry data and a hierarchy of machine learning models, according to some examples of the technology described herein.

FIG. 2B is a flowchart depicting an example implementation of act 206a of process 200 for determining a first type for a first cell, according to some examples of the technology described herein.

FIG. 2C is a flowchart of an illustrative process 250 for determining types for events corresponding to objects in a biological sample being measured by a cytometry platform, according to some embodiments of the technology described herein.

FIG. 3A is an example diagram for determining a type for an event based on an output of binary class classifiers, according to some embodiments of the technology described herein.

FIG. 3B is an example diagram for determining a type for an event based on an output of a multiclass classifier, according to some embodiments of the technology described herein.

FIG. 4 depicts an example hierarchy 400 of cell/particle types and shows an illustrative example for determining one or more types for a cell/particle based on the hierarchy 400, according to some embodiments of the technology described herein.

FIG. 5A is a flowchart of an illustrative process 500 for identifying a subject as a member of a patient cohort, according to some embodiments of the technology described herein.

FIG. 5B is a flowchart depicting an example implementation of act 506 of process 500 for determining cell composition percentages based on estimate cell composition percentages of a common cell type, according to some embodiments of the technology described herein.

FIG. 5C is a flowchart depicting an example implementation of act 506 of process 500 for determining cell composition percentages based on percentages of beads, according to some embodiments of the technology described herein.

FIG. 5D is a flowchart depicting an example implementation of act 508 of process 500 for normalizing cell composition percentages with respect to hierarchical relationships between cell types, according to some examples of the technology described herein.

FIG. 6A depicts an illustrative example for determining cell composition percentages based on cell types determined using cytometry from a single panel, according to some embodiments of the technology described herein.

FIG. 6B and FIG. 6C depict an illustrative example for determining cell composition percentages based on cell types determined using cytometry data from different panels, according to some embodiments of the technology described herein.

FIG. 7A, FIG. 7B, and FIG. 7C are example hierarchical visualizations of cell composition percentages, according to some embodiments of the technology described herein.

FIG. 7D is a screenshot of an example report showing the evaluation of a biomarker based on determined cell composition percentages, according to some embodiments.

FIG. 7E is a screenshot of an example report indicating cell composition percentages in a biological sample from a patient, according to some embodiments of the technology described herein.

FIG. 7F and FIG. 7G are screenshots of an example report showing the deviation of cell composition percentage values relative to the normal ranges of cell composition percentage values associated with reference cohorts, according to some embodiments of the technology described herein.

FIG. 8 is a flowchart depicting an exemplary process 800 for training a plurality of machine learning models to determine whether a cell is of a particular type, according to some examples of the technology described herein.

FIG. 9A shows the results of clustering cytometry data that has not undergone a noise transformation.

FIG. 9B shows the results of clustering cytometry data that has undergone the noise transformation.

FIG. 9C shows the distribution of marker intensities resulting from cytometry data that has not undergone the noise transformation.

FIG. 9D shows the distribution of marker intensities resulting from cytometry data that has undergone the noise transformation according to some example of the technology described herein.

FIG. 9E are plots used by conventional techniques for manually identifying different types of events (e.g., cells or particles) based on marker values.

FIG. 9F is a plot showing event clusters that were manually labelled to indicate the event type.

FIG. 10 depicts an illustrative implementation of a computer system that may be used in connection with some embodiments of the technology described herein.

DETAILED DESCRIPTION

There are many different cell populations in the human immune system, many of which play an important role in fighting disease and protecting the body from foreign substances. In some instances, the immune system may also include diseased cell populations (e.g., diseased B cells). For example, if a mutation occurs in a cell, it may cause one or more cell populations to grow uncontrollably (e.g., thereby forming a cancerous cell population).

Understanding the cellular makeup of the immune system is useful in making diagnoses, developing treatment strategies, and conducting research. For example, knowledge about the proportions of certain cell populations may be used to diagnose a subject with a disease or predict whether a subject will respond to a particular treatment. For example, CD4+ and CD8+ cells are both favorable in patients who are treated with Rituximab.

Cytometry is a tool that can be used for identifying types for individual cells in a biological sample. Conventional techniques include the manual and tedious analysis of data generated during cytometry. In particular, such techniques include plotting the cytometry data in a series of two-dimensional plots and manually identifying regions of interest in each plot, commonly referred to as "gating". To identify such regions of interest, an operator defines boundaries around groups of plotted points. Cells are identified as being of a particular cell type when they have marker values that fall within a region of interest and/or within a combination of regions of interest.

The inventors have recognized that there are a number of problems with conventional techniques for identifying cell types using cytometry. One such problem is that identifying a region of interest is a subjective procedure that results in issues of reproducibility. In particular, different operators make different decisions about where to place boundaries. For example, some operators may place more expansive boundaries, including more points, while other operators may place more restrictive boundaries, including fewer points. As a result, there are variances in the data used to determine types for individual cells, leading to variances in the results of such analyses. This issue becomes more pronounced when analyzing large volumes of cytometry data, since this involves generating and identifying regions of interest in a larger number of plots, leading to greater overall variation in the data used for cell type determination. As a result, different operators may classify the same cell differently (e.g., one operator may classify a cell as being of one type and another operator may classify the cell as being a different type). Consequently, different operators will produce different estimates of cell population percentages (e.g., estimates of the proportion of each of one or more cell types in the overall cell population).

Another problem with conventional techniques for identifying cell types using cytometry is that the manual analysis of such data is very inefficient. This poses challenges for large-scale studies, such as patient cohort and drug screening studies, which generate complex, multidimensional datasets. Manually processing such datasets is extremely time-consuming, leading to high costs, which in turn affects the quality of the data processing results or the study overall. For example, low quality data may result from an analysis of a two-dimensional cytometry data plot, where boundaries around plotted marker values have not been carefully defined. Determining a type for a cell based on such data will result in inaccuracies, especially in closely related cell types which share highly similar marker values and are thus at a greater risk of being grouped together by boundaries that do not carefully distinguish them from each other.

Inaccurately and unreliably determining cell types for a sample, using conventional cytometry techniques, also affects the accuracy and reliability of estimating the cellular composition of the sample. The cellular composition of a sample may be estimated based on the relative number of cells of each cell type in the sample. When based upon cell types that are inaccurate, the estimated cellular composition will not accurately reflect the relative number of cells in each cell population. As explained above, the cellular composition may be used to diagnose a subject with a disease or predict whether the subject will respond to a particular treatment. When the estimated cellular composition is inaccurate, the conventional techniques may diagnose the subject with the wrong disease, or incorrectly predict how the subject will respond to a treatment. For example, a relatively large cell composition percentage of CD4+ cells may indicate that a patient will respond well to Rituximab. If many cells of a patient's sample are incorrectly determined to be CD4+ cells, then the conventional technique may incorrectly predict that the patient will respond well to Rituximab when, in fact, they may respond negatively or not at all.

Furthermore, when there are variations in data used for cell type determination, there will also be variations in cellular composition estimates. For example, when different operators determine different types for the same cell, there will be differences in the resulting number of cells estimated for each cell population. Such variations make it challenging to compare data collected from different studies, since the same sample may be estimated by different operators to have different cellular compositions. As a result, it is challenging to extract meaningful insights from the data, such as correlations between cellular composition and therapeutic response, diagnosis, and other clinically relevant results. For example, a clinician may receive conflicting cell composition estimates from different cytometry operators, making it challenging to diagnose the patient or select a treatment based on such estimates.

The inventors have developed techniques for more accurately, reliably, and efficiently determining types for cells included in a biological sample based on cytometry data that address the above-described problems of conventional techniques. The techniques include processing cytometry data using multiple machine learning models to identify types of cells present in a biological sample. In some embodiments, the cytometry data includes cytometry measurements (e.g., marker values) obtained during respective cytometry events ("events").

An "event" corresponds to an object (e.g., a cell, debris, a bead, a doublet, or an unidentified object) in a biological sample being measured by a cytometry platform (e.g., a flow cytometry platform or a mass cytometry platform). For example, an event may correspond to a cell in the biological sample being measured by a cytometry platform, and the measurements obtained during the event may be included in the cytometry data. In some embodiments, the multiple machine learning models used to process the cytometry data include a first machine learning model and a second machine learning model different from the first machine learning model. In some embodiments, the first machine learning model is used to process cytometry measurements corresponding to a particular event to determine an event type for the particular event. The event type may indicate whether an event corresponds to a cell being measured by the cytometry platform, debris being measured by the cytometry platform, or a bead being measured by the cytometry platform. For example, the first machine learning model may include a multiclass classifier trained to distinguish between at least some event types. In some embodiments, when the determined event type indicates that the particular event corresponds to the cell being measured by the cytometry platform, the second machine learning model is used to process the cytometry measurements corresponding to the particular event to determine a type of cell for the particular event. For example, the second machine learning model may include a multiclass classifier trained to distinguish between at least some event cell types.

In some examples, the techniques include processing cytometry data for cells (e.g., a type of event) in the biological sample using a hierarchy of machine learning models corresponding to a hierarchy of cell types. A machine learning model in the hierarchy of machine learning models may be trained to predict a particular type for a cell using the cytometry data corresponding to the cell. Additionally or alternatively, a machine learning model in the hierarchy of machine learning models may include a multiclass classifier trained to distinguish between at least some cell types at a particular level in the hierarchy. Different levels of the hierarchy of machine learning models may be used to predict a type for a cell with different levels of specificity (e.g., a general cell type or a specific subtype).

Such techniques improve cytometry by improving the accuracy and reproducibility of the cell type determination results. In particular, at least one machine learning model is specifically trained to predict a type for a cell in a biological sample based on cytometry data. In some cases, the machine learning model determines a confidence that the cell is of the particular type and will not identify the type for the cell when the confidence does not exceed a threshold. This prevents inaccurate cell type identification of cells that may falsely appear to be of the particular type (e.g., having marker values that are similar to those of other cells of that type). Using one or more machine learning models in this way eliminates the subjective processes of conventional techniques, including the processes that rely on a human operator to identify points to be included in or excluded from a region of interest. As described above, such conventional techniques can produce overinclusive or underinclusive results, leading to inaccurate, inconsistent identification of cell types. By contrast, the systems and methods described herein, through the use of the machine learning models, produce more accurate and reproducible results. Accurate and reproducible cell type identification is important to applications of cytometry where cell count and/or cell composition percentages are used to inform diagnosis and/or have treatment implications.

Furthermore, the systems and methods described herein include techniques for identifying particles in the biological sample, contributing to the accuracy of the cell type determination results and to improvements to cytometry. In particular, at least one machine learning model (e.g., in the hierarchy of machine learning models) is trained to determine a type for a particle (e.g., a bead, doublet, or debris) in the biological sample. Rather than discarding all particles from analysis, or treating them as a whole, the particles can be distinguished from one another and used to produce more accurate results. For example, particles that are identified as doublets can be split and analyzed as single cells for further analysis, improving the accuracy of total cell counts and counts of cells of a particular type. Additionally, or alternatively, particles that are identified as beads can be used to determine a percentage of the beads included in the biological sample, which can be used to improve the analysis of cells included in the biological sample (e.g., by informing the estimation of cell composition percentages).

Furthermore, using the hierarchy of machine learning models improves the specificity of the cell type determination results. In particular, different levels of the hierarchy are used to determine a type for a cell at different levels of specificity. Machine learning models at each level are trained to distinguish between cell types at the same level, allowing for the identification of the minute differences in cytometry data that are not readily apparent with conventional techniques. Cell type specificity is important to applications of cytometry where the presence of a particular cell type, and the degree of the presence of cell type relative to other cell types, is a predictive and/or prognostic biomarker.

Accordingly, provided herein are computer-implemented techniques for determining types of cells present in biological samples using cytometry and multiple machine learning models. The techniques include obtaining cytometry data (e.g., flow cytometry data or mass cytometry data) for a biological sample (blood, saliva, a biopsy, etc.) previously-obtained from a subject e.g., a subject having, suspected of having, or at risk of having cancer (for example, lymphoma) or an immune-related disease (for example, rheumatoid arthritis). The biological sample includes a plurality of cells. The cytometry data may include cytometry measurements (e.g., marker values, fluorescence intensity values, intensity of heavy metal ion tags, etc.) obtained during respective cytometry events ("events"). The events correspond to particular objects (e.g., cells, debris, or beads) in the biological sample being measured by a cytometry platform. The cytometry events include a subset of events corresponding to cells in the biological sample being measured by the cytometry platform. For example, the subset of events may include one, some, or all of the cytometry events.

The techniques include identifying types of cells in the plurality of cells using multiple machine learning models including a first machine learning model and a second machine learning model different from the first machine learning model. In some embodiments, the identifying is performed for each particular event in the subset of events. The identifying includes, for each particular event, obtaining cytometry measurements corresponding to the particular event. For example, the cytometry measurements may be obtained from the cytometry data obtained for the biological sample. The identifying further includes determining an event type for the particular event by processing the cytometry measurements corresponding to the particular event using the first machine learning model. The event type indicates whether the particular event corresponds to a particular object (e.g., a cell, debris, or bead) being measured by the cytometry platform. When the determined event type indicates that the particular event corresponds to a cell being measured by the cytometry platform, the identifying further includes determining a type of the cell (e.g., one or more of the cell types listed in Table 1). This includes processing the cytometry measurements corresponding to the particular event using the second machine learning model.

In some embodiments, the subset of events comprises at least 5,000 events, at least 10,000 events, at least 20,000 events, at least 50,000 events, at least 100,000 events, at least 500,000 events, at least 600,000 events, at least 900,000 events, between 500 events and 1 million events, between 5,000 events and 900,000 events, or between 20,000 events and 700,000 events.

In some embodiments, the first machine learning model comprises a first multiclass classifier, and the second machine learning model comprises a second multiclass classifier. Additionally, or alternatively, the first machine learning model and/or the second machine learning model may include one or more binary class classifiers. In some embodiments, the first machine learning model comprises a first decision tree classifier, a first gradient boosted decision tree classifier, or a first neural network, and the second machine learning model comprises a second decision tree classifier, a second gradient boosted decision tree classifier, or a second neural network.

Some embodiments further comprise: determining cell composition percentages of different types of cells in the biological sample based on the identified plurality of cell types. For example, this may include determining a cell composition percentage for one or more cell types (or subtypes) listed in Table 1. In some embodiments, determining the cell composition percentages comprises: determining a first cell composition percentage for a first type of cell by determining a ratio between a number of cells in the plurality of cells identified as being of the first type and a total number of the cells in the plurality of cells.

In some embodiments, the determined cell composition percentages are used to identify a treatment for the subject when the subject is suspected of having, or is at risk of having cancer. For example, identifying the treatment for the subject based on the determined cell composition percentages may comprise: determining a ratio between a cell composition percentage of CD8+PD-1+ cells and a cell composition percentage of CD4+PD-1, and identifying immune checkpoint blockade therapy for the subject when the determined ratio is above a threshold. As another example, identifying the treatment for the subject based on the determined cell composition percentages may include: identifying ipilimumab for the subject when a cell composition percentage of peripheral blood mononuclear cells (PBMCs) is below a threshold. The identified treatment may be administered the subject (e.g., used to treat the subject).

In some embodiments, the determined cell composition percentages are used to identify a subject as a member of a cohort. For example, the patient cohort may comprise healthy cohort, a cohort of patients with a disease (e.g., cancer or immune-related disease), or a cohort of patients who have received a treatment. Identifying the subject as a member of a cohort includes, in some embodiments, comparing a cell composition percentage of the determined cell composition percentages to a range of cell composition percentages associated with a patient cohort; and identifying the subject as a member of the patient cohort based on a result of the comparing. For example, the range of cell composition percentages may be obtained from a data store and/or from one or more other subjects for which cell composition percentages have been determined.

In some embodiments, the cytometry events additionally include a second subset of events corresponding to beads in the biological sample being measured by the cytometry platform, and a third subset of events corresponding to debris in the biological sample being measured by the cytometry platform. In some embodiments, the techniques include processing cytometry measurements corresponding to one or more events in the second and/or third subset of events to determine an event type the one or more events. For example, this may include processing the cytometry measurements for the one or more events using the first machine learning model to determine the event type for the one or more events.

In some embodiments, the plurality of events includes a first plurality of events and a second plurality of events. The cytometry data may include first cytometry data for the first plurality of events (e.g., corresponding to objects in a first sub-sample of the biological sample being measured with a cytometry platform) and second cytometry data for the second plurality of events (e.g., corresponding to objects in a first sub-sample of the biological sample being measured with a cytometry platform). The first cytometry data may include values (e.g., fluorescence intensity values, relative intensity of heavy metal ion tags) for a first subset (e.g., a first panel) of a plurality of markers for each of at least some of the first plurality of event and the second cytometry data may include values (e.g., fluorescence intensity values, relative intensity of heavy metal ion tags) for a second subset (e.g., a second panel) of the plurality of markers for each of at least some of the second plurality of events. In some embodiments, the first and second subsets of the plurality of markers are different. For example, the first subset of the plurality of markers may include none, some, half, or most of the markers included in the second subset of the plurality of markers. In some embodiments, the first cytometry data comprises data from a first panel, and the second cytometry data comprises data from a second panel different from the first panel.

Also described herein are computer-implemented techniques for determining types for cells in a biological sample using cytometry data and a hierarchy of machine learning models. In some cases, the techniques include obtaining cytometry data for a biological sample (e.g., blood, saliva, a biopsy, etc.) from a subject (e.g., a subject having, suspected of having, or at risk of having cancer (for example, lymphoma) or an immune-related disease (for example, rheumatoid arthritis)). The biological sample may include a plurality of cells including a first cell, and the cytometry data may include first cytometry data (e.g., one or more marker values) for the first cell. In some cases, the techniques include determining a respective type for each of at least some (e.g., at least 10%, at least 30%, at least 50%, at least 70%, at least 90%, etc.) of the plurality of cells using a hierarchy of machine learning models (e.g., decision tree classifiers, gradient boosted decision tree classifiers, etc.), corresponding to a hierarchy of cell types (e.g., cell types and cell subtypes). For example, a machine learning model in the hierarchy of machine learning models may be trained to determine whether a cell is of a particular type, corresponding to the hierarchy of cells. In some cases, the determining comprises determining a first type for the first cell by processing the first cytometry data using a first subset (e.g., a first plurality) of the hierarchy of machine learning models. For example, the first subset of machine learning models may correspond to a particular path through the hierarchy.

In some cases, the plurality of cells includes a second cell of a different type than the first cell, and the cytometry data includes second cytometry data (e.g., one or more marker values) for the second cell, In some cases, determining a respective type for a cell further includes processing the second cytometry data using a second subset (e.g., a second plurality) of the hierarchy of machine learning models, the second subset of the hierarchy of machine learning models being different than the first subset of the hierarchy of machine learning models. For example, the second subset of the hierarchy of machine learning models may represent a second path through the hierarchy. In some cases, the first and second subsets of the hierarchy of machine learning models may include one or more of the same machine learning models. Additionally, or alternatively, the first and second subsets may not include any of the same machine learning models.

In some cases, the techniques include determining a respective type for each of at least 5,000 cells, at least 10,000 cells, at least 20,000 cells, at least 50,000 cells, at least 100,000 cells, at least 500,000 cells, at least 600,000 cells, or at least 900,000 cells.

In some cases, processing the first cytometry data using the first subset of the hierarchy of machine learning models includes processing the first cytometry data using a first machine learning model (e.g., a machine learning model trained to determine whether the first cell is of a first type). For example, the first machine learning model may be trained to determine whether the first cell is a lymphocyte. The processing may further include identifying, based on the output of the first machine learning model (e.g., an indication of whether the first cell is of the first type), a second machine learning model (e.g., a machine learning model trained to determine whether the first cell is of a second type) in the first subset of the hierarchy of machine learning models. For example, if the output of the first machine learning model indicates that the first cell is of the first type (e.g., a lymphocyte), then the techniques may include identifying a second machine learning model trained to determine whether the first cell is of a subtype of the first cell type (e.g., a T cell). In some cases, the techniques include processing the first cytometry data using the second machine learning model to obtain a second output (e.g., an indication of whether the first cell is of the second type).

In some cases, the first output indicates a first type for the first cell and the second output indicates a subtype for the first cell. For example, the first type may include a leukocyte, while the subtype of the first type includes a granulocyte. As another example, the first type may include a lymphocyte, while the subtype of the first type includes a B cell. As yet another example, the first cell type may include a T helper cell, while the subtype of the first type may include a memory T helper cell. Examples of cell types and the relationships between cell types are provided herein, including at least in Table 1.

In some cases, processing the first cytometry data using the first subset of machine learning models further includes identifying, based on the first output (e.g., a first type for the first cell) of the first machine learning model, a third machine learning model (e.g., trained to determine whether the cell is of a third type) in the first subset of the hierarchy of machine learning models. For example, identifying the third machine learning model may include identifying a machine learning model trained to determine whether the cell is of a different subtype (e.g., a B cell) of the first cell type (e.g., a lymphocyte). The techniques may further include processing the first cytometry data using the third machine learning model.

In some cases, processing the first cytometry using the first subset of the hierarchy of machine learning models further includes identifying, based on the second output (e.g., a second type for the second cell) of the second machine learning model, a third machine learning model in the first subset of the hierarchy of machine learning models (e.g., trained to determine whether the cell is of a third type). The third machine learning model may be trained to determine whether the cell is of a subtype of the second cell type determined by the second machine learning model. For example, if the second output of the second machine learning model indicates that the cell is a B cell, the third machine learning model may be trained to determine whether the cell is a memory B cell. In some cases, the techniques then include processing the first cytometry data using the third machine learning model.

In some cases, a machine learning model in the hierarchy of machine learning models may include a decision tree classifier, a gradient boosted decision tree classifier, a neural network, or any other suitable type of machine learning model, as aspects of the technology described herein are not limited in this respect. In some cases, a machine learning model in the hierarchy of machine learning models may include an ensemble of any suitable type of machine learning models.

In some cases, the techniques further include determining a respective cell composition percentage for each of at least some of the determined types of cells. This may include determining a first cell composition percentage for the first type of cell. For example, this may include estimating the percentage of B cells in the biological sample. In some cases, determining the first cell composition percentage includes determining a ratio between a number of cells estimated to be of the first type (e.g., based on the cytometry data) and a total number of cells of at least some of the plurality of cells in the biological sample.

In some cases, the techniques include comparing a determined cell composition percentage (e.g., the first cell composition percentage) to a range of cell composition percentages associated with a patient cohort. For example, the patient cohort may include patients that are diagnosed with a disease, patients receiving a particular treatment, healthy patients and/or patients with one or more other conditions or characteristics. Based on a result of the comparing, the techniques may include identifying the subject as a member of the patient cohort. For example, this may include identifying the subject as being healthy, as having a particular disease, as likely to have a response to a particular therapy, and/or of having another condition.

In some cases, the techniques include generating a visualization of the determined cell composition percentages, the visualization indicating the result of comparing the first cell composition percentage to the range of cell composition percentages associated with the patient cohort. For example, the visualization may include a graphic indicating the range of reference cell composition percentages and an indication of where, in the range, the first cell composition percentage falls.

In some cases, the techniques include comparing the first cell composition percentage to a range of cell composition percentages associated with a study. In some cases, the study evaluates effectiveness of one or more treatments in treating a disease and identifying a treatment for the subject based on a result of the comparing. For example, such a treatment may be identified by comparing the determined cell composition percentage to the cell composition percentages for patients with good or poor survival rates after receiving different treatments.

In some cases, the techniques include generating, using the hierarchy of cell types, a visualization of the determined cell composition percentages. The visualization may include a plurality of nodes organized into a hierarchy, with at least some of the plurality of nodes linked by respective edges. The plurality of nodes may include a first node representing a first cell type and a second node representing a subtype of the first cell type, while the edges may include a first edge connecting the first node and the second node.

In some cases, the visualization includes an indication of a subset of cell types of the hierarchy of cell types. In some cases, cell composition percentages determined for the subset of cell types include abnormal cell composition percentages relative to reference cell composition percentages. For example, abnormal cell composition percentages may include those that fall outside a range of cell composition percentages associated with a patient cohort (e.g., of healthy patients, of patients diagnosed with a particular disease, and/or of patients who have received a particular treatment.)

In some cases, the visualization further includes, for the first cell type, a first number indicating a percentage of cells having the first cell type in the biological sample. For example, the first number may be shown proximate the first node. In some embodiments, the size of a node, relative to the sizes of other nodes in the visualization, may represent the relative proportion of cells having a particular cell type in the biological sample. For example, larger nodes may represent a larger percentage of cells having the particular cell type.

In some cases, determining the respective cell composition percentage further includes determining one or more second cell composition percentages for one or more second types of cells that are subtypes of the first type of cell. As a nonlimiting example, this may include determining a first cell composition percentage for lymphocytes and second cell composition percentages for B cells and T cells.

In some cases, the techniques include comparing the sum of the one or more second cell composition percentages (e.g., the sum of the percentages of B cells and T cells) to the first cell composition percentage (e.g., the percentage of lymphocytes). The techniques may include determining a normalization coefficient (e.g., a ratio between the first cell composition percentage and the sum of the one or more second cell composition percentages) based on the result of the comparing and applying the normalization coefficient to the one or more second cell composition percentages. For example, the normalization coefficient may account for differences between the first cell composition percentage and the sum of the one or more second cell composition percentages.

In some cases, the techniques further include determining whether the one or more second types of cells include all subtypes of the first type of cell and applying the normalization coefficient to the one or more second cell composition percentages when the one or more second types of cells comprise all of the subtypes of the first type of cell. As a nonlimiting example, since B cells and T cells do not comprise all subtypes of lymphocytes, the normalization coefficient may not be applied to the cell composition percentages of B cells and T cells.

In some cases, the techniques include comparing a sum of the one or more second cell composition percentages (e.g., the sum of the percentages of B cells and T cells) to the first cell composition percentage (e.g., the percentage of lymphocytes). The techniques may include determining, based on a result of the comparing, a type-specific normalization coefficient for each of the one or more second types of cells. For example, this may include determining a normalization coefficient for B cells and a normalization coefficient for T cells. In some cases, the techniques include applying the type-specific normalization coefficients to the respective one or more cell composition percentages.

In some cases, the obtained flow cytometry data comprises noise-transformed flow cytometry data. For example, any suitable noise transformation technique may be used to reduce noise.

In some cases, the cytometry data includes values (e.g., fluorescence intensity measurements) for each of at least some of a plurality of markers (e.g., fluorescently labelled antibodies or fluorescent dyes or stains, heavy metal ion tags) for each of at least some of the plurality of cells.

In some cases, the plurality of cells includes a first plurality of cells and a second plurality of cells. The cytometry data may include a first subset of cytometry data for the first plurality of cells (e.g., included in a first sub-sample of the biological sample) and a second subset of cytometry data for the second plurality of cells (e.g., included in a second sub-sample of the biological sample). The first subset of cytometry data may include values (e.g., fluorescence intensity values, relative intensity of heavy metal ion tags) for a first subset (e.g., a first panel) of the plurality of markers for each of at least some of the first plurality of cells and the second subset of cytometry data may include values (e.g., fluorescence intensity values, relative intensity of heavy metal ion tags) for a second subset (e.g., a second panel) of the plurality of markers for each of at least some of the second plurality of markers. In some cases, the first and second subsets of the plurality of markers are different. For example, the first subset of the plurality of markers may include none, some, half, or most of the markers included in the second subset of the plurality of markers.

In some cases, determining a respective type for each of at least some of a plurality of cells includes determining a respective first plurality of types for the first plurality of cells and determining a respective second plurality of types for the second plurality of cells. For example, this may include determining types for cells in a first sub-sample of the biological sample and determining types for cells in a second sub-sample of the biological sample. The techniques may include determining, using the first plurality of cell types, a first plurality of cell composition percentages. This may include determining a respective cell composition percentage for each of at least some of the first plurality of cell types. The techniques may further include determining, using the second plurality of cell types, a second plurality of cell composition percentages for each of at least some of the second plurality of cell types.

In some cases, the techniques include determining a respective cell composition percentage for each of at least some of the determined types of cells. For example, this may include determining cell composition percentages for each of at least some of the plurality of cell types based on a total number of cells included in both the first and second pluralities of cells. In some cases, the determining may include combining at least some of the first plurality of cell composition percentages and at least some of the second plurality of cell composition percentages.

In some cases, combining at least some of the first plurality of cell composition percentages and at least some of the second plurality of cell composition percentages may be based on estimated composition percentages of a cell type included in the first plurality of cells and the second plurality of cells. For example, each of at least some of the first plurality of cell composition percentages may be normalized with respect to an estimated composition percentage of a common cell type in the first plurality of cells. Similarly, each of at least some of the first plurality of cell composition percentages may be normalized with respect to an estimated composition percentage of the common cell type in the second plurality of cells.

In some cases, the techniques include combining at least some of the first plurality of cell composition percentages and the at least some of the second plurality of cell composition percentages based on data obtained using beads included in the biological sample. For example, this may include normalizing some of the first plurality of cell composition percentages with respect to a concentration of beads included in the first plurality of cells and normalizing some of the second plurality of cell composition percentages with respect to a concentration of beads included in the second plurality of cells.

In some cases, the biological sample includes a plurality of particles (e.g., debris, doublets, beads, etc.), and the cytometry data includes cytometry data (e.g., marker values) for a first particle of the plurality of particles.

In some cases, the techniques include determining a respective particle type for each of at least some of the plurality of particles using the hierarchy of machine learning models. This may include determining a first particle type for the first particle using the hierarchy of machine learning models. A machine learning model of the hierarchy of machine learning models may be trained to determine whether a particle or a cell is of a particular particle type. For example, a machine learning model may be trained to determine whether a particle or cell is a bead (or debris or doublet). In some embodiments, this may also include determining whether the first particle cannot be identified.

In some cases, obtaining the cytometry data include processing the biological sample using a cytometry platform.

In some cases, the hierarchy of machine learning models includes at least 50 machine learning models, at least 100 machine learning models, at least 200 machine learning models, at least 250 machine learning models, at least 300 machine learning models, at least 350 machine learning models, or at least 400 machine learning models.

Following below are more detailed descriptions of various concepts related to, and embodiments of, the cell type determination systems and methods developed by the inventors. It should be appreciated that various aspects described herein may be implemented in any of numerous ways. Examples of specific implementations are provided herein for illustrative purposes only. In addition, the various aspects described below may be used alone or in any combination and are not limited to the combinations explicitly described herein.

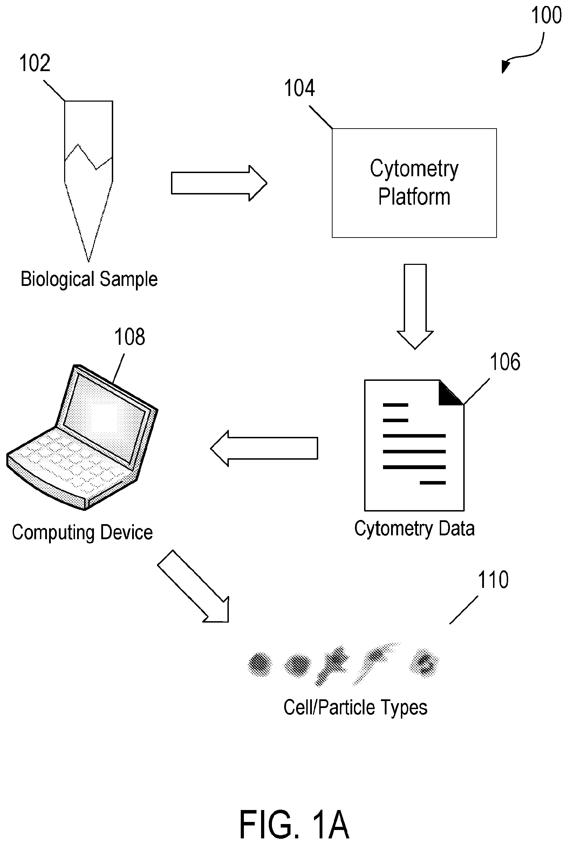

**FIG. 1A** depicts an illustrative technique 100 for determining a respective type 110 for each of one or more events. As described herein, an event corresponds to obtaining measurements for an object in a biological sample 102. Obtaining measurements for an object, in some embodiments, includes obtaining cytometry data, such as cytometry data 106. In some embodiments, the cytometry data 106 is obtained by processing the object using a cytometry platform 104. Additionally, or alternatively, the cytometry data 106 may have been previously-obtained using a cytometry platform 104. A respective type 110 for each of one or more events is determined by processing the cytometry data 106 using computing device 108. In some embodiments, the computing device 108 may be part of cytometry platform 104. In other embodiments, the computing device 108 may be separate from the cytometry platform 104 and may receive cytometry data 106, directly or indirectly, from the cytometry platform 104.

In some embodiments, the illustrated technique 100 may be implemented in a clinical or laboratory setting. For example, the illustrated technique 100 may be implemented on a computing device 108 that is located within the clinical or laboratory setting. In some embodiments, the computing device 108 may directly obtain cytometry data 106 from a cytometry platform 104 within the clinical or laboratory setting. For example, a computing device 108 included within the cytometry platform 104 may directly obtain cytometry data 106 from the cytometry platform 104. In some embodiments, the computing device 108 may indirectly obtain cytometry data 106 from a cytometry platform 104 that is located within or external to the clinical or laboratory setting. For example, a computing device 108 that is located within the clinical or laboratory setting may obtain cytometry data 106 via a communication network, such as Internet or any other suitable network, as aspects of the technology described herein are not limited to any particular communication network.

Additionally, or alternatively, the illustrated technique 100 may be implemented in a setting that is remote from a clinical or laboratory setting. For example, the illustrated technique 100 may be implemented on a computing device 108 that is located externally from a clinical or laboratory setting. In this case, the computing device 108 may indirectly obtain cytometry data 106 that is generated using a cytometry platform 104 located within or external to a clinical or laboratory setting. For example, the cytometry data 106 may be provided to computing device 108 via a communication network, such as Internet or any other suitable network, as aspects of the technology described herein are not limited to any particular communication network.

As shown in FIG. 1A, the technique 100 involves processing a biological sample 102 using a cytometry platform 104, which produces cytometry data 106. The biological sample 102 may be obtained from a subject having, suspected of having, or at risk of having cancer or any immune-related diseases. The biological sample 102 may be obtained by performing a biopsy or by obtaining a blood sample, a salivary sample, or any other suitable biological sample from the subject. The biological sample 102 may include diseased tissue (e.g., cancerous), and/or healthy tissue. In some embodiments, the origin or preparation methods of the biological sample may include any of the embodiments described herein including with respect to the "Biological Samples" section.

The cytometry platform 104 may include any suitable instrument and/or system configured to perform flow or mass cytometry, as aspects of the technology described herein are not limited to any particular type of cytometry system. For example, the cytometry platform may include any suitable flow cytometry platform. Additionally, or alternatively, the cytometry platform may include any suitable mass cytometry platform. In some embodiments, the biological sample 102 may be prepared according to manufacturer's protocols associated with the cytometry platform 104. In some embodiments, the biological sample may be prepared according to any suitable protocol, as embodiments of the technology described herein are not limited to any particular preparation protocol. In some embodiments, flow cytometry techniques may include any of the embodiments described herein including with respect to the "Flow Cytometry" section. In some embodiments, mass cytometry techniques may include any of the embodiments described herein including with respect to the "Mass Cytometry" section.

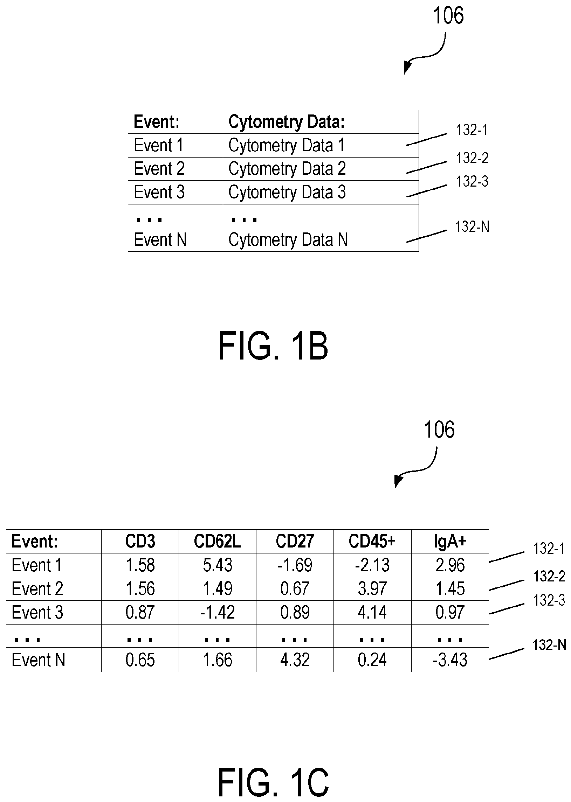

The cytometry data 106 includes cytometry data for each of one or more events. Each event may correspond to obtaining cytometry measurements for an object in the biological sample using a cytometry platform. In some embodiments, the objects include cells, particles, and/or unidentified objects. In some embodiments, the particles include beads, debris, and/or doublets. "Beads," or calibration beads, are particles of a known concentration that can be mixed with a known volume of a biological sample, prior to being processed by a flow cytometer or a mass cytometer. The proportion of beads detected and identified in cytometry data for a subsample can be used to determine the number of cells in the subsample and/or the number of cells of a particular type in the subsample. A "doublet" is a pair of two independent particles or cells that are processed and classified by the cytometry platform as a single particle. This occurs when two cells or particles pass through the cytometry platform very close to one another. The cytometry data 106 is further described herein including at least with respect to FIGS. 1B-1C.

The cytometry data 106 may be processed using computing device 108. In some embodiments, computing device 108 can be one or multiple computing devices of any suitable type. For example, the computing device 108 may be a portable computing device (e.g., a laptop, a smartphone) or a fixed computing device (e.g., a desktop computer, a server). When computing device 108 includes multiple computing devices, the device(s) may be physically co-located (e.g., in a single room) or distributed across multiple physical locations. In some embodiments, the computing device 108 may be part of a cloud computing infrastructure. In some embodiments, one or more computer(s) 108 may be co-located in a facility operated by an entity (e.g., a hospital, a research institution). In some embodiments, the one or more computing device(s) 108 may be physically co-located with a medical device, such as a cytometry platform 104. For example, a cytometry platform 104 may include computing device 108.

In some embodiments, the computing device 108 may be operated by a user such as a doctor, clinician, researcher, patient, or other individual. For example, the user may provide the cytometry data 106 as input to the computing device 108 (e.g., by uploading a file), and/or may provide user input specifying processing or other methods to be performed using the cytometry data 106.

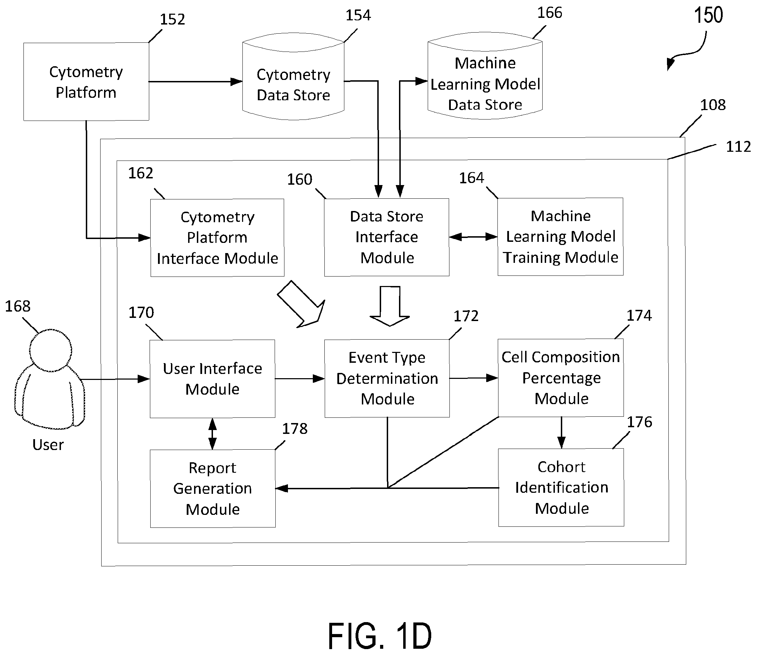

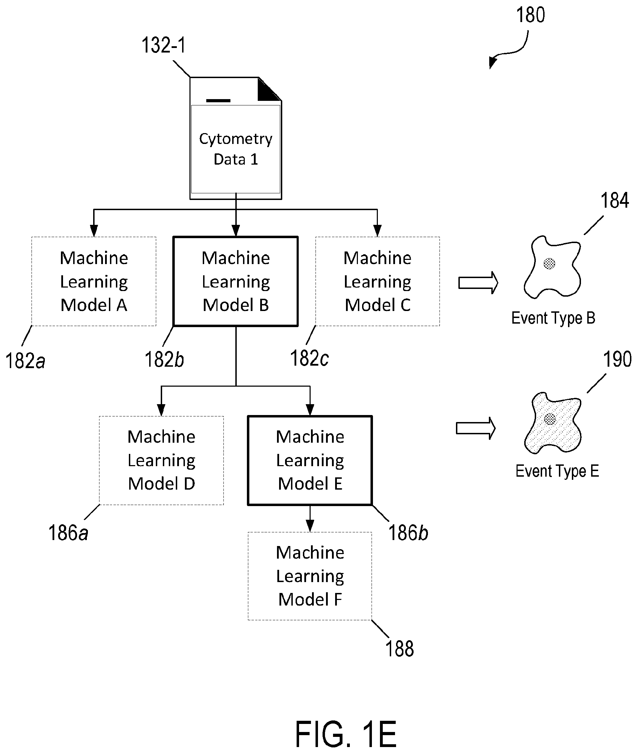

In some embodiments, computing device 108 includes software configured to perform various functions with respect to the cytometry data 106. An example of computing device 108 including such software is described herein including at least with respect to FIG. 1D. In some embodiments, software on computing device 108 is configured to process the cytometry data to identify a respective cell or particle type 110 for each of the one or more events. Example techniques for processing the cytometry data 106 are described herein including at least with respect to FIG. 1E.

In some embodiments, technique 100 additionally includes processing the cytometry data and/or the identified cell or particle types using computing device 108 to determine one or more cell composition percentages for cell types in the biological sample. A cell composition percentage indicates the proportion of a particular cell type in the biological sample 102.

In some embodiments, a cell composition percentage for the biological sample 102 is compared to a cell composition percentage associated with a cohort to predict a diagnosis for the subject, to predict how the subject is likely to respond to a particular treatment, to select a treatment for the subject, or for any other suitable application, as aspects of the technology described herein are not limited in this respect. For example, if the cell composition percentage determined for the biological sample 102 for the subject is comparable to the cell composition percentage associated with a cohort of patients who responded well to a particular treatment, then this may indicate that the subject is likely to respond well to that treatment. Additionally, or alternatively, if the cell composition percentage determined for the biological sample 102 for the subject is comparable to the cell composition percentage associated with a cohort of patients diagnosed with a particular disease, then it may be likely that the subject has the disease.

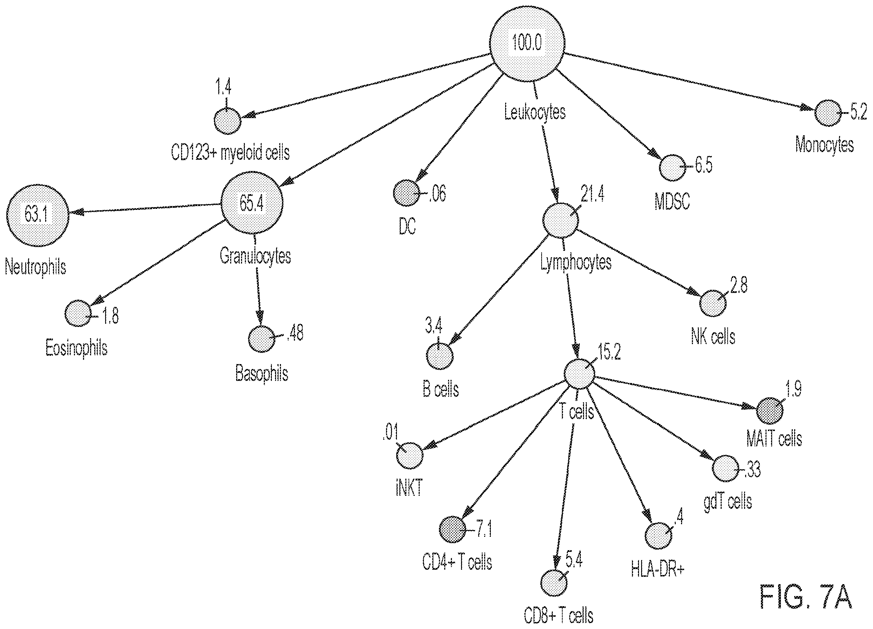

In some embodiments, technique 100 may include generating a report indicating the determined cell and/or particle types 110, cell composition percentages, predicted treatments, predicted diagnoses, and/or any other suitable data resulting from technique 100. In some embodiments, the report may include graphics and/or text. In some embodiments, the report may be stored to memory or displayed via a user interface (e.g., a graphical user interface (GUI)) of a computing device (e.g., computing device 108). Techniques for generating example reports are described herein including at least with respect to FIGS. 1D and FIGS. 7A-7G.

As a nonlimiting example, technique 100 may be performed to determine cell composition percentages of different cell types in a subject suspected of having leukemia. A blood sample may be obtained by a physician and processed using a cytometry platform to obtain cytometry data. The cytometry data may be processed using a computing device to determine a respective cell or particle type for each event and to determine cell composition percentages for the determined cell and particle types. The cell composition percentages indicate the proportion of different cell populations (e.g., populations of different cell types) in the blood sample. For example, this could include determining the percentage of T cells in the blood sample. In some embodiments, the cell composition percentages are compared to those associated with different patient cohorts. For example, the estimate percentage of the subject's T cells may be compared to the percentage of T cells associated with a cohort of patients diagnosed with a particular disease. If the subject has a comparable T cell composition percentage, this may indicate that the subject is a member of the cohort (e.g., has the disease). A report may be generated that indicates the cohort identified for the subject and a visualization of the cell populations in the blood sample.

As shown in **FIG. 1B,** cytometry data 106 includes cytometry data for each of multiple events -event 1 to event N, in this example. The cytometry data 106 includes cytometry data 132-1 for the first event, cytometry data 132-2 for the second event, cytometry data 132-3 for the third event, etc. In some embodiments, all of the events 1 to N correspond to cells. In some embodiments, a portion of the events 1 to N correspond to cells, while a portion of the events 1 to N correspond to particles.

In some embodiments, the cytometry data 106 indicates one or more values for one or more markers used to obtain the cytometry data. A "marker" may include a protein found in a particular cell type or cell types. A "marker value" may be indicative of the expression of such a protein.

In some embodiments, the marker may be fluorescently labelled, and a flow cytometry platform may measure the intensity of the fluorescent light emitted from a particular cell as it is processed. Cells which express the marker at a greater expression level will result in higher marker values (e.g., a higher intensity measurement).

In some embodiments, different markers are labelled with differently-colored fluorescent proteins. This helps to distinguish between the expression of the different markers. In some embodiments, fluorescence intensity is measured for each color of fluorescence emitted from a cell, each of which may be associated with a particular respective marker. For example, if a cell emits green, red, and blue, fluorescent light, this may indicate that the cell expresses three different markers.

Additionally, or alternatively, in some embodiments, the marker may be labelled using a heavy metal ion tag, and a mass cytometry platform may measure the relative intensity (or abundance) of the heavy metal ion tag. The relative intensity of a tag quantifies the amount of the ion produced in relation to the amount of the most abundant ion. In some embodiments, relative intensity is measured for each heavy metal ion tag detected from a cell, each of which may be associated with a particular respective marker.

**FIG. 1C** shows example marker values included in the cytometry data 106 for example markers (e.g., CD3, CD62L, CD27, CD45+, and IgA+).

According to some embodiments, each of the example markers is labelled with a particular color fluorescent protein, and the corresponding marker value indicates the intensity of the fluorescence of that color emitted from the cell. For example, consider a marker CD3 that is labelled with a green fluorescent protein. The marker values for CD3 for the multiple cells represents the intensity of green fluorescence emitted from those cells.

According to some embodiments, each of the example markers is labelled with a particular heavy metal ion tag, and the corresponding marker value indicates the intensity of the tag relative to the most abundant ion.

It should be appreciated that the markers shown in FIG. 1C are nonlimiting examples and any suitable marker or combination of markers may be used in conjunction with some aspects of the technology described herein. Example markers are described herein including at least with respect to Table 2.

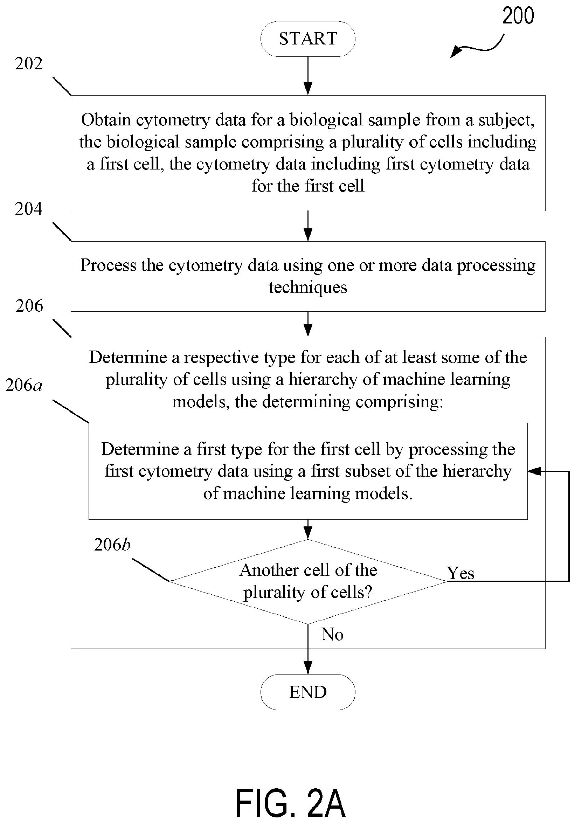

In some embodiments, computing device 108 includes software 112 configured to perform various functions with respect to the cytometry data 106. In some embodiments, software 112 includes a plurality of modules. A module may include processor-executable instructions that, when executed by at least one computer hardware processor, cause the at least one computer hardware processor to perform the function(s) of the module. Such modules are sometimes referred to herein as "software modules." each of which includes processor executable instructions configured to perform one or more processes, such as the processes described herein including at least with respect to FIGS. 2A-2C, FIGS. 5A-5D, and FIG. 8.

**FIG. 1D** is a block diagram of a system 150 including example computing device 108 and software 112, according to some embodiments of the technology described herein. Software 112 includes one or more software modules for processing cytometry data, such as an event type determination module 172, a cell composition percentage module 174, a cohort identification module 176, and a report generation module 178. In some embodiments, the software 112 additionally includes a user interface module 170, a cytometry platform interface module 162, and/or a data store interface module 160 for obtaining data (e.g., user input, cytometry data, one or more machine learning models). In some embodiments, data is obtained from cytometry platform 152, cytometry data store 154, and/or machine learning model data store 166. In some embodiments, the software 112 further includes a machine learning model training module 164 for training one or more machine learning models (e.g., stored in machine learning model data store 166.)

In some embodiments, data obtained from the cytometry data store 154 and/or the cytometry platform 152 and machine learning models obtained from the machine learning model data store 166 are used by the event type determination module 172 to determine types for one or more events. In some embodiments, the obtained data includes cytometry data for a biological sample from a subject. For example, the cytometry data may include cytometry data for each of multiple events in the biological sample, such as first cytometry data for a first event. In some embodiments, the machine learning models obtained from the machine learning model data store 166 include machine learning models that are organized into a hierarchy of machine learning models corresponding to a hierarchy of cell types. In some embodiments, the obtained machine learning models may include one, some, most, or all of the machine learning models included in the hierarchy of machine learning models.

The event type determination module 172 determines a respective type for each of at least some of the events in the biological sample using at least one machine learning model. For example, this may include processing cytometry measurements corresponding to a particular event using a first machine learning model to determine an event type for the event. For example, the first machine learning model may include a multiclass classifier trained to predict an event type from among multiple event types. Additionally, or alternatively, the first machine learning model may include one or more binary class classifiers each trained to predict whether the event is of a particular event type. When the event type indicates that the particular event corresponds to a cell being processed with a cytometry platform, then the event type determination module 172 processes the cytometry measurements corresponding to the particular event using a second machine learning model to determine a type of cell for the event. For example, the second machine learning model may include one or more multiclass classifiers trained to predict a type of cell for the event from among multiple cell types. Additionally, or alternatively, the second machine learning model may include one or more binary class classifiers, each trained to predict whether the event corresponds to obtaining measurements for a cell of a particular type. The first machine learning model is different from the second machine learning model.

In some embodiments, the event type determination module 172 determines a respective type for each of at least some of the events in the biological sample using the hierarchy of machine learning models corresponding to a hierarchy of event types. In some embodiments, this includes determining a first type for a first event in the biological sample by processing first cytometry data using a subset of the hierarchy of machine learning models. For example, the subset of the hierarchy of machine learning models includes one or more of the machine learning models obtained from the machine learning model data store 166. In some embodiments, the event type determination module 172 processes the cytometry data according to the techniques described herein, including at least with respect to FIGS. 2A-C, to determine types for the events included in the biological sample.

In some embodiments, the event type determination module 172 obtains the cytometry data and/or the machine learning models via one or more interface modules. In some embodiments, the interface modules include cytometry platform interface module 162 and data store interface module 160. The cytometry platform interface module 162 may be configured to obtain (either pull or be provided) cytometry data from the cytometry platform 152. The data store interface module 160 may be configured to obtain (either pull or be provided) cytometry data and/or machine learning models from the cytometry data store 154 and/or the machine learning model data store 166, respectively. The data and/or machine learning models may be provided via a communication network (not shown), such as Internet or any other suitable network, as aspects of the technology described herein are not limited to any particular communication network.

In some embodiments, cytometry data store 154 includes any suitable data store, such as a flat file, a data store, a multi-file, or data storage of any suitable type, as aspects of the technology described herein are not limited to any particular type of data store. The cytometry data store 154 may be part of software 108 (not shown) or excluded from software 108, as shown in FIG. 1D.

The cytometry data store 154 may store cytometry data obtained from biological sample(s) of one or more subjects. In some embodiments, the cytometry data may be cytometry data from cytometry platform 152 and/or cytometry data obtained from one or more public data stores and/or studies. In some embodiments, a portion of the cytometry data may be processed with the event type determination module 172 to determine types for events associated with the cytometry data. In some embodiments, a portion of the cytometry may be used to train one or more machine learning models (e.g., with the machine learning model training module 164). In some embodiments, a portion of the cytometry data may include additional data (e.g., event types and/or cell composition percentages) and may be associated with one or more patients in a cohort. This portion of cytometry may be used, for example, by the cohort identification module 176 to identify a subject as a member of a cohort.

In some embodiments, machine learning model data store 166 includes any suitable data store, such as a flat file, a data store, a multi-file, or data storage of any suitable type, as aspects of the technology described herein are not limited to any particular type of data store. The machine learning model data store 166 may be part of software 108 (not shown) or excluded from software 108, as shown in FIG. 1D.

The machine learning model data store 166 may store one or more machine learning models. For example, the machine learning model data store 166 may store a machine learning model trained to predict an event type for an event. Additionally, or alternatively, the machine learning model data store 166 may store one or more machine learning models trained to predict a type of cell for an event. In some embodiments, the machine learning model data store 166 stores a hierarchy of machine learning models used to determine types for events based on cytometry data. In some embodiments, the hierarchy of machine learning models corresponds to a hierarchy of event types. The relationships between the machine learning models in the hierarchy may be stored in the machine learning model data store 166. For example, the machine learning model data store 166 may store a relationship between a machine learning model trained to predict a particular event type and a machine learning model trained to predict a subtype of the event type.

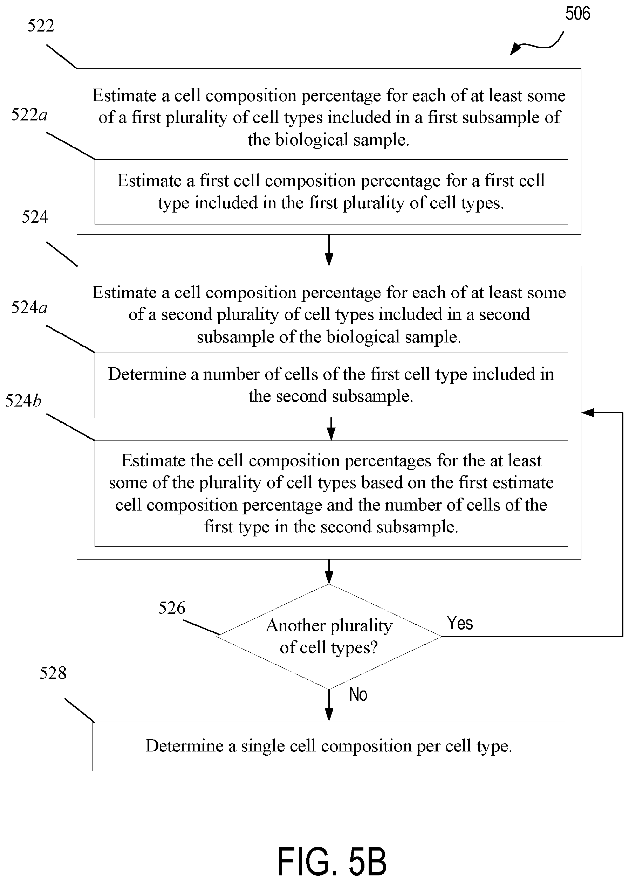

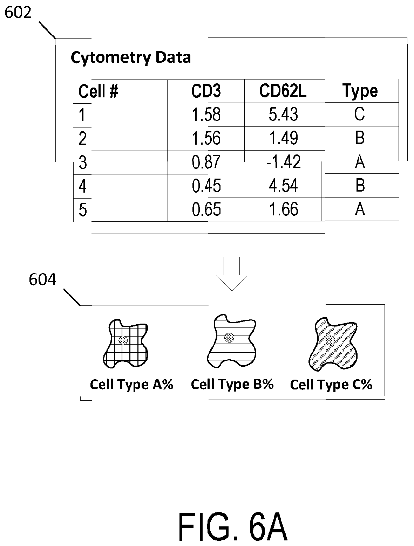

The cell composition percentage module 174 may estimate cell composition percentages for cell populations of different types in a biological sample. In doing so, the cell composition percentage module 174 may use labelled cytometry data indicating the event types of events for which cytometry data has been obtained. For example, the labelled cytometry data may include cytometry data for a first event that is labelled with the type for the first event. In some embodiments, the labels may be determined by the event type determination module 172 and/or determined through alternative means. In some embodiments, the cell composition percentage module 174 estimates the cell composition percentages for cell types included in the biological sample. Additionally, or alternatively, the cell composition percentage module 174 may estimate cell composition percentages for cell types included in subsamples of the biological sample. Example techniques for estimating cell composition percentages are described herein including at least with respect to FIGS. 5B-5C and 6A-6C.

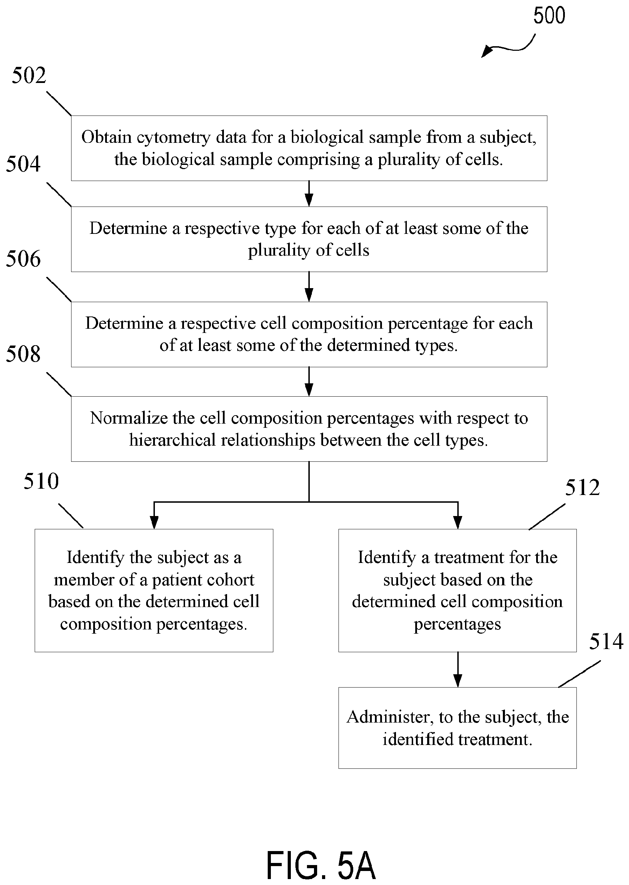

The cohort identification module 176 may identify a patient cohort to which the subject (e.g., from whom the biological sample was obtained) belongs. This may include comparing the cell composition percentages of the subject to those associated with one or more patient cohorts. In some embodiments, the cohort identification module 176 may obtain data associated with the one or more patient cohorts from the data store interface module 160. Additionally, or alternatively, the cohort identification module 176 may obtain input from user 168 via user interface module 170 indicating one or more cohorts (and their associated cell composition percentages) to which the cell composition percentages of the subject should be compared. Example techniques for identifying a subject as a member of a cohort are described herein including at least with respect to FIG. 5A.

In some embodiments, report generation module 178 processes results obtained from the event type determination module 172, the cell composition percentage module 174, and/or the cohort identification module 176 to generate one or more reports. For example, the one or more reports may indicate the event types included in the biological sample, the proportions of cell populations (e.g., cell composition percentages) in the biological sample, and/or one or more cohorts to which the subject belongs. Additionally, or alternatively, the one or more reports may indicate any other suitable information, such as, for example, a diagnosis for the subject, a suggested treatment, and/or relationships between cell populations. In some embodiments, the reports may include visualizations such as charts, graphs, tables, and/or any other suitable visualization for displaying the data. Example reports are described herein including at least with respect to FIGS. 7A-7G.

User interface 170 may be a graphical user interface (GUI), a text-based user interface, and/or any other suitable type of interface through which a user may provide input. For example, in some embodiments, the user interface may be a webpage or web application accessible through an Internet browser. In some embodiments, the user interface may be a graphical user interface (GUI) of an app executing on the user's mobile device. In some embodiments, the user interface may include a number of selectable elements through which a user may interact. For example, the user interface may include dropdown lists, checkboxes, text fields, or any other suitable element.

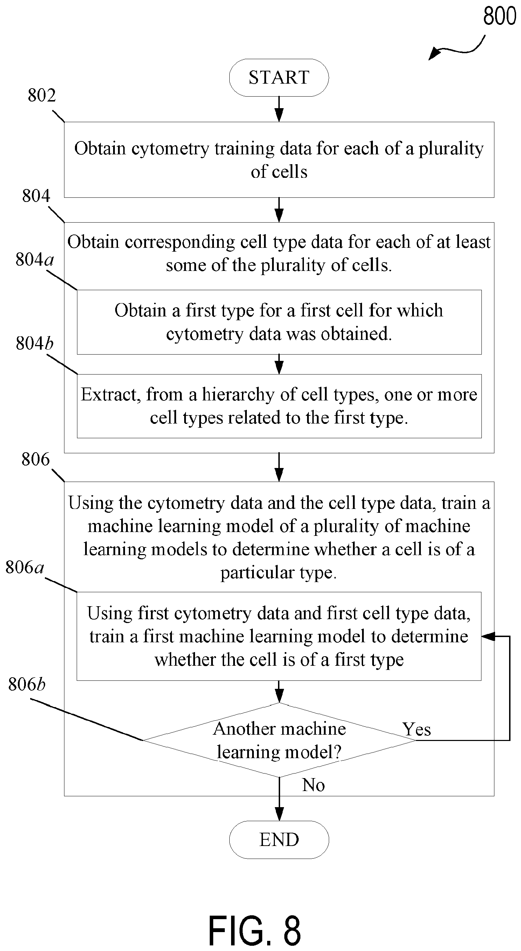

In some embodiments, machine learning model training module 164, referred to herein as training module 164, is configured to train the one or more machine learning models used to determine a type for an event. This may include training a machine learning model to determine whether an event is of a particular type. In some embodiments, the training module 164 trains a machine learning model using a training set of cytometry data. For example, the training module 164 may obtain training data via data store interface module 160. In some embodiments, the training module 164 may provide trained machine learning models to the machine learning model data store 166 via data store interface module 160. Techniques for training machine learning models are described herein including at least with respect to FIG. 8.

**EP 4 473 288 B1**

**FIG. 1E** depicts an illustrative technique 180 for processing cytometry data 106 using computing device 108 to determine a respective type for one or more events (e.g., cells or particles). Illustrative technique 190 includes providing first cytometry data 132-1 for a first event as input to a hierarchy of machine learning models, which is used to determine one or more event types 184, 190 for the first event.

As described with respect to FIG. 1B, cytometry data 106 may include cytometry data for each of multiple cells and particles processed using a cytometry platform 104. For example, the cytometry data 106 includes first cytometry data 132-1 for a first event (e.g., "Event 1" of FIGS. 1B-C). FIG. 1E shows processing the first cytometry data 132-1 to determine one or more types for the first event. However, it should be appreciated that illustrative technique 180 can be used to process cytometry data for any suitable number of events, such as second cytometry data for a second event (e.g., "Event 2" of FIGS. 1B-C), as aspects of the technology described herein are not limited to processing cytometry data for any particular number of events.

In some embodiments, the technique 180 includes processing first cytometry data 132-1 with the hierarchy of machine learning models (e.g., with the event type determination module 172 of FIG. 1D). FIG. 1E shows an example hierarchy of machine learning models, which includes machine learning models 182*a-c*, 186*a-b,* 188.

A machine learning model may be trained to determine whether the first event is of a particular type, based on the first cytometry data 132-1. In some embodiments, this may include determining a probability that the first event is of the particular type. As an example, machine learning model 182*a* may be trained to determine whether the first event is of Type A. As another example, machine learning model 186b may be trained to determine whether the first event is of Type E.

Additionally, or alternatively, a machine learning model may include a multiclass classifier trained to determine whether the first event is one of multiple different event types, based on the first cytometry data 132-1. For example, machine learning model A 182*a* may be trained to determine whether the first event is of Type A1, Type A2, or Type A3. For example, the machine learning model may output the most probable type (e.g., of Type A1, Type A2, or Type A3) for the first event. Such a machine learning model may output a type and/or the probability that the event is of the identified type. For example, machine learning model A 182*a* may identify that the event is more likely Type A2 than Type A1 or Type A3, along with the probability that the event is Type A2.

In some embodiments, different levels of the hierarchy of machine learning models may be used to determine event types with different levels of specificity. For example, machine learning models 182*a-c* may be used to determine that the first event is of Type B 184, while machine learning models 186*a-b* may be used to determine that the first event is of Type E 190, a subtype of Type B 184.

In some embodiments, outputs of machine learning models 182*a-c* are used to inform which machine learning model(s) of the hierarchy will subsequently be used to process the first cytometry data 132-1. For example, the outputs of machine learning models 182*a-c* may indicate which event type, out of the event types associated with each of the models, is the most probable event type for the first event. As shown in the example, the output of machine learning models 182*a-c* indicates that the first event is of Type B 184. Based on the output, the technique 180 may continue with determining whether the first event is of a subtype of Type B 184. Therefore, in some embodiments, machine learning models 186*a-b,* which are trained to determine whether an event is a subtype of Type B 184, may be used to process the first cytometry data 132-1.

In some embodiments, a level of the hierarchy of machine learning models may not indicate any type for the first event. For example, the level of the hierarchy including machine learning model 188 does not indicate a type for the first event. In some embodiments, this may indicate that none of the machine learning models on that level of the hierarchy predicted the first event to be of the particular event type associated with the machine learning model (e.g., for which the machine learning model was trained to determine). For example, machine learning model 188 predicted that the first event is not of Type F. In some embodiments, if a level of the hierarchy does not indicate an event type, then the event type indicated at the previous level of the hierarchy may be determined to be the type for the first event. For example, Type E 190 may be determined as the type for the first event. In this case, Type E 190 represents the most specific type for the first event since Type E 190 is a subtype of Type B 184.

As a nonlimiting example of technique 180, first cytometry data 132-1 may be provided to three machine learning models at a first level of the hierarchy (e.g., machine learning models 182*a-c*). The three machine learning models 182*a-c* may be trained to determine, respectively, the probability that the first event is a monocyte (e.g., Type A), lymphocyte (e.g., Type B), or granulocyte (e.g., Type C). Based on the outputs of machine learning models 182*a-c,* it is determined that the first event is most likely a lymphocyte. The technique 180 may then proceed with determining whether the first event is a subtype of a lymphocyte. For example, this may include processing the first cytometry data 132-1 using machine learning models 186*a-b,* which may be trained, respectively to determine whether the first event is a T cell (e.g., Type D) or B cell (e.g., Type E). As shown, based on the output of machine learning models 186*a-b,* it is determined that the first event is a T cell. Machine learning model 188 may then be used to process first cytometry data 132-1 to determine whether the first event is a CD4 T cell (e.g., Type F), a subtype of the T cell. Since the output of the machine learning model 188 indicates that it is unlikely that the first event is a CD4 T cell, the technique 180 ends. As a result, the first event is determined to be a T cell.

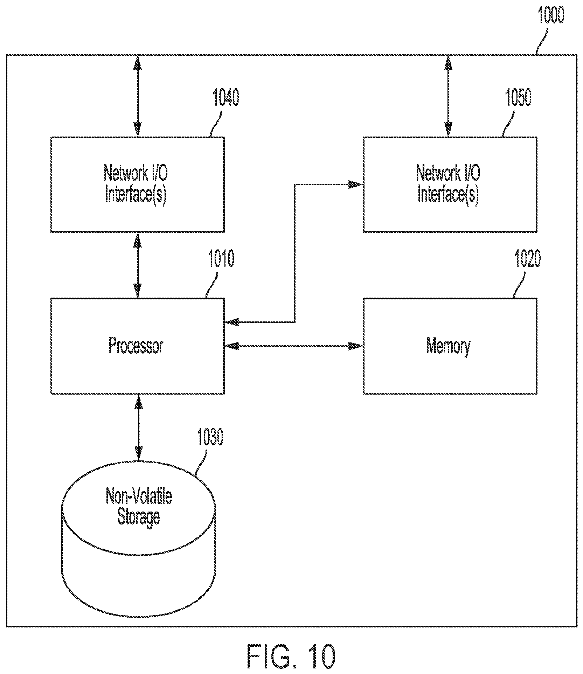

**FIG. 2A** is a flowchart depicting an illustrative process 200 for determining a respective type for one or more cells

16

using cytometry data and a hierarchy of machine learning models, in accordance with some embodiments of the technology described herein. Process 200 may be performed by a laptop computer, a desktop computer, one or more servers, in a cloud computing environment, computing device 108 as described herein with respect to FIG. 1D, computing device 1000 as described herein with respect to FIG. 10, or in any other suitable way.

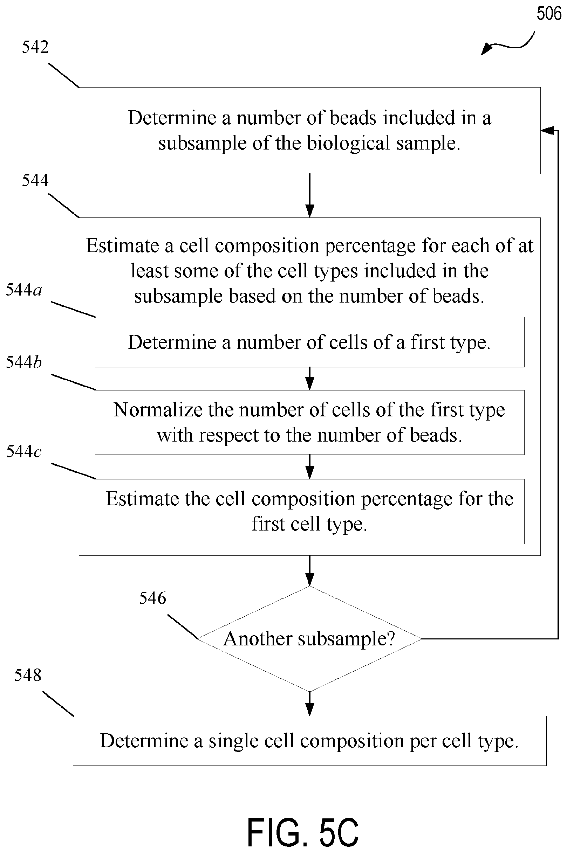

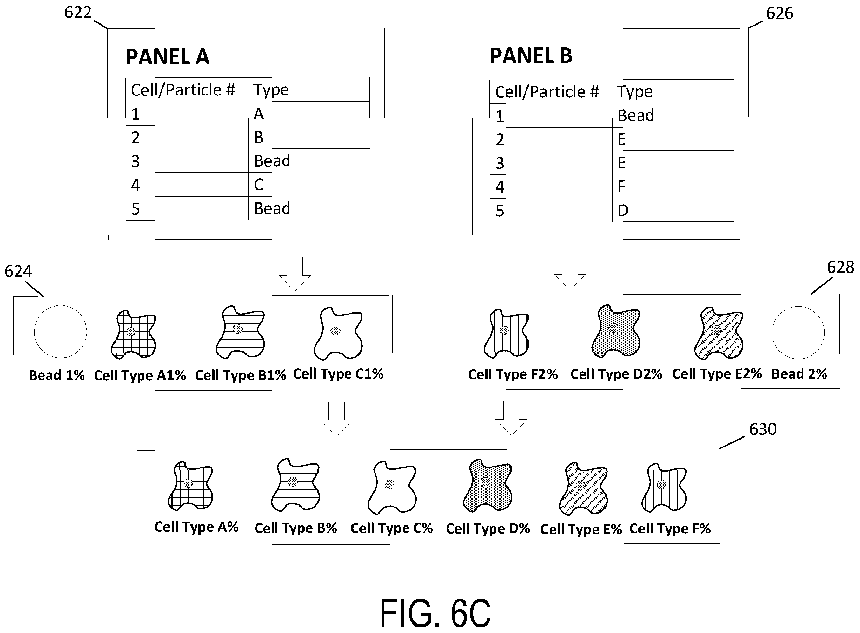

Process 200 begins at act 202, where cytometry data is obtained for a biological sample from a subject. In some embodiments, obtaining the cytometry data includes obtaining cytometry data from the subject in a clinical or research setting and/or from a data store storing such information. The biological sample includes a plurality of cells, such as a first cell and a second cell. In some embodiments, the biological sample may additionally include particles such as debris or beads. For example, beads may be added to the biological sample in order to determine cell composition percentages of different cell types in the sample, as described herein including at least with respect to FIGS. 5C and 6C. In some embodiments, the cytometry data includes first cytometry data for the first cell. For example, the first cytometry data may include data indicating values of markers for the first cell. In some embodiments, the cytometry data includes cytometry data for particles and/or doublets (e.g., when two or more cells are processed as a single cell) in the biological sample. Examples of cytometry data are described herein including at least in the "Flow Cytometry" and "Mass Cytometry" sections.

At act 204, the cytometry data is processed using one or more data processing techniques. In some embodiments, this may include applying a nonlinear transformation to the cytometry data. For example, this may include applying a hyperbolic sine function, such as the function shown in Equation 1, to transform the cytometry data.

$$f(x) = \operatorname{arcsinh}\left(\frac{x}{c}\right) \qquad \text{(Equation 1)}$$

where x is a marker value and c is a cofactor that influences the quality of clustering the cytometry data. According to some embodiments, c is determined experimentally and is selected to produce the highest quality of clustering. For example, the cofactor, c, may equal 190.

Additionally, or alternatively, in some embodiments, processing the data may include normalizing the data to account for asymmetrical data distribution. For example, this may include applying a skewness-adjusted normalization. In some embodiments, this may further include scaling the data. For example, M. Hubert and E. Vandervieren ("An adjusted boxplot for skewed distributions," in Computational Statistics & Data Analysis, vol. 52, no. 12, pp. 5186-5201, 2008), describe example techniques for scaling. However, it should be appreciated that any suitable data processing techniques may be used to process the cytometry data, as embodiments of the technology described herein are not limited to any particular data processing techniques.

Next, process 200 proceeds to act 206, where a respective type is determined for each of at least some of the plurality of cells using a hierarchy of machine learning models corresponding to a hierarchy of cell types. In some embodiments, the hierarchy of machine learning models may include a plurality of machine learning machines, each of which is trained to determine whether a cell is of a particular type or subtype. For example, each machine learning model may be trained to determine the probability that the cell is of the particular type or subtype. Table 1 shows a nonlimiting example of a hierarchy of cell types. A machine learning model in the hierarchy of machine learning models may include one or more of the machine learning models described in the "Machine Learning" section

In some embodiments, the hierarchy of machine learning models may include different levels of machine learning models. For example, machine learning models at one level of the hierarchy may trained to determine relatively general cell types for the cell, while machine learning models at a subsequent level may be trained to determine more specific cell types (e.g., subtypes) for the cell. As a non-limiting example, one level of the hierarchy may be trained to determine whether the cell is a lymphocyte or monocyte, while a subsequent level of the hierarchy may determine whether the cell is a subtype of lymphocytes or monocytes.

In some embodiments, determining a type for a particular cell may include processing the cytometry data for the cell using a subset of the machine learning models included in the hierarchy. Act 206*a* includes determining a first type for the first cell by processing the first cytometry data using a first subset of the hierarchy of machine learning models. In some embodiments, machine learning models included in the first subset are identified during implementation of process 200. After determining a first type for the first cell, process 200 proceeds to act 206*b*, for determining whether the cytometry data includes cytometry data for another cell of the plurality of cells. For example, this includes determining whether the cytometry data includes second cytometry data for a second cell. If, at act 206*b*, it is determined that the cytometry data includes cytometry data for another cell, then process 200 returns to act 206*a*, where a type is determined for that cell by processing the cytometry data (e.g., second cytometry data) using a subset (e.g., a second subset) of the hierarchy of machine learning models. If, at act 206*b*, it is determined that the cytometry data does not include cytometry data for another cell of the plurality of cells, then process 200 ends.

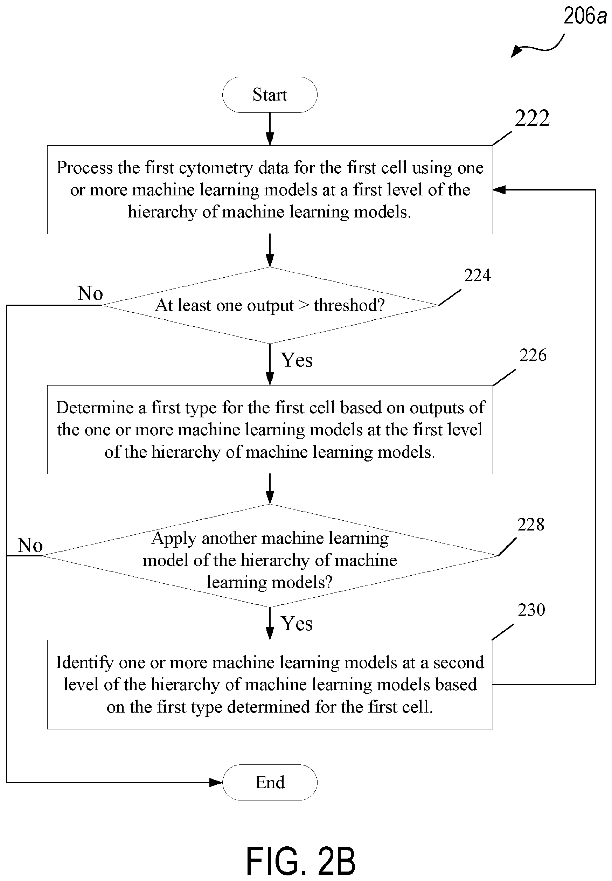

**FIG. 2B** shows an example implementation of act 206*a* for determining a first type for a first cell. The example implementation begins at act 222, which includes processing the first cytometry data using one or more machine learning models at a first level of the hierarchy of machine learning models.

In some embodiments, the outputs of the one or more machine learning models indicate the probability that the cell is of one or more cell types. For example, a machine learning model at the first level of the hierarchy may be trained to determine the probability that a cell is of Type A. In some embodiments, act 224 includes determining whether any of the outputs (e.g., probabilities) exceeds a specified threshold. If at least one of the outputs does not exceed the threshold, then example implementation may end. For example, if none of the outputs exceed the threshold, then the example implementation may end.

In the case that at least one of the outputs exceeds the threshold at act 224, example implementation proceeds to act 226, where a first cell type is determined for the first cell based on the outputs of the one or more machine learning model at the first level of the hierarchy of machine learning models. In some embodiments, this may include identifying the output indicating the highest probability that the cell is of a particular type. For example, the output of a first machine learning model at the first level of the hierarchy may indicate that there is a 70% probability that the cell is of Type A, while a second machine learning model at the first level of the hierarchy may indicate that there is a 30% probability that the cell is of Type B. In this instance, Type A would be identified as the first cell type for the first cell since Type A corresponds to the highest relative probability.

At act 228, process example implementation 206a includes determining whether to process the first cytometry data for the first cell using another machine learning model of the hierarchy of machine learning models. For example, this may include determining whether there are any subtypes of the first type determined for the first cell. For example, if the first cell type is a lymphocyte, then act 228 may include determining that T cells and B cells are subtypes of lymphocytes.

Additionally, or alternatively, determining whether to process the first cytometry data for the first cell using another machine learning model, at act 228, may be based on input (e.g., user input) indicating the degree of specificity to which a cell type should be determined for the first cell. For example, the input may indicate that the cell type should be determined broadly or narrowly for the first cell. Processing the cytometry data with fewer levels of the hierarchy of machine learning model may yield a broader classification, while processing the cytometry data with more levels of the hierarchy of machine learning models may yield a narrower classification.

If, at act 228, it is determined that another machine learning model should not be used, then the example implementation 206a ends. If, at act 228, it is determined that another machine learning model should be applied, then the example implementation 206a proceeds to act 230.

At act 230, one or more machine learning models at a second level of the hierarchy of machine learning models are identified based on the first cell type determined for the first cell. In some embodiments, this may include identifying machine learning models trained to determine whether a cell is a subtype of the first type. For example, if, at act 226, the first cell type determined for the cell was a memory B cell, then a machine learning model trained to determine whether the cell is a class-switched memory B cell and a machine learning model trained to determine whether the cell is a non-switched memory B cell may be identified at act 230.

Example implementation 206a then returns to act 222, where the first cytometry is processed using the one or more machine learning models identified at act 230.

While only a first and a second level of the hierarchy of machine learning models were described with respect to the example implementation 206a, it should be appreciated that the hierarchy of machine learning models may include any suitable number of machine learning models and any suitable number of levels, as aspects of the technology described herein are not limited to any particular number of machine learning models or to any particular number of levels of a machine learning model. For example, the hierarchy may include a third level of machine learning models, which are each trained to determine cell types at a higher degree of specificity than those at the second level. Additionally, or alternatively, the hierarchy of machine learning models may only include a single level of machine learning models used for determining a type for the first cell.

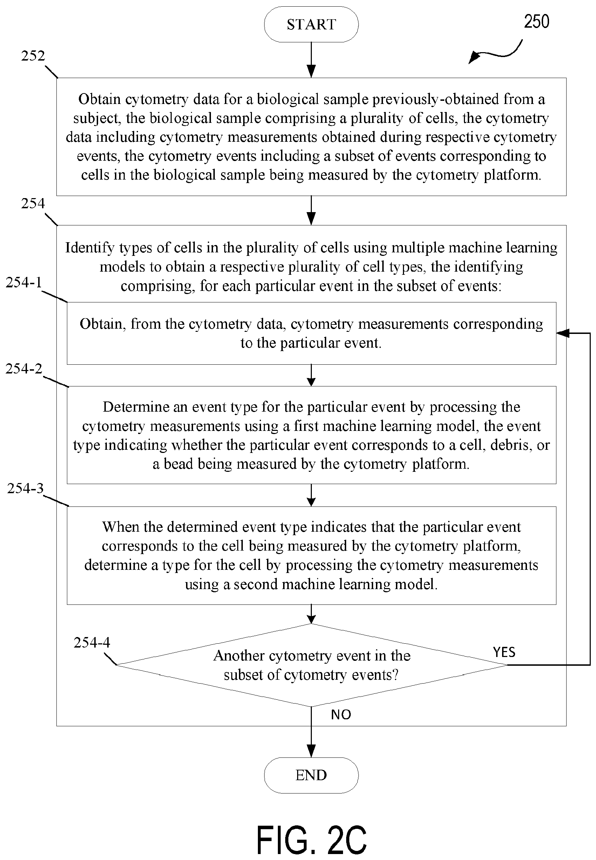

**FIG. 2C** is a flowchart of an illustrative process 250 for determining types for events corresponding to objects in a biological sample being measured by a cytometry platform, according to some embodiments of the technology described herein. Process 200 may be performed by a laptop computer, a desktop computer, one or more servers, in a cloud computing environment, computing device 108 as described herein with respect to FIG. 1D, computing device 1000 as described herein with respect to FIG. 10, or in any other suitable way.

At act 252, the processor obtains cytometry data for a biological sample previously-obtained from a subject. In some embodiments, obtaining the cytometry data includes obtaining cytometry data from the subject in a clinical or research setting and/or from a data store storing such information. The biological sample includes a plurality of objects including a plurality of cells. The plurality of objects may additionally include particles such as debris and/or beads. For example, beads may be added to the biological sample in order to determine cell composition percentages of different cell types in the sample, as described herein including at least with respect to FIGS. 5C and 6C.

The cytometry data includes cytometry measurements obtained during respective cytometry events. As described herein, a cytometry event corresponds to an object (e.g., a cell, debris, a bead, a doublet, or an unidentified object) being measured by a cytometry platform (e.g., a flow cytometry platform or a mass cytometry platform). The cytometry events include a subset of events corresponding to cells in the biological sample being measured by the cytometry platform. For example, the subset of events may include one, some, or all of the cytometry events. The subset of events may include any suitable number of events, as aspects of the technology described herein are not limited in this respect. As nonlimiting examples, the subset of events may include at least 5,000 events, at least 10,000 events, at least 20,000 events, at least 50,000 events, at least 100,000 events, at least 500,000 events, at least 600,000 events, at least 900,000 events, between 500 events and 1 million events, between 5,000 events and 900,000 events, or between 20,000 events and 700,000 events.

The measurements obtained during each of the subset of events is included in the cytometry data obtained at act 252. For example, measurements obtained during a first event of the subsets of events may be included in the cytometry data, where the first event corresponds to a cell in the biological sample being measured by the cytometry platform. Examples of cytometry data are described herein including at least in the "Flow Cytometry" and "Mass Cytometry" sections.

At act 254, the processor identifies types of cells in the plurality of cells using multiple machine learning models to obtain a respective plurality of cell types. For example, the multiple machine learning models include a first machine learning model and a second machine learning model different from the first machine learning model. The first machine learning model and second machine learning model are arranged in a hierarchy of machine learning models. In one nonlimiting example, a hierarchy of machine learning models comprises (a) a first level including a first machine learning model trained to predict an event type for an event and (b) a second level including a second machine learning model trained to predict a type of cell for the event. In one embodiment of the example, the first and the second machine learning models are both multiclass classifiers. In another embodiment of the example, the first machine learning model is a multiclass classifier, and the second machine learning model includes multiple binary class classifiers each trained to determine whether an event is of a respective cell type.

Identifying the cell types using the multiple machine learning models includes performing, for each particular event included in the subset of events, acts 254-1, act 254-2, act 254-3, and act 254-4.

At act 254-1, the processor obtains cytometry measurements corresponding to the particular event. This includes obtaining the cytometry measurements from the cytometry data obtained at act 252. For example, the cytometry measurements may include marker values (e.g., fluorescence intensity values, intensity of heavy metal ion tags, etc.) obtained during the particular event.

At act 254-2, the processor determines an event type for the particular event by processing the cytometry event by processing the cytometry measurements corresponding to the particular event using the first machine learning model. An event type indicates whether the particular event corresponds to a cell being measured by the cytometry platform, debris being measured by the cytometry platform, or a bead being measured by the cytometry platform. Additionally, or alternatively, the event type may indicate whether the particular event corresponds to multiple cells (e.g., a doublet) being measured by the cytometry platform. Additionally, or alternatively, the event type may indicate whether the particular event corresponds to an unidentified object in the biological sample.

The first machine learning model may include any suitable machine learning model configured to predict an event type for an event. For example, the first machine learning model may include a multiclass classifier trained to predict an event type from among multiple event types. Additionally, or alternatively, the first machine learning model may include one or more binary class classifiers, each trained to predict whether the event is of a particular event type. The first machine learning model may be trained to predict a probability that the event is of a particular event type. The first machine learning model may include any suitable machine learning model such as, for example, a decision tree classifier, a gradient boosted decision tree classifier, a neural network, or any other suitable type of machine learning model, as aspects of the technology described herein are not limited in this respect. The first machine learning model may include one or more of the machine learning models described in the "Machine Learning" section.

At act 254-2, when the determined event type indicates that the particular event corresponds to the cell being measured by the cytometry platform, the processor determines a type for the cell by processing the cytometry measurements corresponding to the particular event using the second machine learning model. For example, the processor may determine whether the cell includes one or more of the cell types (or cell subtypes) listed in Table 1.

The second machine learning model may include one or more machine learning models configured to predict a cell type for the event. For example, the second machine learning model may include at least one multiclass classifier trained to predict a type of cell for the particular event from among multiple cell types. Additionally, or alternatively, the second machine learning model may include one or more binary class classifiers each trained to predict whether the event is of a particular cell type. The second machine learning model may be trained to predict a probability that the event is of a particular cell type. The second machine learning model may include any suitable machine learning model such as, for example, a decision tree classifier, a gradient boosted decision tree classifier, a neural network, or any other suitable type of machine learning model, as aspects of the technology described herein are not limited in this respect. The second machine learning model may include one or more of the machine learning models described in the "Machine Learning" section.

At act 254-4, after determining the cell type for the particular event, the processor determines whether the subset of events includes another event. If the subset of events includes another event, one or more of acts 254-1, 254-2, 254-3, and 254-4 may be repeated for the event. For example, the processor may use cytometry measurements corresponding to the next event to determine an event type and/or a cell type for the event. If, at act 254-4, the processor determines that the subset of events does not include another event, then process 250 ends.

It should be appreciated that process 250 may include one or more additional or alternative acts, which are not shown in FIG. 2C. For example, process 250 may include one or more acts for processing the cytometry data, prior to act 254. For example, process 250 may include act 204, described herein including at least with respect to FIG. 2A, for processing the cytometry data.

In some embodiments, as described herein in more detail, the cell types identified as a result of process 250 are used to determine cell composition percentages of different types of cells in the biological sample. For example, determining a first cell composition percentage for a first type of cell may include determining a ratio between a number of cells in the identified as being of the first type and a total number of cells. Example techniques for determining cell composition percentages are described herein including at least with respect to FIGS. 5A-6C.

**Table 1.** Hierarchy of Cell Types.

| Cell type | Cell subtype |
|---|---|
| Generic | Leukocytes |
| Leukocytes | Granulocytes |
| Granulocytes | Eosinophils |
| Granulocytes | Neutrophils |
| Granulocytes | Basophils |
| Leukocytes | Monocytes |
| Monocytes | Classical monocytes |
| Classical monocytes | Classical monocytes FceRI+ |
| Classical monocytes | Classical monocytes FceRI- |
| Monocytes | Non-classical monocytes |
| Leukocytes | Dendritic cells |
| Dendritic cells | cDC |
| cDC | cDC1 |
| cDC | cDC2 |
| Dendritic cells | Plasmacytoid dendritic cells |
| Leukocytes | Lymphocytes |
| Lymphocytes | B cells |
| B cells | Naïve B cells |
| B cells | Memory B cells |
| Memory B cells | Non-switched Memory IgM B cells |
| Memory B cells | Class-switched Memory |
| Class-switched Memory | Switched Memory IgG+ |
| Class-switched Memory | Switched Memory IgA+ |
| B cells | Secreting abs B cells |
| Secreting abs B cells | Plasmablasts |
| Plasmablasts | Plasmablasts IgA+ |
| Plasmablasts | Plasmablasts IgG+ |
| Secreting abs B cells | Plasma cells |
| Plasma cells | Plasma cells IgA+ |

(continued)

| Cell type | Cell subtype |
|---|---|
| Plasma cells | Plasma cells IgG+ |
| Lymphocytes | NK cells |
| NK cells | Immature NK cells |
| NK cells | Mature NK cells |
| Mature NK cells | Mature CD158+ |
| Mature CD158+ | Mature NK CD158+ CD57+ |
| Mature NK cells | Mature CD158- |
| Lymphocytes | T cells |
| T cells | NKT cells |
| T cells | HLA-DR T cells |
| T cells | gdT cells |
| T cells | iNKT |
| T cells | MAIT cells |
| MAIT cells | MAIT CD8+ |
| MAIT cells | MAIT CD8- |
| T cells | CD4 T cells |
| CD4 T cells | CD4 Tregs |
| CD4 Tregs | CD4 Naive Tregs |
| CD4 Tregs | CD4 Memory Tregs |
| CD4 T cells | CD4 T helpers |
| CD4 T helpers | CD4 Naïve T cells |
| CD4 T helpers | CD4 Memory T helpers |
| CD4 Memory T helpers | CD4 Central Memory |
| CD4 Central Memory | CD4 Central Memory CCR4- CCR6- CXCR3+ CXCR5- |
| CD4 Central Memory | CD4 Central Memory CCR4+ CCR6+ CXCR3-CXCR5- |
| CD4 Central Memory | CD4 Central Memory CCR4+ CCR6- CXCR3-CXCR5- |
| CD4 Memory T helpers | CD4 Transitional Memory |
| CD4 Transitional Memory | CD4 Transitional Memory CCR4- CCR6- CXCR3+ CXCR5- |
| CD4 Transitional Memory | CD4 Transitional Memory CCR4+ CCR6+ CXCR3-CXCR5- |
| CD4 Transitional Memory | CD4 Transitional Memory CCR4+ CCR6- CXCR3-CXCR5- |
| CD4 Memory T helpers | CD4 Effector Memory |
| CD4 Effector Memory | CD4 Effector Memory CCR4+ CCR6+ CXCR3-CXCR5- |
| CD4 Effector Memory | CD4 Effector Memory CCR4+ CCR6- CXCR3-CXCR5- |
| CD4 Effector Memory | CD4 Effector Memory CCR4- CCR6- CXCR3+ CXCR5- |
| CD4 Memory T helpers | CD4 TEMRA |
| CD4 Memory Tregs | CD4 Memory Tregs CD39+ |
| CD4 Memory Tregs | CD4 Memory Tregs CD39- |
| CD4 Memory Tregs CD39+ | CD4 Memory Tregs CD39+ ICOS+ |
| CD4 Memory T helpers | CD4 Memory CD39+ |

(continued)

| Cell type | Cell subtype |
|---|---|
| CD4 Memory T helpers | CD4 Memory CD39- |
| T cells | CD8 T cells |
| CD8 T cells | CD8 Naive T cells |
| CD8 Naive T cells | CD8 Stem Cell Memory CD57- CD95+ |
| CD8 Naive T cells | CD8 True Naive T cells |
| CD8 T cells | CD8 Memory T cells |
| CD8 Memory T cells | CD8 Central Memory |
| CD8 Memory T cells | CD8 Transitional Memory |
| CD8 Memory T cells | CD8 Effector Memory |
| CD8 Memory T cells | CD8 TEMRA |
| CD8 Central Memory | CD8 Central Memory PD-1+ |
| CD8 Central Memory | CD8 Central Memory PD-1- |
| CD8 Central Memory PD-1+ | CD8 Central Memory PD-1+ CD39+ |
| CD8 Effector Memory | CD8 Effector Memory PD-1+ |
| CD8 Effector Memory | CD8 Effector Memory PD-1- |
| CD8 Effector Memory PD-1+ | CD8 Effector Memory PD-1+ CD39+ |
| CD8 Transitional Memory | CD8 Transitional Memory PD-1+ |
| CD8 Transitional Memory | CD8 Transitional Memory PD-1- |
| CD8 Transitional Memory PD-1+ | CD8 Transitional Memory PD-1+ CD39+ |
| CD8 TEMRA | CD8 TEMRA PD-1+ |
| CD8 TEMRA | CD8 TEMRA PD-1- |
| CD8 TEMRA PD-1+ | CD8 TEMRA PD-1+ CD39+ |
| CD8 Central Memory | CD8 Central Memory CD57+ |
| CD8 Central Memory | CD8 Central Memory CD57- |
| CD8 Effector Memory | CD8 Effector Memory CD57+ CD95+ |
| CD8 Effector Memory | CD8 Effector Memory CD57- CD95+ |
| CD8 Effector Memory CD57+ CD95+ | CD8 Effector Memory CD57+ CD95+ CX3CR1+ |
| CD8 Effector Memory CD57- CD95+ | CD8 Effector Memory CD57- CD95+ CX3CR1- |
| CD8 Transitional Memory | CD8 Transitional Memory CD57+ |
| CD8 Transitional Memory | CD8 Transitional Memory CD57- |
| CD8 TEMRA | CD8 TEMRA CD57+ |
| CD8 TEMRA | CD8 TEMRA CD57- |

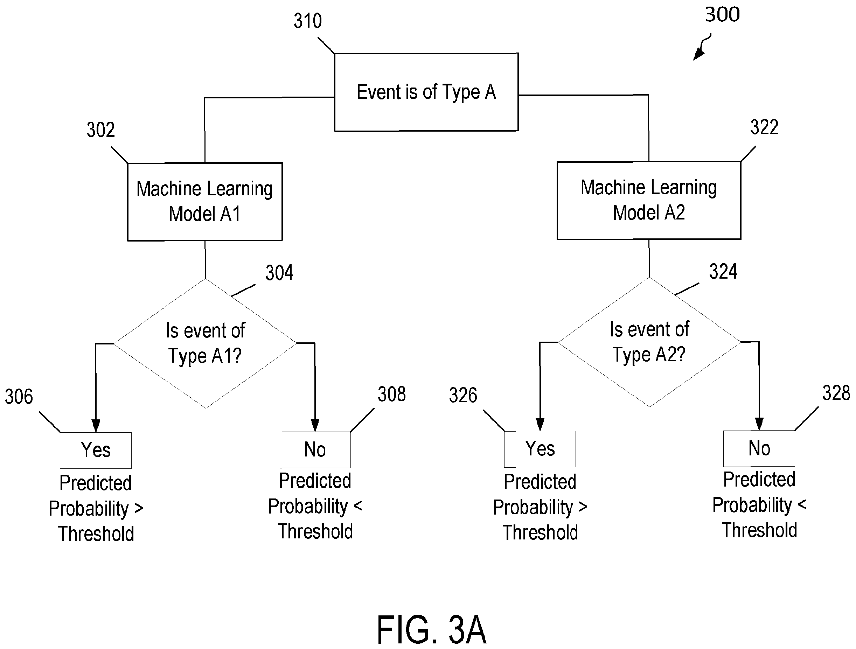

**FIG. 3A** shows an example diagram for determining a type for an event based on an output of binary class classifiers, according to some embodiments of the technology described herein. As shown, the event is determined, at act 310 to be of Type A. The techniques 300 include determining whether the event is of Type A1 or Type A2, both of which are subtypes of Type A.

In some embodiments, machine learning model A1 302 may be trained to estimate a probability that the event is of Type A1. Machine learning model A1 302 may include a decision tree classifier, a gradient boosted decision tree classifier, a neural network, or any other suitable type of machine learning model, as aspects of the technology described herein are not limited in this respect. In some embodiments, machine learning model A1 302 may include an ensemble of machine learning models of any suitable type. For example, machine learning model A1 302 may include an ensemble of decision tree classifiers, an ensemble of gradient boosted decision tree classifiers, or an ensemble of neural networks. The machine learning model A1 302 may include one or more of the machine learning models described herein including in the "Machine Learning" section.

At act 304, machine learning model A1 302 is used to process the cytometry data for the event to determine whether the event is of Type A1. In some embodiments, this may include determining whether the probability predicted by the first machine learning model exceeds a threshold. For example, the threshold may be .2, .3, .5, .6, .7, or any suitable threshold, as aspects of the technology described herein are not limited to any particular threshold.

If, at act 304, it is determined that the probability does not exceed the threshold, then Type A1 is not identified for the event 308. By contrast, if it is determined that the probability exceeds the threshold at act 304, then Type A1 is identified for the event 306.

In some embodiments, machine learning model A2 322 may be trained to estimate a probability that the event is of Type A2. Machine learning model A2 322 may include a decision tree classifier, a gradient boosted decision tree classifier, a neural network, or any other suitable type of machine learning model, as aspects of the technology described herein are not limited in this respect. In some embodiments, machine learning model A2 322 may include an ensemble of machine learning models of any suitable type. For example, machine learning model A2 322 may include an ensemble of decision tree classifiers, an ensemble of gradient boosted decision tree classifiers, or an ensemble of neural networks.

At act 324, machine learning model A2 322 is used to process the cytometry data for the event to determine whether the event is of Type A2. In some embodiments, this may include determining whether the probability predicted by the first machine learning model exceeds a threshold. For example, the threshold may be .2, .3, .5, .6, .7, or any suitable threshold, as aspects of the technology described herein are not limited to any particular threshold.

If, at act 324, it is determined that the probability does not exceed the threshold, then Type A2 is not identified for the event 328. By contrast, if it is determined that the probability exceeds the threshold at act 324, then Type A2 is identified for the event 326.

According to some embodiments, machine learning model A1 302 may output that the event is of Type A1 306, while machine learning model A2 322 may output that the event is not of Type A2 328. In this case, Type A1 is identified for the event.

Similarly, in some embodiments, machine learning model A2 322 may output that the event is of Type A2 326, while machine learning model A1 302 may output that the event is not of Type A1 308. In this case, Type A2 is identified for the event.

Alternatively, in some embodiments, machine learning model A1 302 may output that the event is of Type A1 306 and machine learning model A2 322 may output that the event is of Type A2 326. In some embodiments, to determine the type for the event, the type associated with the greatest probability is selected. For example, if machine leaning model A1 302 output a probability of .8 that the event is of Type A1 and machine learning model A2 322 output a probability of .9 that the event is of Type A2, then Type A2 would be identified for the event.

Alternatively, in some embodiments, machine learning model A1 302 may output that the event is not of Type A1 308 and machine learning model A2 322 may output that the event is not of Type A2 328. In this case, neither type is identified for the event.

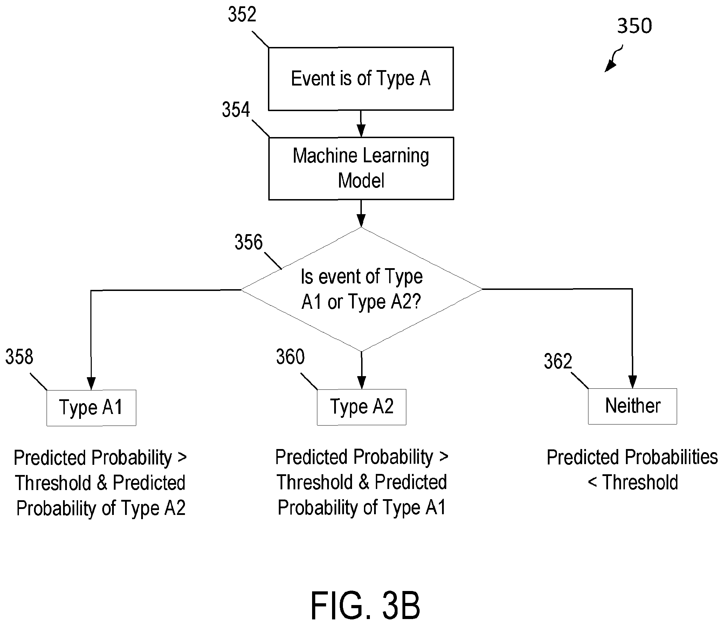

**FIG. 3B** shows an example diagram for determining a type for an event based on an output of a multiclass classifier, according to some embodiments of the technology described herein. As shown, the event is determined, at act 352 to be of Type A. The techniques 350 include determining whether the event is of Type A1 or Type A2, both of which are subtypes of Type A.

In some embodiments, machine learning model 354 may be trained to estimate whether the event is of Type A1, Type A2, or neither Type A1 nor Type A2. Machine learning model 352 may include a decision tree classifier, a gradient boosted decision tree classifier, a neural network, or any other suitable type of machine learning model, as aspects of the technology described herein are not limited in this respect. In some embodiments, machine learning model 352 may include an ensemble of machine learning models of any suitable type. For example, machine learning model 352 may include an ensemble of decision tree classifiers, an ensemble of gradient boosted decision tree classifiers, or an ensemble of neural networks.

At act 356, machine learning model 354 is used to process the cytometry data for the event to determine whether the event is of Type A1 or Type A2. In some embodiments, this may include determining which type is more probable for the event.

Additionally, or alternatively, this may include determining whether that probability is greater than a threshold. For example, the threshold may be .2, .3, .5, .6, .7, or any suitable threshold, as aspects of the technology described herein are not limited to any particular threshold.

If, at act 356, it is determined that the probability that the event is of Type A1 exceeds (a) the threshold and (b) the probability that the event is of Type A2, then Type A1 is output 358. If, at act 356, it is determined that the probability that the event is of Type A2 exceeds (a) the threshold and (b) the probability that the event is of Type A1, then Type A2 is output 360. If, at act 356, neither probability exceeds the threshold, then neither Type A1 nor Type A2 is output 362.

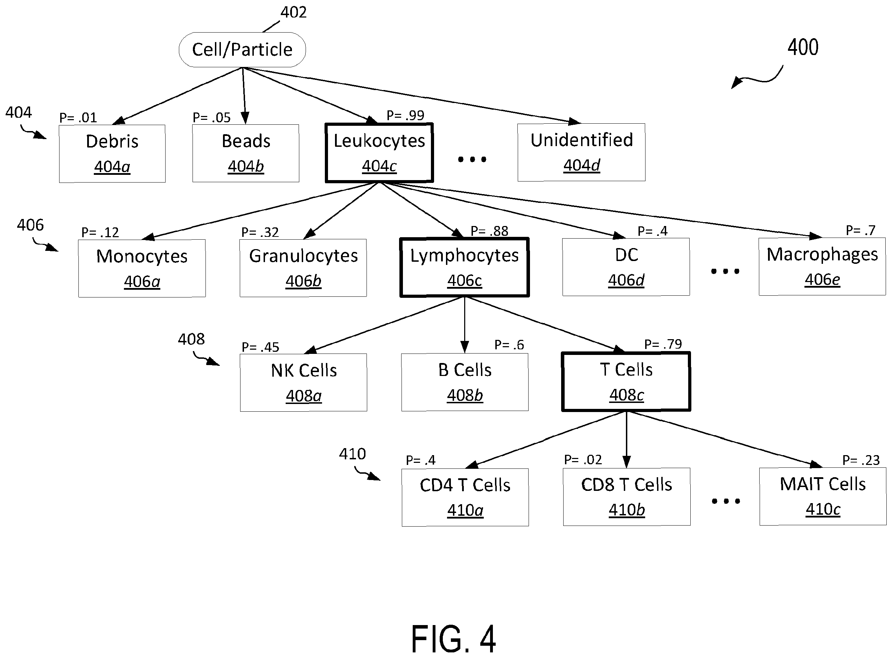

**FIG. 4** depicts an illustrative example for determining one or more types for an event based on a hierarchy 400 of event types, according to some embodiments of the technology described herein.

In some embodiments, the different event types shown in the hierarchy 400 represent potential types for the event 402. In some embodiments, an event type may correspond to a machine learning model trained to determine, based on cytometry data for the event 402, whether the event 402 is of the particular event type. For example, a machine learning model may be trained to determine the probability that the event 402 is a leukocyte 404c and whether that probability exceeds a threshold. Here, the machine learning model corresponding to leukocytes 404c output a .99 probability that the event 402 is a leukocyte 404c, which exceeds an example threshold of .5.

In some embodiments, in addition to processing the cytometry data for event 402 with a machine learning model trained to determine whether the event 402 is a leukocyte 404c, the techniques may include processing the cytometry data with a machine learning model trained to determine whether the event 402 is debris 404a and a machine learning model trained to determine whether the event 402 is a bead 404b. Here, the machine learning models output probabilities of .01 and .05, respectively. Because the probabilities are less than the example threshold of .5, it is determined that the event 402 is not a bead 404a or debris 404b.

Because the probability of the event 402 being a leukocyte 404c is greater than both the threshold and the other probabilities output at level 404, the event 402 may therefore be identified as a leukocyte 404c at this level.

As shown in the hierarchy, level 404 corresponds to the broadest classification of the event 402. Based on the output of level 404, it may be possible to determine a more specific type for the event 402. For example, since the event 402 is determined to be a leukocyte *404c,* it may be possible to determine whether the event 402 is a specific subtype of a leukocyte 404c. In particular, level 406 corresponds to some event types (e.g., monocytes 406a, granulocytes 406b, lymphocytes 406c, DC 406d, and macrophages 406e) which are subtypes of leukocytes 404c.

To determine whether the event 402 is of a subtype of a leukocyte 404c, the cytometry data of event 402 may be processed with the machine learning models trained to determine whether event 402 is of one of the subtypes 406a-e, as was done at level 404. Here, the probabilities of the event 402 being a lymphocyte 406c (e.g., .88) and being a macrophage 406e (.7), each exceeded the example threshold (e.g., .5). Since the probability of the event 402 being a lymphocyte 406c (e.g., .88) is greater than the probability of the event being a macrophage 406e (e.g., .7), the event is identified as being a lymphocyte 406c.

Level 408 corresponds to cell types which are subtypes 408a-c of lymphocytes 406c. The same processing techniques may again be used to determine the probabilities of the event 402 being each of the subtypes 408a-c. Here, T cells 408c are identified as the type for the event 402, with a probability of .79 that is both greater than the example threshold (e.g., .5) and greater than the probabilities of the event 402 being an NK cell 408a (e.g., .45) and the event 402 being a B cell 408b (e.g., .6).

Level 410 corresponds to event types which are subtypes 410a-c of T cells 408c. The same processing techniques may again be used to determine the probabilities of the event 402 being each of the subtypes 410a-c. Here, no event type is determined for the event 402 at this level 410 because none of the determined probabilities (e.g., .4, .02, or .23) are greater than the example threshold (e.g., .5). Therefore, the event type determination is complete.

Here, three types are determined for event 402: Leukocyte, Lymphocyte, and T cell, ranging from least specific to most specific. In some embodiments, the techniques may output one, some, most, or all types determined for the event 402.

*Cell Composition Percentages and Patient Cohorts*

**FIG. 5A** is a flowchart of an illustrative process 500 for identifying a subject as a member of a patient cohort, according to some embodiments of the technology described herein. Process 500 may be performed by a laptop computer, a desktop computer, one or more servers, in a cloud computing environment, computing device 108 as described herein with respect to FIG. 1D, computing device 1000 as described herein with respect to FIG. 10, or in any other suitable way.

Process 500 begins at act 502 for obtaining cytometry data for a biological sample from a subject, the biological sample including a plurality of cells. In some embodiments, act 502 may be performed according to the techniques described herein, including at least with respect to act 202 of process 200 and/or act 252 of process 250.

At act 504, a respective type is determined for each of at least some of the plurality of cells. In some embodiments, act 504 may be performed according to the techniques described herein including at least with respect to FIGS. 2A-C for determining types for cells in a biological sample. Additionally, or alternatively, act 504 may include processing the cytometry data in any suitable way to determine cell types for the cells in the biological sample, as aspects of the technology described herein are not limited to any particular techniques for processing the cytometry data.

At act 506, a cell composition percentage is determined for each of at least some of the determined cell types. In some embodiments, determining a cell composition percentage for a cell type for a biological sample may include determining a ratio between the number of cells of a particular type and a total number of cells for which cytometry data has been obtained. For example, **FIG. 6A** shows example cytometry data for a biological sample for which types are determined for cells 1-5. Determining a cell composition percentage for cells of Type A may include determining the ratio of the number of cells of Type A to the total number of cells. In this example, the cell composition percentage of cells of Type A would therefore be 2/5.

In some embodiments, the cytometry data may be obtained for multiple subsamples of the biological sample using different panels of markers. As described herein, a panel of markers may be used to identify a particular cell type or cell types, but it may not be able to distinguish all cell types in the subsample. Therefore, a first panel may be used to determine a number of cells of Type A, along with a number of unidentifiable cells, while a second panel may be used to determine a number of cells of Type B, along with a number of unidentifiable cells. While the first panel may include cells of Type B, they may not be identified in the cytometry data associated with that panel (e.g., included in the unidentified cells). Similarly, while the second panel may include cells of Type A, they may not be identified in the cytometry data associated with that panel. Therefore, it may be challenging to determine the total number of cells of a particular type (e.g., Type A, Type B) in the biological sample and the corresponding cell composition percentage.

Accordingly, in some embodiments, act 506 may include performing one or more normalization techniques to account for data from individual panels. FIGS. 5B and 5C are flowcharts depicting example implementations of act 506.

**FIG. 5B** is a flowchart depicting an example implementation of act 506 of process 500 for determining cell composition percentages based on estimate cell composition percentages of a common cell type.

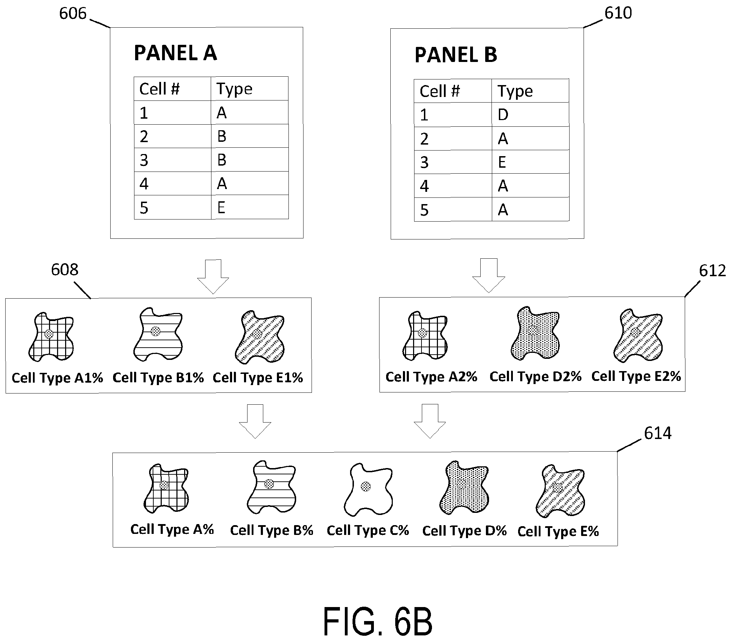

The example implementation begins at act 522 for estimating a cell composition percentage for each of at least some of a first plurality of cell types included in a first subsample of the biological sample. In some embodiments, the first plurality of cell types may be associated with a first panel of cytometry data obtained for the first subsample of the biological sample. For example, as shown in **FIG. 6B,** this may include estimating cell composition percentages 608 for the cell types associated with panel A 606.

Act 522*a* includes estimating a first cell composition percentage for a first cell type included in the first plurality of cells. In some embodiments, estimating the first cell composition percentage may include determining a ratio between the number of cells of the first type and the total number of cells included in the first subsample. In some embodiments, the first cell type may be considered the "reference cell type" and the cell composition percentage may be considered a "reference cell composition percentage". For example, as shown in FIG. 6B, this may include determining the cell composition percentage 608 for cell type A.

At act 524, the example implementation 506 includes estimating a cell composition for each of at least some of a second plurality of cell types includes in a second subsample of the biological sample. In some embodiments, the second plurality of cell types may include cells of the first type (e.g., the reference cell type) and may be associated with a second panel of cytometry data obtained for a second subsample of the biological sample. For example, as shown in FIG. 6B, panel B 610 includes data for a plurality of cell types, including cell type A. Cell composition percentages 612 may then be determined for the cell types associated with panel B 610.

Act 524*a* includes determining a number of cells in the second subsample that are of the first cell type. For example, with respect to FIG. 6, this may include determining that there are three cells of type A associated with panel B 610.

Act 524b includes estimating a cell composition percentage for the at least some of the plurality of cell types based on the first estimate cell composition percentage (e.g., the reference cell composition percentage) and the number of cells of the first type (e.g., the reference cell type) in the second subsample. In some embodiments, this may include normalizing a cell composition percentage for a cell type based on the first and second estimate cell composition percentages. For example, a cell composition percentage (CCP) for a cell type N may be estimated according to Equation 2:

$$CCP = \frac{(\#\ Cells\ of\ Type\ N) \times (\#\ Cells\ of\ Ref.Type)}{Ref\ Cell\ Composition\ Percentage} \qquad \text{(Equation 2)}$$

where, the number of cells of type N are the number of cells of Type N in the second subsample, the number of cells of the reference type are the number of cells of the reference type in the second subsample (e.g., associated with the second panel), and the reference cell composition percentage is the cell composition percentage of the reference type in the first subsample (e.g., the first panel).

The example implementation of act 506 then proceeds to act 526, for determining whether there is another plurality of cell types (e.g., a third plurality of subtypes) associated with another subsample (e.g., a third sample). If there is another plurality of cell types, then the implementation 506 returns to act 524 for estimating cell composition percentages for the plurality of cell types. If there is not another plurality of cell types, then the implementation proceeds to act 528.

Act 528 includes determining a single cell composition percentage per cell type. For example, if different subsamples include cells of the same type, and cell composition percentages were determined for that cell type for both subsamples, then a single cell composition percentage may be determined for that cell type. In some embodiments, this may include averaging the cell composition percentages. Additionally, or alternatively, this may include selecting one of the cell composition percentages. As a nonlimiting example, consider FIG. 6B. Panel A 606, corresponding to a first subsample, and panel B 610, corresponding to a second subsample, each include cells of type A and cells of type E. Cell composition percentages 608, 610 are determined for each type (e.g., Cell Type A1%, Cell Type A2%, Cell Type E1%, and Cell Type E2%). The cell composition percentages are then averaged 614 to obtain one cell composition percentage for each cell type for the biological sample.

**FIG. 5C** is a flowchart depicting an example implementation of act 506 of process 500 for determining cell composition percentages based on percentages of beads, according to some embodiments of the technology described herein.

Act 542 includes determining a number of beads included in a subsample of a biological sample. For example, as shown in **FIG. 6C,** this may include determining a number of beads (e.g., 2) included in a subsample associated with panel A 622.

Act 544 includes estimating a cell composition percentage for each of at least some of the cell types included in the subsample based on the number of beads determined at act 542.

In some embodiments, this may include, at act 544*a*, determining a number of cells of a first type in the subsample. For example, as shown in FIG. 6C, panel A 622 associated with a first subsample includes one cell of type A.

At act 544*b*, the number of cells of the first type (e.g., cell count) is normalized with respect to the number of beads. In some embodiments, the cell count is normalized according to Equation 3:

$$Normalized\ Cell\ Count = \frac{\#\ Cells\ of\ Type\ N}{\#\ Beads} \times Bead\ Conc. \qquad \text{(Equation 3)}$$

where the number of beads is the number of beads determined for the subsample at act 552, the number of cells of Type N is the number of cells of the first type determined at act 544*a*, and the bead concentration is the concentration of beads included in the biological sample. In some embodiments, the concentration of beads may be measured in beads per million cell units.

Act 544*c* incudes estimating a cell composition percentage for the first cell type. In some embodiments, this includes determining a ratio between the normalized cell count and the total number of cells in the biological sample.

Consider, as a nonlimiting example of act 544, that a biological sample is partitioned into subsample A and subsample B, associated respectively with panel A 622 and panel B 626, as shown in FIG. 6C. To estimate a cell composition percentage for cell type C, associated with panel A 622, the number of cells of type C (e.g., one cell) may be normalized with respect to the number of beads (e.g., two beads) associated with panel A 627 and the concentration of beads (e.g., 5,000 beads per million cell units) to determine a normalized cell count. To estimate the cell composition percentage of cell type C for subsample A, a ratio between the normalized cell count and the total number of cells in the biological sample (e.g., seven) may be determined.

The example implementation of act 506 then proceeds to act 546, for determining whether there is another subsample of the biological sample. If there is another subsample, then the implementation 506 returns to act 542 for determining the number of beads in the subsample. If there is not another plurality of cell types, then the implementation proceeds to act 548.

Act 548 includes determining a single cell composition percentage per cell type. In some embodiments, the techniques may include those described herein including at least with respect to act 528.

Returning now to FIG. 5A, after determining a respective cell composition percentage for each of at least some of the cell types of the biological sample, process 500 proceeds to act 508.

Act 508 includes normalizing the cell composition percentages with respect to hierarchical relationships between the cell types. As described above, some techniques include determining cell composition percentages for different levels of a hierarchy of cell types. The cell composition percentage of a more general cell type (e.g., a "parent" cell type) should, in theory, be equivalent to the sum of its "descendant" cell types. For example, B cells, T cells, and NK cells are subtypes of lymphocytes. Therefore, the sum of the cell composition percentages for these types should be equal to the cell composition percentage determined for lymphocytes.

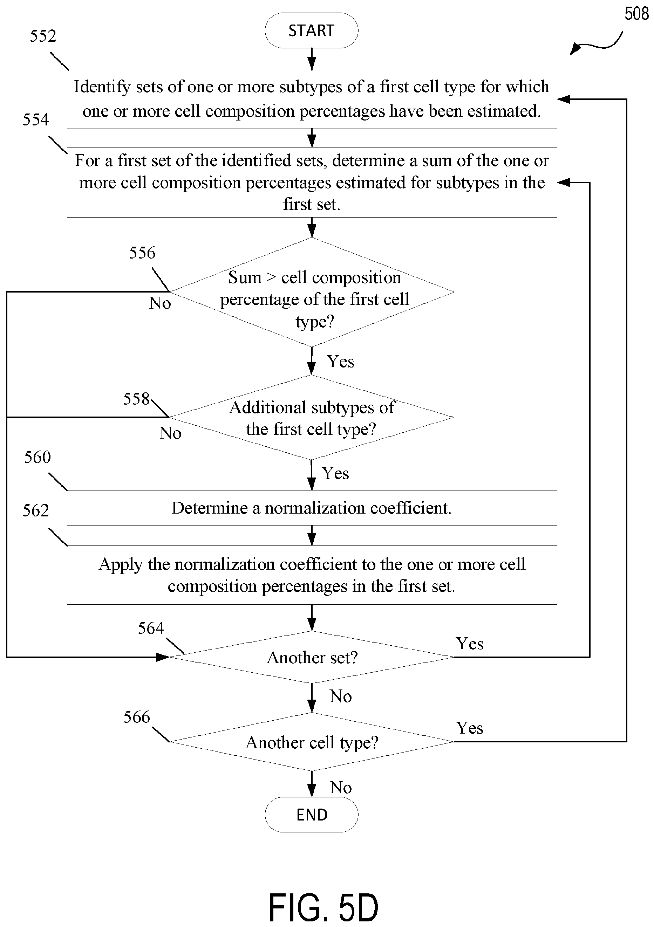

However, in some embodiments, the sum of the estimated cell composition percentages of descendant cell types may exceed the estimated cell composition percentage of the parent cell type. Therefore, described herein, including with respect to FIG. 5D, are techniques for normalizing the estimate cell composition percentages, such that the sum of the estimate cell composition percentages of the descendant cell types do not exceed the cell composition percentage of the parent cell type.

In some embodiments, there may be challenges associated with normalizing cell composition percentages with respect to hierarchical relationships between cell types. In particular, such challenges may arise when determining cell composition percentages based on data from multiple different subsamples, as described herein including at least with respect to FIGS. 5B-5C. Therefore, the techniques described herein including with respect to FIG. 5D include techniques for normalizing cell composition percentages when there are multiple subsamples.

Consider, for example, a first subsample including cells of Type A1 and Type A2 and a second subsample including cells of Type A3 and Type A4, where each of cell Types A1, A2, A3, and A4 are subtypes of Type A. For the first subsample, the cell composition percentage of Type A should be equivalent to the sum of the cell composition percentage of Type A1 and the cell composition percentage of Type A2 (e.g., Type A = Type A1 + Type A2). For the second subsample, the cell composition percentage of Type A should be equivalent to the sum of the cell composition percentage of Type A3 and the cell composition percentage of Type A4 (e.g., Type A = Type A3 + Type A4). However, when combining data from different subsamples to determine cell composition percentages for the biological sample, the combined cell composition percentage of Type A is not equivalent to sum of the cell composition percentages of Types A1, A2, A3, and A4 (e.g., Type A $\neq$ Type A1 + Type A2 + Type A3 + Type A4). Therefore, the subtypes of different subsamples may be treated independently from one another when normalizing according to the techniques described with respect to FIG. 5D.

FIG. 5D is a flowchart depicting an example implementation of act 508 of process 500 for normalizing cell composition percentages with respect to hierarchical relationships between cell types.

Example implementation 508 begins at act 552 for identifying sets of one or more subtypes of a first cell type for which one or more cell composition percentages have been estimated. When cell composition percentages were estimated from a single panel of cytometry data for the biological sample, there may be only one set of cell subtypes. For example, for a leukocyte, a set of subtypes may include monocytes, granulocytes, lymphocytes, and macrophages. Additionally, or alternatively, when cell composition percentages were estimated from two or more panels of cytometry data corresponding to multiple subsamples of the biological sample, there may be multiple respective sets of cell subtypes. For example, for a leukocyte, a first set may include monocytes and macrophages, while a second set may include lymphocytes and granulocytes.

Example implementation 508 then proceeds to act 554, where, for a first set of the identified sets of cell composition percentages, the techniques include determining a sum of the cell composition percentages estimated for subtypes included in the first set. The sum is then compared to the cell composition percentage of the first cell type, at act 556, to determine whether it exceeds the cell composition percentage of the first cell type. For example, the sum of the cell composition percentages of granulocytes and lymphocytes may be compared to the cell composition percentage of leukocytes.

If the sum does not exceed the cell composition percentage of the first cell type, then example implementation 508 proceeds to act 564 for determining whether there is another set (e.g., a second set) of cell subtypes that was identified at act 552. If the sum does exceed the cell composition percentage of the first cell type, then example implementation proceeds to act 558.

Act 558 includes determining whether the set of cell subtypes identified at act 559 include all possible subtypes of the first cell type. In some embodiments, this may include obtaining data from one or more data stores indicating potential cell types for the first type. For example, if the first cell type is a leukocyte, then act 558 may include accessing a data store to identify potential subtypes of leukocytes and comparing those to the subtypes already identified.

If, at act 558, it is determined that there are additional potential subtypes of the first cell type, then example implementation proceeds to act 564 for determining whether there is another set (e.g., a second set) of cell subtypes that was identified at act 552. If it is determined that there are no other subtypes, then example implementation 508 proceeds to act 560.

Act 560 includes determining a normalization coefficient for the first cell type. In some embodiments, determining the normalization coefficient includes determining a ratio between the cell composition percentage of the first cell type and the sum of the cell composition percentages of the subtypes included in the first set. Additionally, or alternatively, a normalization coefficient may be determined in any suitable way, as aspects of the technology are not limited to any particular technique for determining a normalization coefficient.

Act 562 includes applying the determined normalization coefficient to at least some of the one or more cell composition percentages estimated for the cell subtypes included in the first set. For example, this may include multiplying a cell composition percentage with the normalization coefficient.

Example implementation then proceeds to act 564 for determining whether there is another set (e.g., a second set) of subtypes identified at act 522 for the first cell type. If there is another set, then example implementation returns to act 554 for determining a sum of the one or more cell composition percentages estimated for cell subtypes included in the next set. If there is not another set, then example implementation proceeds to act 566.

At act 566, the techniques include determining whether there are other cell types (e.g., a second cell type) for which the normalization techniques described herein may be applied. If there is another cell type, then the example implementation 508 returns to act 552. Otherwise, example implementation 508 ends.

Returning now to FIG. 5A, after normalizing the cell composition percentage(s), process 500 proceeds to act(s) 510 and/or 512. For example, one or both of acts 510 and/or 512 may be implemented as part of process 500.

Act 510 includes identifying the subject as a member of a patient cohort based on the determined cell composition percentages. In some embodiments, this may include comparing one or more cell composition percentages to those associated with a patient cohort. As a nonlimiting example, this may include comparing the percentage of a subject's T cells to the average percentage of T cells in patients who responded positively to a particular treatment. As another example, this may include comparing the percentage of a subject's CD39+ cells to the average percentage of CD39+ cells in patients who were diagnosed with a particular cancer.

In some embodiments, identifying a subject as a member of a cohort may be useful in making diagnoses, developing treatment plans, identifying effective drugs, and conducting research. However, it should be appreciated that this is a non-exhaustive list.

Act 512 includes identifying a treatment for the subject based on the determined cell composition percentages. The determined cell composition percentages may serve as biomarkers that can be used to identify treatments for the subject. For example, the cell composition percentage of peripheral blood mononuclear cells (PBMC) may serve as a biomarker for identifying ipilimumab as a treatment for subjects with HLA-DRlow Monocytes. In some embodiments, if the determined cell composition percentage of PBMCs for the subject is below a threshold value, then ipilimumab may be identified as a treatment for the subject. For example, if the cell composition percentage of PBMCs is below a threshold value of 10%, 11%, 12%, 13%, 13.05%, 13.1%, 13.5%, 14%, 15%, 16%, or a threshold value between 10% and 16%, then the ipilimumab may be identified as a treatment for the subject. As another example, the cell composition percentages of CD8+PD-1+ cells and CD4+PD-1+ cells may serve as biomarkers for identifying immune checkpoint blockade therapy for a subject with non-small cell lung cancer (NSCLC). For example, the ratio of the cell composition percentage of CD8+PD-1+ cells to CD4+PD-1+ cells may serve as such a biomarker. In some embodiments, if the ratio exceeds a threshold value, then immune checkpoint blockade therapy is identified as a treatment for the subject. For example, if the ratio exceeds a threshold value of 1.5, 1.6, 1.7, 1.8, 1.85, 1.89, 1.9, 1.91, 1.92, 1.93, 1.95, 2.0, 2.1, 2.2, 2.3, or a threshold value between 1.5 and 2.3, then immune checkpoint blockade therapy may be identified as a treatment for the subject.

Act 514 includes administering, to the subject, the treatment identified at act 514. Techniques for administering the treatment are described herein including at least in the "Methods of Treatment" section.

In some embodiments, the results of the processes described herein including at least with respect to FIGS. 2A-C and FIGS. 5A-D may be used to generate one or more visualizations or reports. FIGS. 7A-D show example visualizations and reports that may be generated as a result of these processes.

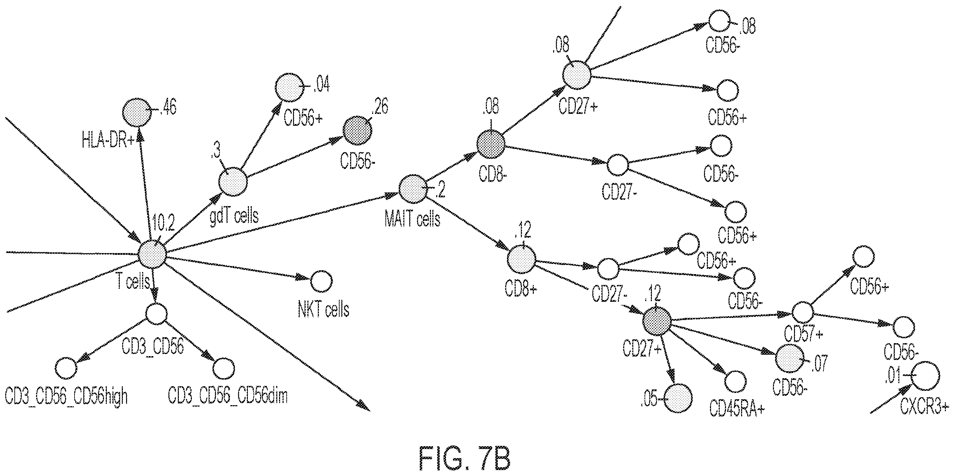

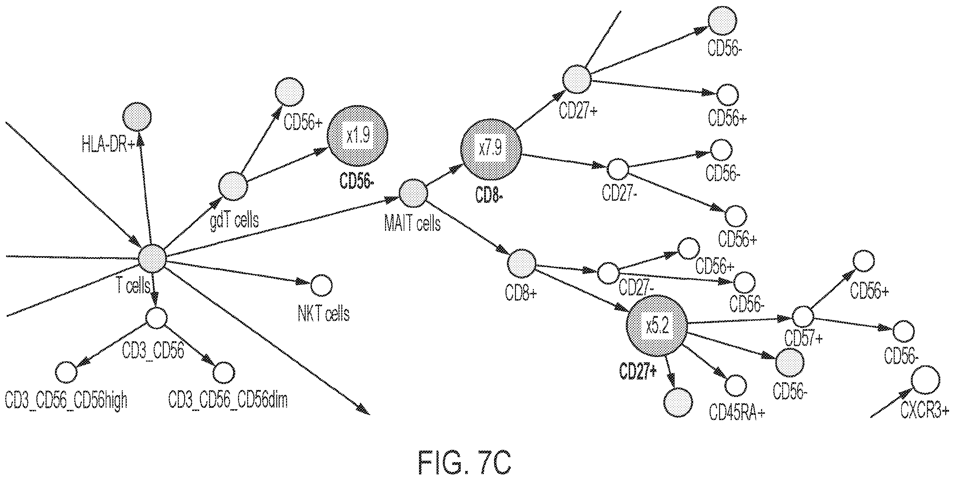

**FIG. 7A, FIG. 7B,** and **FIG. 7C** are example visualizations of cell composition percentages. In particular, they each show a graph with nodes and edges. A node represents a cell population of a particular type, while an edge represents a relationship between two nodes.

As shown, the nodes are organized and connected in a manner that shows the hierarchy of cell types, from general cell populations to more specific subtype populations. Each node is labelled with the name of the cell type, and some nodes, for which cell composition percentages are available, are labelled with the cell composition percentage (e.g., percentage or fraction).

As shown in FIG. 7A, the size of some nodes reflects the relative size (e.g., cell composition percentage) of the cell population that it represents. As shown, the leukocyte population has the largest cell composition percentage value, which is reflected by its size.

In some embodiments, the color or shading of the node may reflect information about one or more cohorts identified for the subject. For example, a node is shaded grey when the subject's cell composition percentage value is within a particular percentile bounds within a cohort. The node is shaded red if the value exceeds the upper percentile bound and is shaded blue if it is below the lower bound.

Additionally, or alternatively, in contrast to FIG. 7A, the size of some nodes may represent cell populations with abnormal cell composition percentages compared to patient cohorts. For example, FIG. 7C represents abnormal cell populations with larger nodes. The example nodes also include the exceed or reduction factor associated with that population.

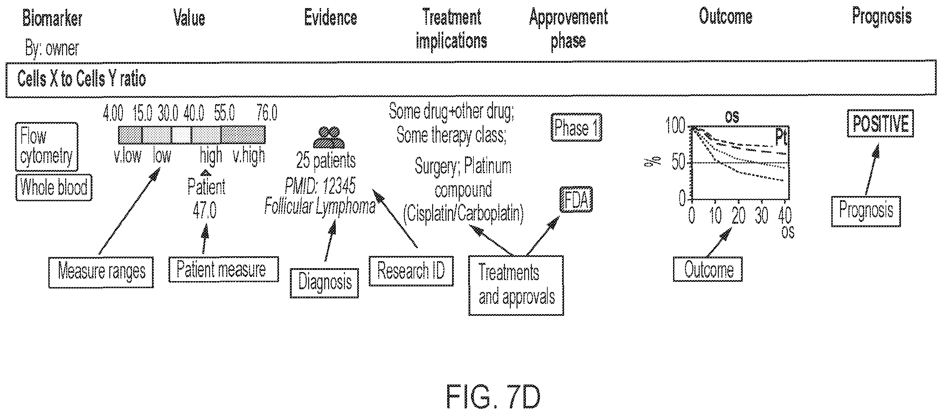

FIG. 7D is a screenshot of an example report showing the evaluation of a biomarker based on determined cell composition percentages, according to some embodiments. A biomarker is a biological measure which may affect the effectiveness of a certain treatment for patients with a certain disease. According to some embodiments, the effectiveness of a treatment is estimated using Kaplan-Meier curves showing the statistics for overall survival rate of patients along a time axis. Different curves refer to different ranges of biomarker values. According to some embodiments, by comparing the biomarker value for the patient to the different ranges of biomarker values, it is possible to predict a survival rate for the patient.

According to some embodiments, a biomarker may include one or more metrics indicative of cell composition percentages. For example, as shown in the example report, the evaluated biomarker includes the ratio of the cells of type X to the cells of type Y in a biological sample. As shown in the example, the "patient measure" refers to the numerical value (e.g., the biomarker value) of the ratio of the cells of type X to the cells of type Y in the biological sample for the patient. The "measure ranges" refer to the different ranges of biomarker values according to the Kaplan-Meier curves. As shown, the patient measure falls within the "high" measure range, indicating a high predicted survival rate.

The example report shown in FIG. 7D also indicates the diagnosis for the patient, a research ID, treatments for the diagnosis, and approval and approval phase information associated with the treatment.

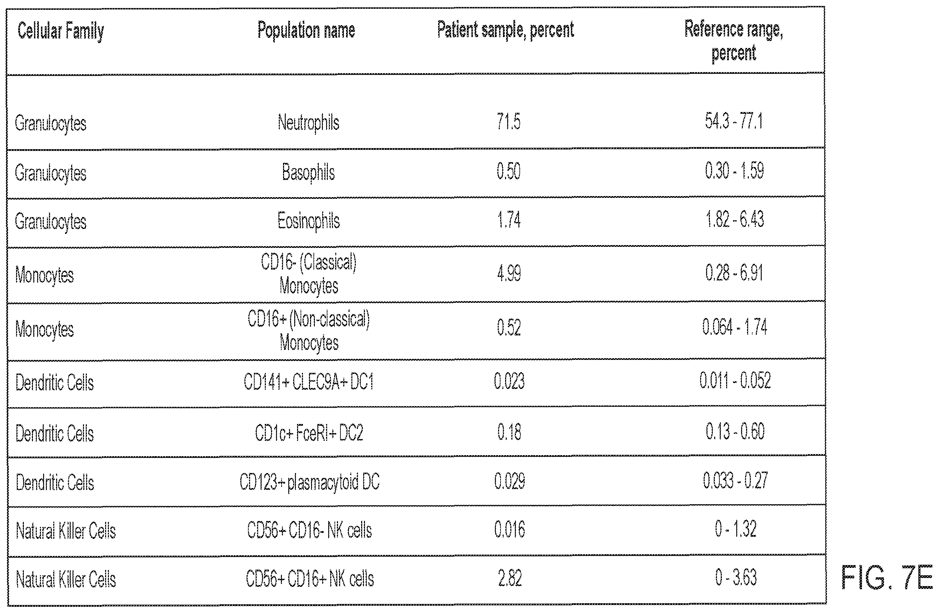

**FIG. 7E** is a screenshot of an example report indicating cell composition percentages in a biological sample from a patient. As shown, cell types are grouped based on a general cell type, or "cellular family," from which it descends. For example, neutrophils, basophils, and eosinophils belong to the granulocyte cellular family and as shown in Table 1, descend from granulocytes. Similarly, CD16- (Classical) Monocytes and CD16+ (Non-classical) Monocytes each belong to the monocytes cellular family and, as shown in Table 1, descend from monocytes.

The example table shown in FIG. 7E also shows the cell composition percentage determined for each cell type in the biological sample for the patient. For example, neutrophils comprise 71.5% of the biological sample for the patient.

The table also provides a range (e.g., upper and lower bounds) of cell composition percentages for a reference population (e.g., a reference cohort). For example, the range of cell composition percentages for neutrophils in the reference population is 54.3% to 77.1%. According to some embodiments, the reference data is determined based on a statistical analysis of a reference cohort of donors (e.g., healthy donors, donors diagnosed with a particular disease, etc.). For example, the upper and lower bounds of the range may be calculated based on the 90th and 10th percentile of the reference cohort. However, it should be appreciated that the upper and lower bounds may be determined using any suitable techniques, as aspects of the technology described herein are not limited in this respect.

In some embodiments, the report may provide an indication that a cell composition percentage for a patient falls outside the bounds of the provided range of cell composition percentages. For example, as shown in FIG. 7E, the entry for Eosinophils is shaded grey, which indicates that its cell composition percentage of 1.74% falls outside of the reference range of 1.82% to 6.43%.

Additionally, or alternatively, though not shown, the table may include two reference ranges. For example, the first reference range may indicate the range of cell composition percentages for a reference cohort of healthy donors, and the second range may indicate the range of cell composition percentages for a reference cohort of patients who are diagnosed with a particular disease.

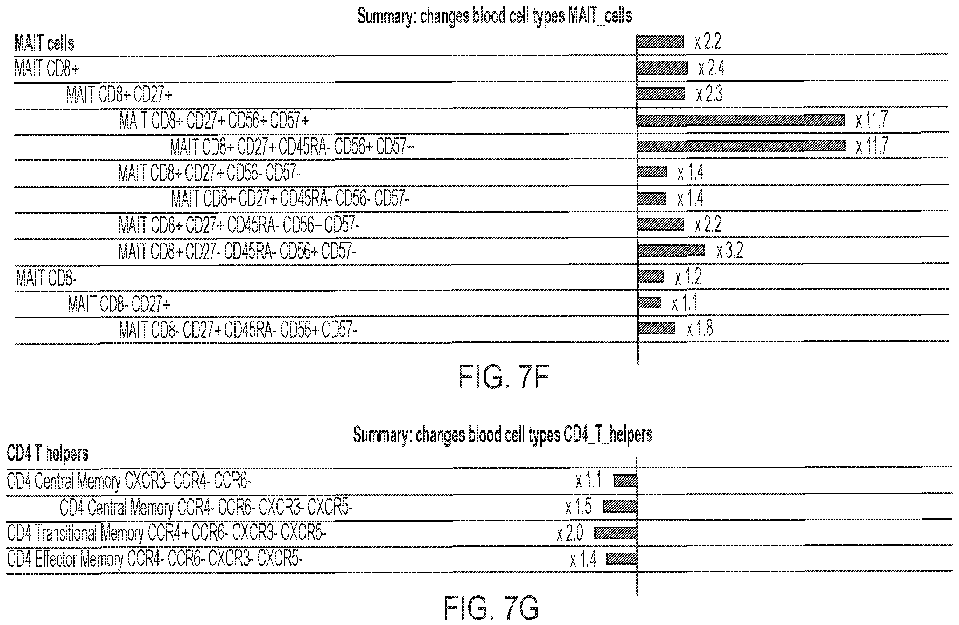

**FIG. 7F** is a screenshot of an example report showing the deviation of cell composition percentages for cell types that descend from MAIT cells. The red bar next to the label "MAIT cells" indicates that the cell composition percentage for MAIT cells in the biological sample exceeds the median for a reference cohort. For example, the reference cohort may include healthy donors or donors diagnosed with a particular disease. The number adjacent to the red bar indicates that the cell composition percentage exceeds the median of the reference cohort by a factor of 2.2.

The example report also includes cell types which descend from MAIT cells, and which also have abnormal cell composition percentages. For example, MAIT CD8+ cells descend from MAIT cells, and the cell composition percentage for MAIT CD8+ cells for the biological sample exceeds the median for a respective reference cohort by a factor of 2.4. Similarly, the cell composition percentage for MAIT CD8+ CD27+ CD45RA- CD56+ CD57+ cells, which descends from both MAIT CD8+ cells and MAIT cells, exceeds the median for a respective reference cohort by a factor of 11.7.

**FIG. 7G** is a screenshot of an example report showing the deviation of cell composition percentage values for cell types that descend from CD4 T helpers. The blue bars indicate that cell composition percentages for the listed cell types are lower than the medians for respective reference cohorts. For example, the blue bar next to the label "CD4 Central Memory CXCR3- CCR4- CCR6-," which descends from CD4 T helpers, indicates that the cell composition percentage for CD4 Central Memory CXCR3- CCR4- CCR6- is lower than the median for a respective reference cohort. The number adjacent to the blue bar indicates that the cell composition percentage is lower than the median of the reference cohort by a factor of 1.1.

*Machine Learning Model Training*

**FIG. 8** is a flowchart depicting an exemplary method for training a plurality of machine learning models. Process 800 may be performed by a laptop computer, a desktop computer, one or more servers, in a cloud computing environment, computing device 108 as described herein with respect to FIG. 1D, computing device 1000 as described herein with respect to FIG. 10, or in any other suitable way.

Process 800 begins at act 802, where cytometry data is obtained for each of a plurality of cells. In some embodiments, the cytometry data may be obtained in any suitable way, as aspects of the technology described herein are not limited to any particular technique for obtaining cytometry data. For example, this may include obtaining the cytometry data by processing one or more biological samples. As another example, this may include obtaining cytometry data from one or more data stores and/or storage devices.

Act 804 includes obtaining corresponding cell type data for each of at least some of the plurality of cells for which cytometry data was obtained. In some embodiments, act 804 includes sub-act 804*a* and sub-act 804*b*.

Sub-act 804*a* includes obtaining a first type for a first cell for which cytometry data was obtained. In some embodiments, this includes processing the obtained cytometry data using gating and/or clustering techniques, such as those described herein including at least with respect to "Training Data". In other embodiments, obtaining the first type for the first cell may include accessing data from one or more data stores storing information indicating the types for cells for which cytometry data was obtained.

Sub-act 804b includes extracting, from a hierarchy of cell types, one or more cell types related to the first type. In some embodiments, this may include determining one or more subtypes of the first type and one or more "parent" types of the first types. For example, consider a first cell that was determined to be a T cell at sub-act 804a. Sub-act 804b may include extracting cell types related to T cells, such as lymphocytes, leukocytes, memory T cells, to name a few examples. In some embodiments, sub-act 804b may include accessing a hierarchy of cell types (e.g., from a data store), such as the hierarchy of cell types described with respect to Table 1.

Act 806 includes training a machine learning model of a plurality of machine learning models to determine whether a cell is of a particular type using the cytometry data and the cell type data. In some embodiments, the obtained data may be split into train, test, and validation data sets for training, testing, and validating each machine learning model. In some embodiments, the training data may be processed according to the techniques described herein including at least with respect to act 204 of process 200, prior to being used for training, testing, or validating the machine learning model.

In some embodiments, act 806 may include training a machine learning model for each of at least some of the cell types obtained at act 804. Sub-act 806a includes training a first machine learning model to determine whether the cell is of a first type using first cytometry data and first cell type data, or "first obtained data". For example, sub-act 806a may include training a machine learning model to determine whether a cell is a T cell.

In some embodiments, the first obtained data may include "positive" data and "negative" data. In some embodiments positive data may include data for cells that should be positively identified as the first cell type using the first machine learning model, while negative data may include data for cells that should not be identified as the first cell type using the first machine learning model.

In some embodiments, positive data may include cytometry data for cells that are determined to be of the first type and/or for cells which are determined to be of a type related to the first type. For example, if the first type is a T cell, then the positive data may include cytometry data for cells determined to be T cells. Additionally, or alternatively, if the first type is a T cell, then the positive data may include cytometry data for cells determined to be lymphocytes, leukocytes, memory T cells, or any other type related to the first cell type. In this example, the related cell types include parent cell types and/or subtypes of the first cell type. Parent cell types and subtypes of the first cell type may be positioned at different levels of the cell hierarchy than the first cell type.

In some embodiments, negative data may include cytometry data for cells that are of types at a same level of the cell hierarchy and/or subtypes of cell types at the same level of the cell hierarchy. For example, if the first cell type is a T cell, then cell types at the same level of the cell hierarchy may include B cells, while subtypes of the cell type at the same level may include memory B cells. Additionally, or alternatively, negative data may include cytometry data for cells for which a type cannot be identified. In other embodiments, the data may not include any negative training data for the first cell type.

In some embodiments, the first cytometry data includes values for particular markers obtained for the cells. In some embodiments, the markers may be selected based on the first cell type for which the first machine learning model is being trained. For example, if the first machine learning model is being trained to determine whether the cell is a T cell, then the first cytometry data may include values of one or more particular markers useful for distinguishing T cells. Examples of markers for different cell types are shown in Table 2.

According to some embodiments, training the first machine learning model on all samples in the training data may use significant computational resources, since all the training data is stored in RAM during training. In some cases, there may be insufficient RAM, which can lead to the interruption of training the first machine learning model and the loss of training results.

Accordingly, in some embodiments, the first machine learning model is trained using batches of training data. Batches may be obtained by dividing the training data into batches of a specified number of samples. For example, a batch may consist of a relatively small number of samples (e.g., 6 sample, 8 samples, 10 samples, 12 samples, 14 samples, etc.). In some embodiments, the batches do not overlap, and their union is equivalent to the original set of samples.

In some embodiments, training the first machine learning model using the batches of training data includes training the first machine learning model on each of at least some of the batches. Accordingly, only a relatively small number of samples (e.g., the number of samples in a batch) is stored in RAM during training, which avoids issues associated with RAM shortage.

Act 806b includes determining whether there is another machine learning model of the plurality of machine learning models that should be trained to determine another cell type. For example, this may include determining whether there is a second machine learning model to be trained to determine whether a cell if of a second type. If it is determined that there is another machine learning model at act 806b, then process 800 returns to act 806a for training the next machine learning model. In some embodiments, any number of machine learning models may be trained according to the techniques described herein, as aspects of the technology described herein are not limited to any particular number of machine learning models.

If at act 806b, there is not another machine learning model to be trained, then process 800 ends.

**Table 2.** Markers corresponding to particular cell types.

| Cell type | Parent cell type | Markers |
|---|---|---|
| Leukocytes | | |
| Granulocytes | Leukocytes | |
| Eosinophils | Granulocytes | CD45+ CD3- CD19- CD14- CD56- CD13+ CD66b+ CCR3- |
| Neutrophils | Granulocytes | CD45+ CD3- CD19- CD14- CD56- CD13+ CD66b+ CCR3+ |
| Basophils | Granulocytes | CD45+ CD3- CD19- CD14- CD56- CD13+ CD66b+ CCR3+ CD123+ |
| Monocytes | Leukocytes | CD45+ CD3- CD19- CD7- CD15- HLA-DR+ CD33+ CD14+ |
| Classical monocytes | Monocytes | CD45+ CD3- CD19- CD7- CD15- HLA-DR+ CD33+ CD14+ CD16- |
| Classical monocytes FceRI+ | Classical monocytes | CD45+ CD3- CD19- CD7- CD15- HLA-DR+ CD33+ CD14+ CD16- FceRI+ |
| Classical monocytes FceRI- | Classical monocytes | CD45+ CD3- CD19- CD7- CD15- HLA-DR+ CD33+ CD14+ CD16- FceRI- |
| Non-classical monocytes | Monocytes | CD45+ CD3- CD19- CD7- CD15- HLA-DR+ CD33+ CD14lo CD16+ |
| Dendritic cells | Leukocytes | CD45+ CD3- CD19- CD14- CD16- HLA-DR+ |
| cDC | Dendritic cells | CD45+ CD3- CD19- CD14- CD16- HLA-DR+ CD11c+ |
| cDC1 | cDC | CD45+ CD3- CD19- CD14- CD16- HLA-DR+ CD141+ CLEC9A+ |
| cDC2 | cDC | CD45+ CD3- CD19- CD14- CD16- CD13+ CD123- HLA-DR+ CD1c+ FceRI+ |
| Plasmacytoid dendritic cells | Dendritic cells | CD45+ CD3- CD19- CD14- CD16- HLA-DR+ FceRI+ CD123+ |
| Lymphocytes | Leukocytes | |
| B cells | Lymphocytes | CD3- CD14- CD15- CD56- CD19+ |
| Naïve B cells | B cells | CD3- CD14- CD15- CD56- CD19+ IgD+ CD27- |
| Memory B cells | B cells | CD3- CD14- CD15- CD56- CD19+ CD27+ |
| Non-switched Memory IgM B cells | Memory B cells | CD3- CD14- CD15- CD56- CD19+ IgD+ CD27+ |
| Class-switched Memory | Memory B cells | CD3- CD14- CD15- CD56- CD19+ IgD- CD27+ |
| Switched Memory IgG+ | Class-switched Memory | CD3- CD14- CD15- CD56- CD19+ IgD- CD27+ IgG+ |
| Switched Memory IgA+ | Class-switched Memory | CD3- CD14- CD15- CD56- CD19+ IgD- CD27+ IgA+ |
| Secreting abs B cells | B cells | CD3- CD14- CD15- CD56- CD19+ IgD- CD27+ CD38+ |
| Plasmablasts | Secreting abs B cells | CD3- CD14- CD15- CD56- CD19+ IgD- CD27+ CD38+ CD138- |
| Plasmablasts IgA+ | Plasmablasts | CD3- CD14- CD15- CD56- CD19+ IgD- CD27+ CD38+ CD138- IgA+ |
| Plasmablasts IgG+ | Plasmablasts | CD3- CD14- CD15- CD56- CD19+ IgD- CD27+ CD38+ CD138- IgG+ |
| Plasma cells | Secreting abs B cells | CD3- CD14- CD15- CD56- CD19+ IgD- CD27+ CD138+ |

(continued)

| Cell type | Parent cell type | Markers |
|---|---|---|
| Plasma cells IgA+ | Plasma cells | CD3- CD14- CD15- CD56- CD19+ IgD- CD27+ CD138+ IgA+ |
| Plasma cells IgG+ | Plasma cells | CD3- CD14- CD15- CD56- CD19+ IgD- CD27+ CD138+ IgG+ |
| NK cells | Lymphocytes | CD45+ CD3- CD19- CD14- CD56+ |
| Immature NK cells | NK cells | CD45+ CD3- CD19- CD14- CD56+ CD16- |
| Mature NK cells | NK cells | CD45+ CD3- CD19- CD14- CD56+ CD16+ |
| Mature CD158+ | Mature NK cells | CD45+ CD3- CD19- CD14- CD56+ CD16+ CD158+ |
| Mature NK CD158+ CD57+ | Mature CD158+ | CD45+ CD3- CD19- CD14- CD56+ CD16+ CD158+ CD57+ |
| Mature CD158- | Mature NK cells | CD45+ CD3- CD19- CD14- CD56+ CD16+ CD158- |
| T cells | Lymphocytes | CD19- CD14- CD15- CD3+ |
| NKT cells | T cells | CD19- CD14- CD15- CD3+ CD56+ |
| HLA-DR T cells | T cells | CD19- CD14- CD15- CD3+ HLA-DR+ |
| gdT cells | T cells | CD19- CD14- CD15- CD3+ TCR gamma delta (11F2)+ |
| iNKT | T cells | CD19- CD14- CD15- CD3+ TCR Valpha24-Jalpha18 (6B11)+ |
| MAIT cells | T cells | **CD19- CD14- CD15- CD3+ TCR V-alpha 7.2+ CD161+** |
| MAIT CD8+ | MAIT cells | CD19- CD14- CD15- CD3+ TCR V-alpha 7.2+ CD161+ CD8+ |
| MAIT CD8- | MAIT cells | CD19- CD14- CD15- CD3+ TCR V-alpha 7.2+ CD161+ CD8- |
| CD4 T cells | T cells | CD19- CD14- CD15- TCRgd- CD4+ CD3+ CD8- |
| CD4 Tregs | CD4 T cells | **CD19- CD14- CD15- TCRgd- CD4+ CD3+ CD8-IL7-RAlo CD25+** |
| CD4 Naive Tregs | CD4 Tregs | CD19- CD14- CD15- TCRgd- CD4+ CD3+ CD8-IL7-RAlo CD25+ CD27+ CD45RA+ CD62L+ |
| CD4 Memory Tregs | CD4 Tregs | CD19- CD14- CD15- TCRgd- CD4+ CD3+ CD8-IL7-RAlo CD25+ CD45RA- |
| CD4 T helpers | CD4 T cells | CD19- CD14- CD15- TCRgd- CD4+ CD3+ CD8-not(IL7RAlo CD25+) |
| CD4 Naive T cells | CD4 T helpers | CD19- CD14- CD15- TCRgd- CD4+ CD3+ CD8-CD27+ CD45RA+ CD62L+ not(IL7RAlo CD25+) |
| CD4 Memory T helpers | CD4 T helpers | CD19- CD14- CD15- TCRgd- CD4+ CD3+ CD8-not(CD27+ CD45RA+ CD62L+) not(IL7RAlo CD25+) |
| CD4 Central Memory | CD4 Memory T helpers | CD19- CD14- CD15- TCRgd- CD4+ CD3+ CD8-CD27+ CD45RA- CD62L+ not(IL7RAlo CD25+) |
| CD4 Central Memory CCR4-CCR6- CXCR3+ CXCR5- | CD4 Central Memory | CD19- CD14- CD15- TCRgd- CD4+ CD3+ CD8-CD27+ CD45RA- CD62L+ not(IL7RAlo CD25+) CCR4- CCR6- CXCR3+ CXCR5- |
| CD4 Central Memory CCR4+ CCR6+ CXCR3-CXCR5- | CD4 Central Memory | CD19- CD14- CD15- TCRgd- CD4+ CD3+ CD8-CD27+ CD45RA- CD62L+ not(IL7RAlo CD25+) CCR4+ CCR6+ CXCR3- CXCR5- |

(continued)

| Cell type | Parent cell type | Markers |
|---|---|---|
| CD4 Central Memory CCR4+ CCR6- CXCR3-CXCR5- | CD4 Central Memory | CD19- CD14- CD15- TCRgd- CD4+ CD3+ CD8- CD27+ CD45RA- CD62L+ not(IL7RAlo CD25+) CCR4+ CCR6- CXCR3- CXCR5- |
| CD4 Transitional Memory | CD4 Memory T helpers | CD19- CD14- CD15- TCRgd- CD4+ CD3+ CD8- CD27+ CD45RA- CD62L- not(IL7RAlo CD25+) |
| CD4 Transitional Memory CCR4- CCR6- CXCR3+ CXCR5- | CD4 Transitional Memory | CD19- CD14- CD15- TCRgd- CD4+ CD3+ CD8- CD27+ CD45RA- CD62L- not(IL7RAlo CD25+) CCR4- CCR6- CXCR3+ CXCR5- |
| CD4 Transitional Memory CCR4+ CCR6+ CXCR3- CXCR5- | CD4 Transitional Memory | CD19- CD14- CD15- TCRgd- CD4+ CD3+ CD8- CD27+ CD45RA- CD62L- not(IL7RAlo CD25+) CCR4+ CCR6+ CXCR3- CXCR5- |
| CD4 Transitional Memory CCR4+ CCR6- CXCR3- CXCR5- | CD4 Transitional Memory | CD19- CD14- CD15- TCRgd- CD4+ CD3+ CD8- CD27+ CD45RA- CD62L- not(IL7RAlo CD25+) CCR4+ CCR6- CXCR3- CXCR5- |
| CD4 Effector Memory | CD4 Memory T helpers | CD19- CD14- CD15- TCRgd- CD4+ CD3+ CD8-CD27- CD45RA- CD62L- not(IL7RAlo CD25+) |
| CD4 Effector Memory CCR4+ CCR6+ CXCR3-CXCR5- | CD4 Effector Memory | CD19- CD14- CD15- TCRgd- CD4+ CD3+ CD8-CD27- CD45RA- CD62L- not(IL7RAlo CD25+) CCR4+ CCR6+ CXCR3- CXCR5- |
| CD4 Effector Memory | CD4 Effector | CD19- CD14- CD15- TCRgd- CD4+ CD3+ CD8- |
| CCR4+ CCR6- CXCR3- CXCR5- | Memory | CD27- CD45RA- CD62L- not(IL7RAlo CD25+) CCR4+ CCR6- CXCR3- CXCR5- |
| CD4 Effector Memory CCR4- CCR6- CXCR3+ CXCR5- | CD4 Effector Memory | CD19- CD14- CD15- TCRgd- CD4+ CD3+ CD8-CD27- CD45RA- CD62L- not(IL7RAlo CD25+) CCR4-CCR6- CXCR3+ CXCR5- |
| CD4 TEMRA | CD4 Memory T helpers | CD19- CD14- CD15- TCRgd- CD4+ CD3+ CD8-CD27- CD45RA+ CD62L- not(IL7RAlo CD25+) |
| CD4 Memory Tregs CD39+ | CD4 Memory Tregs | CD19- CD14- CD15- TCRgd- CD4+ CD3+ CD8-IL7-RAlo CD25+ CD45RA- CD39+ |
| CD4 Memory Tregs CD39- | CD4 Memory Tregs | CD19- CD14- CD15- TCRgd- CD4+ CD3+ CD8-IL7-RAlo CD25+ CD45RA- CD39- |
| CD4 Memory Tregs CD39+ ICOS+ | CD4 Memory Tregs CD39+ | CD19- CD14- CD15- TCRgd- CD4+ CD3+ CD8-IL7-RAlo CD25+ CD45RA- CD39+ ICOS+ |
| CD4 Memory CD39+ | CD4 Memory T helpers | CD19- CD14- CD15- TCRgd- CD4+ CD3+ CD8-not(IL7RAlo CD25+) CD45RA- CD39+ |
| CD4 Memory CD39- | CD4 Memory T helpers | CD19- CD14- CD15- TCRgd- CD4+ CD3+ CD8-not(IL7RAlo CD25+) CD45RA- CD39- |
| CD8 T cells | T cells | CD19- CD14- CD15- TCRgd- CD4- CD3+ CD8+ |
| CD8 Naive T cells | CD8 T cells | CD19- CD14- CD15- TCRgd- CD4- CD3+ CD8+ CD27+ CD45RA+ CD62L+ |
| CD8 Stem Cell Memory CD57- CD95+ | CD8 Naive T cells | CD19- CD14- CD15- TCRgd- CD4- CD3+ CD8+ CD27+ CD45RA+ CD62L+ CD95+ CD57- |
| CD8 True Naive T cells | CD8 Naive T cells | CD19- CD14- CD15- TCRgd- CD4- CD3+ CD8+ CD27+ CD45RA+ CD62L+ CD95- CD57- |
| CD8 Memory T cells | CD8 T cells | CD19- CD14- CD15- TCRgd- CD4- CD3+ CD8+ not(CD27+ CD45RA+ CD62L+) |

(continued)

| Cell type | Parent cell type | Markers |
|---|---|---|
| CD8 Central Memory | CD8 Memory T cells | CD19- CD14- CD15- TCRgd- CD4- CD3+ CD8+ CD27+ CD45RA- CD62L+ |
| CD8 Transitional Memory | CD8 Memory T cells | CD19- CD14- CD15- TCRgd- CD4- CD3+ CD8+ CD27+ CD45RA- CD62L- |
| CD8 Effector Memory | CD8 Memory T cells | CD19- CD14- CD15- TCRgd- CD4- CD3+ CD8+ CD27- CD45RA- CD62L- |
| CD8 TEMRA | CD8 Memory T cells | CD19- CD14- CD15- TCRgd- CD4- CD3+ CD8+ CD27- CD45RA+ CD62L- |
| CD8 Central Memory PD-1+ | CD8 Central Memory | CD19- CD14- CD15- TCRgd- CD4- CD3+ CD8+ CD27+ CD45RA- CD62L+ PD-1+ |
| CD8 Central Memory PD-1- | CD8 Central Memory | CD19- CD14- CD15- TCRgd- CD4- CD3+ CD8+ CD27+ CD45RA- CD62L+ PD-1- |
| CD8 Central Memory PD-1+ CD39+ | CD8 Central Memory PD-1+ | CD19- CD14- CD15- TCRgd- CD4- CD3+ CD8+ CD27+ CD45RA- CD62L+ PD-1+ CD39+ |
| CD8 Effector Memory PD-1+ | CD8 Effector Memory | CD19- CD14- CD15- TCRgd- CD4- CD3+ CD8+ CD27- CD45RA- CD62L- PD-1+ |
| CD8 Effector Memory PD-1- | CD8 Effector Memory | CD19- CD14- CD15- TCRgd- CD4- CD3+ CD8+ CD27- CD45RA- CD62L- PD-1- |
| CD8 Effector Memory PD-1+ CD39+ | CD8 Effector Memory PD-1+ | CD19- CD14- CD15- TCRgd- CD4- CD3+ CD8+ CD27- CD45RA- CD62L- PD-1+ CD39+ |
| CD8 Transitional Memory PD-1+ | CD8 Transitional Memory | CD19- CD14- CD15- TCRgd- CD4- CD3+ CD8+ CD27+ CD45RA- CD62L- PD-1+ |
| CD8 Transitional Memory PD-1- | CD8 Transitional Memory | CD19- CD14- CD15- TCRgd- CD4- CD3+ CD8+ CD27+ CD45RA- CD62L- PD-1- |
| CD8 Transitional Memory PD-1+ CD39+ | CD8 Transitional Memory PD-1+ | CD19- CD14- CD15- TCRgd- CD4- CD3+ CD8+ CD27+ CD45RA- CD62L- PD-1+ CD39+ |
| CD8 TEMRA PD-1+ | CD8 TEMRA | CD19- CD14- CD15- TCRgd- CD4- CD3+ CD8+ CD27- CD45RA+ CD62L- PD-1+ |
| CD8 TEMRA PD-1- | CD8 TEMRA | CD19- CD14- CD15- TCRgd- CD4- CD3+ CD8+ CD27- CD45RA+ CD62L- PD-1- |
| CD8 TEMRA PD-1+ CD39+ | CD8 TEMRA PD-1+ | CD19- CD14- CD15- TCRgd- CD4- CD3+ CD8+ CD27- CD45RA+ CD62L- PD-1+ CD39+ |
| CD8 Central Memory CD57+ | CD8 Central Memory | CD19- CD14- CD15- TCRgd- CD4- CD3+ CD8+ CD27+ CD45RA- CD62L+ CD57+ |
| CD8 Central Memory CD57- | CD8 Central Memory | CD19- CD14- CD15- TCRgd- CD4- CD3+ CD8+ CD27+ CD45RA- CD62L+ CD57- |
| CD8 Effector Memory CD57+ CD95+ | CD8 Effector Memory | CD19- CD14- CD15- TCRgd- CD4- CD3+ CD8+ CD27- CD45RA- CD62L- CD57+ |
| CD8 Effector Memory CD57- CD95+ | CD8 Effector Memory | CD19- CD14- CD15- TCRgd- CD4- CD3+ CD8+ CD27- CD45RA- CD62L- CD57- |
| CD8 Effector Memory CD57+ CD95+ CX3CR1+ | CD8 Effector Memory CD57+ CD95+ | CD19- CD14- CD15- TCRgd- CD4- CD3+ CD8+ CD27- CD45RA- CD62L- CD57+ CX3CR1+ |
| CD8 Effector Memory CD57- CD95+ CX3CR1- | CD8 Effector Memory CD57-CD95+ | CD19- CD14- CD15- TCRgd- CD4- CD3+ CD8+ CD27- CD45RA- CD62L- CD57- CX3CR1- |
| CD8 Transitional Memory CD57+ | CD8 Transitional Memory | CD19- CD14- CD15- TCRgd- CD4- CD3+ CD8+ CD27+ CD45RA- CD62L- CD57+ |

(continued)

| Cell type | Parent cell type | Markers |
|---|---|---|
| CD8 Transitional Memory CD57- | CD8 Transitional Memory | CD19- CD14- CD15- TCRgd- CD4- CD3+ CD8+ CD27+ CD45RA- CD62L- CD57- |
| CD8 TEMRA CD57+ | CD8 TEMRA | CD19- CD14- CD15- TCRgd- CD4- CD3+ CD8+ CD27- CD45RA+ CD62L- CD57+ |
| CD8 TEMRA CD57- | CD8 TEMRA | CD19- CD14- CD15- TCRgd- CD4- CD3+ CD8+ CD27- CD45RA+ CD62L- CD57- |

Training Data

As described herein, including with respect to FIG. 8, training the plurality of machine learning models includes obtaining training data including cytometry data for cells and the corresponding cell types. In some embodiments, obtaining the training data includes obtaining cytometry data for one or more biological samples and manually processing the cytometry data to determine types for cells in the biological sample.

In some embodiments, processing the cytometry data may include gating the cytometry data. For example, this may include manually gating the cytometry data to separate discrete cell populations based on shared marker expression. In some embodiments, gating may be performed using any suitable gating techniques, such as by using FlowJo™ (FlowJo™ Software. Ashland, OR: Beckton, Dickinson and Company; 2021).

In some embodiments, gating may result in a file (e.g., a Workspace (WSP) file) that includes any suitable information about the gating, such as information about the coordinates of the gates, axes transformation, statistics, and layouts.

In some embodiments, processing the cytometry data may additionally or alternatively include clustering the cytometry data for a sample. This may include calculating two-dimensional t-SNE plots for a sample and calculating FlowSOM for the sample. FlowSOM is described by Van Gassen et al. ("FlowSOM: Using self-organizing maps for visualization and interpretation of cytometry data," in Journal of Quantitative Cell Science, vol. 87, no. 7, pp. 636-645, 2015).

Prior to clustering, in some embodiments, processing the cytometry data may include a noise transformation of the cytometry data. This may include transforming the intensity of the markers to reduce the influence of noise on the clustering results. In some embodiments, transforming the intensity of a marker includes reducing the intensity of the marker lower than a specified border. Such a border may be identified based a result of gating or using Fluorescence Minus One controls. In some embodiments, a border is defined as a border between a positive signal from a marker and the intensity of noise in the channel of the marker. Equations 4 and 5 describe the intensity of a marker after the noise transformation ($I_{after\ transform}$):

$$I_{after\ transform} = I_{initial}, if\ I_{initial} \geq border \qquad \text{(Equation 4)}$$

$$I_{after\ transform} = \frac{I_{initial}}{k}, if\ I_{initial} < border \qquad \text{(Equation 5)}$$

where $I_{initial}$ is the initial intensity of the marker from the cytometry data, *border* is the border of reduction for the intensity of the marker, and *k* is the coefficient of reduction. In some embodiments, the coefficient of reductions is a constant, user-defined value. In some embodiments, the coefficient of reduction linearly increases from 1 at the border value to a user-defined maximum value at the minimum intensity of the marker.

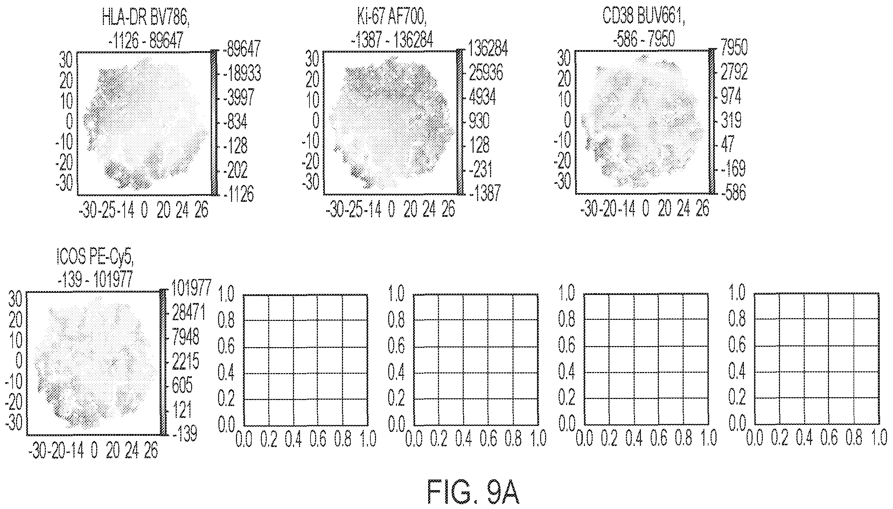

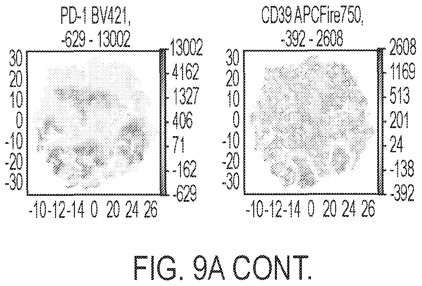

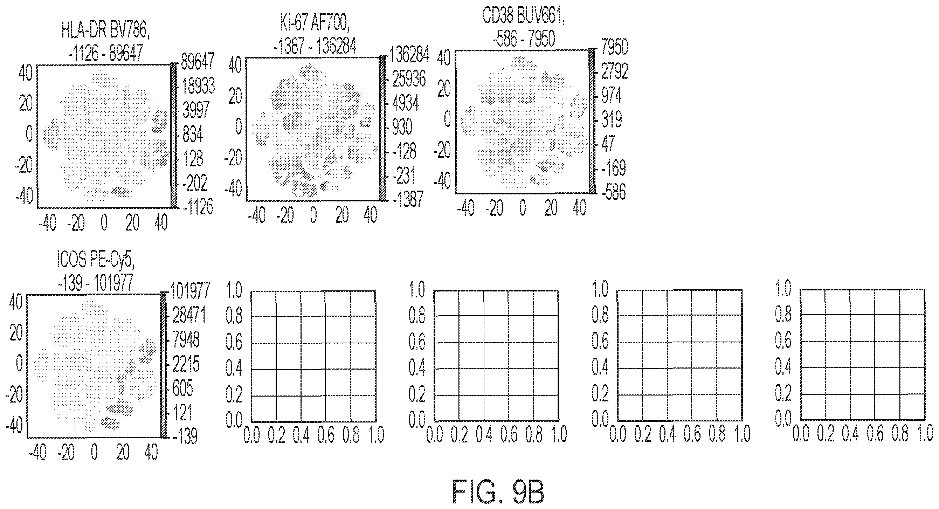

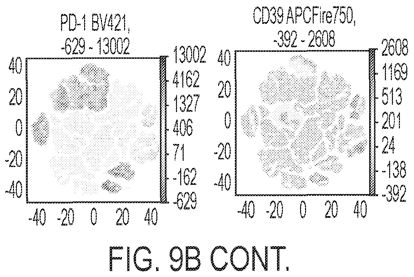

**FIGS. 9A-9B** show the difference between clustered cytometry data before the noise transformation and after the noise transformation. As shown in FIG. 9B, the clusters are more distinct from one another after the noise transformation.

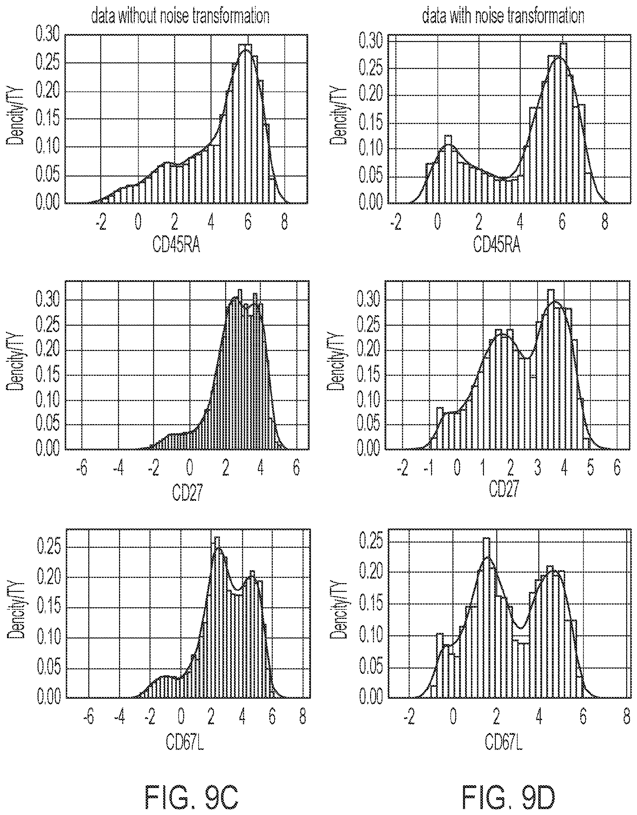

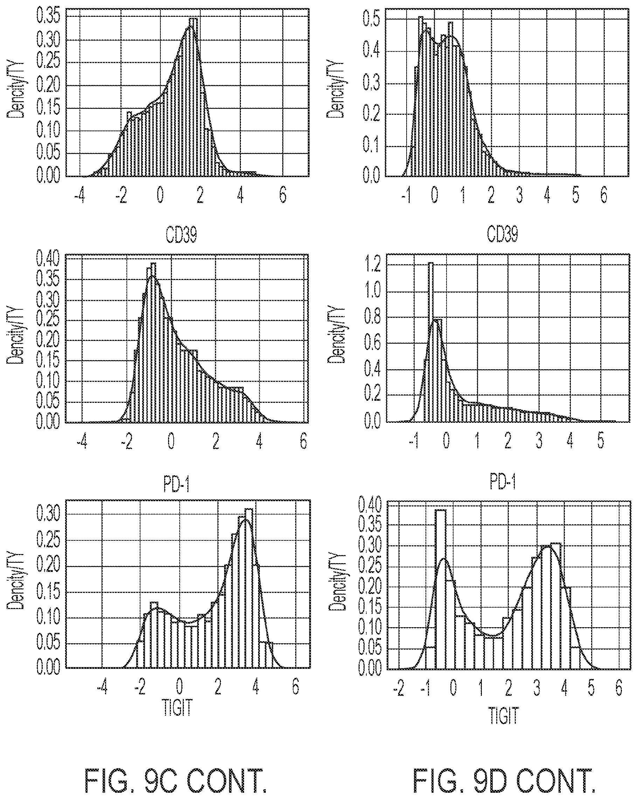

**FIGS. 9C-9D** show the difference between the distribution of marker intensities before the noise transformation and after the noise transformation. As shown in FIG. 9D, the distributions of marker after the noise transformation more closely resemble bimodal distributions.

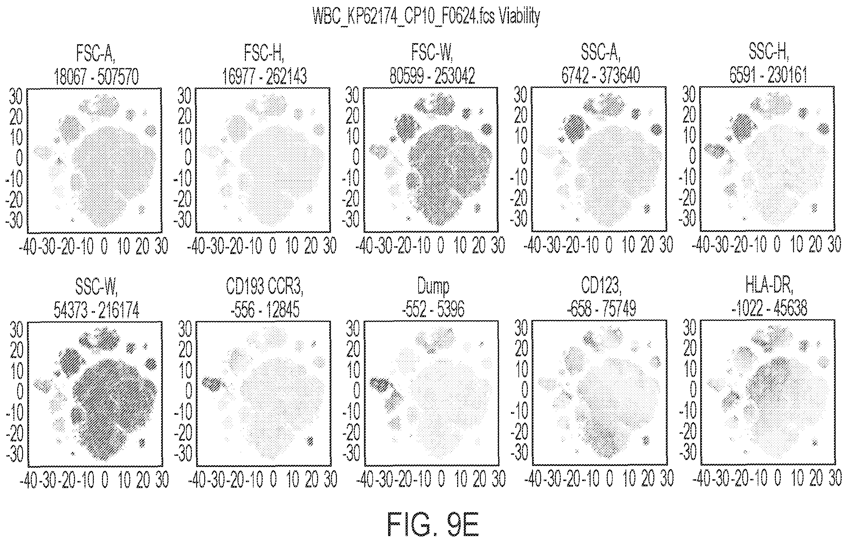

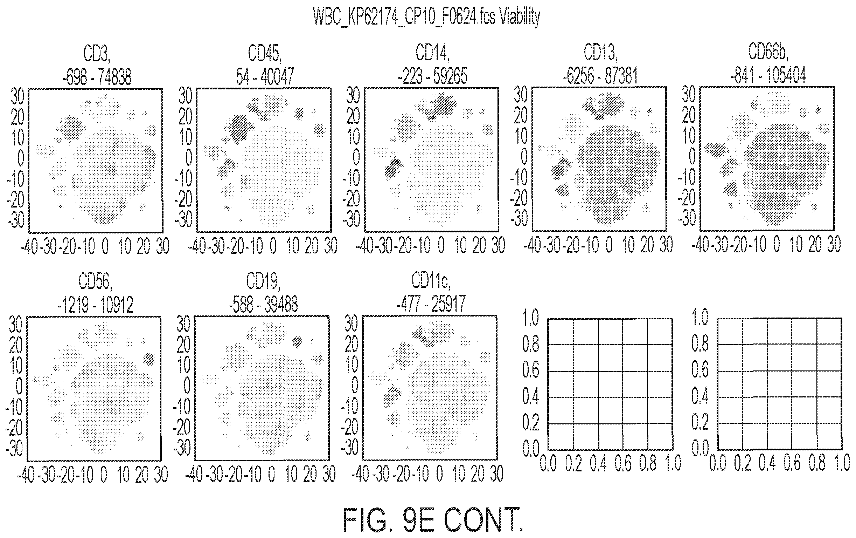

Regardless of whether the noise transformation techniques are used, after clustering, the techniques may include plotting t-SNE multiplot with the intensity of markers and scatter light. Example plots are shown in **FIG. 9E.** In some embodiments, each point may correspond with the value of a cell, particle, or debris for which cytometry data was obtained. In some embodiments, the plots may be used to identify different clusters, which may correspond to populations of cells, particles, or debris.

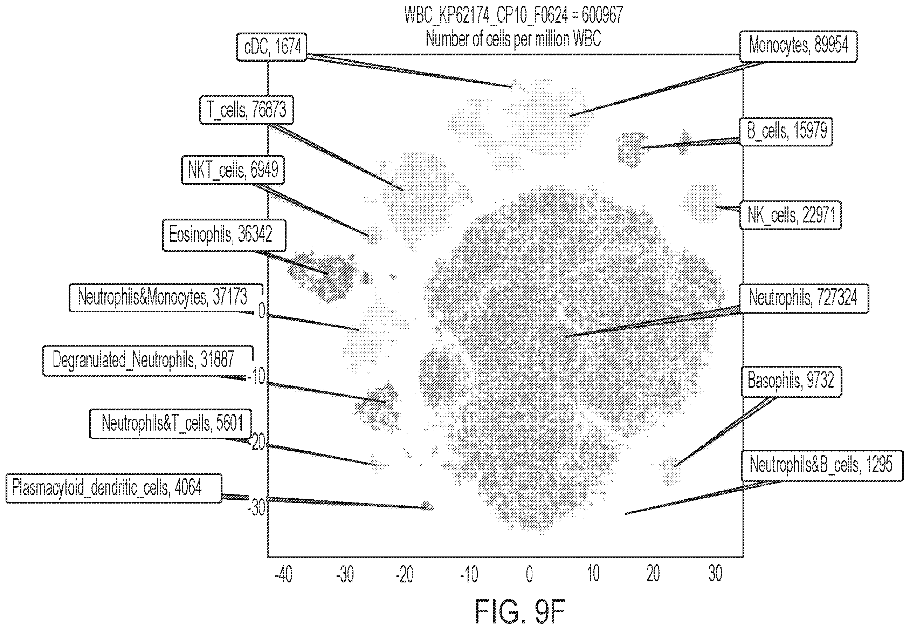

In some embodiments, a user may manually label the clusters with a corresponding cell type. For example, as shown in **FIG. 9F,** different clusters are labeled with different cell types. Points within each labeled cluster may correspond to a particular cell, particle, or debris in the cytometry data.

In some embodiments, an automatic labeling algorithm is used to label the clusters with corresponding cell types. A label may be selected based on the positive or negative signals from the specified markers. A positive signal from a marker is when the intensity of the marker is greater than, or equal to, the border. A negative signal from the marker is when the intensity of the marker is less than the border. As described above, the border for the intensity of a marker may be obtained from gating or from Fluorescence Minus One controls. The border is the border between the positive signal from the marker and the intensity of noise in the channel of the marker.

In some embodiments, the techniques may further include discarding some of the identified clusters. For example, clusters corresponding to debris and/or particles may be discarded. In some embodiments, the steps for calculating and plotting the t-SNE plots and for labelling the clusters may be repeated without the discarded clusters.

While various techniques for processing cytometry data have been described, it should be appreciated that any suitable techniques may be used to process such data, as aspects of the technology described herein are not limited in this respect.

*Machine Learning*

In some embodiments, the machine learning model may include a decision tree classifier, a gradient boosted decision tree classifier, a neural network, a support vector machine classifier, or any other suitable type of machine learning model, as aspects of the technology described herein are not limited in this respect. In some embodiments, the machine learning model may include an ensemble of machine learning models of any suitable type (the machine learning models part of the ensemble may be termed "weak learners"). For example, the machine learning model may include an ensemble of decision tree classifiers.

As described above, in some embodiments, the machine learning model may be implemented as a decision tree classifier. Any suitable type of decision tree classifier may be used and may be trained using any suitable supervised decision tree learning technique. For example, the decision tree classifier may be trained by the iterative dichotomiser technique (e.g., the ID3 algorithm as described, for example, in Quinlan, J. R. 1986. Induction of Decision Trees. Mach. Learn. 1, 1 (Mar. 1986), 81-106)), the C4.5 technique (e.g., as described, for example, in Quinlan, J. R. C4.5: Programs for Machine Learning. Morgan Kaufmann Publishers, 1993), the classification and regression tree (CART) technique (e.g., as described, for example, in Breiman, Leo; Friedman, J. H.; Olshen, R. A.; Stone, C. J. (1984). Classification and regression trees. Monterey, CA: Wadsworth & Brooks/Cole Advanced Books & Software). It should be appreciated that a decision tree classifier may be trained using any other suitable training method, as aspects of the technology described herein are not limited in this respect.

In some embodiments, a gradient-boosted decision tree classifier may be used. The gradient-boosted decision tree classifier may be an ensemble of multiple decision tree classifiers (sometimes called "weak learners"). The prediction (e.g., classification) generated by the gradient-boosted decision tree classifier is formed based on the predictions generated by the multiple decision trees part of the ensemble. The ensemble may be trained using an iterative optimization technique involving calculation of gradients of a loss function (hence the name "gradient" boosting). Any suitable supervised training algorithm may be applied to training a gradient-boosted decision tree classifier including, for example, any of the algorithms described in Hastie, T.; Tibshirani, R.; Friedman, J. H. (2009). "10. Boosting and Additive Trees". The Elements of Statistical Learning (2nd ed.). New York: Springer. pp. 337-384. In some embodiments, the gradient-boosted decision tree classifier may be implemented using any suitable publicly-available gradient boosting framework such as XGBoost (e.g., as described, for example, in Chen, T., & Guestrin, C. (2016). XGBoost: A Scalable Tree Boosting System. In Proceedings of the 22nd ACM SIGKDD International Conference on Knowledge Discovery and Data Mining (pp. 785-794). New York, NY, USA: ACM.). The XGBoost software may be obtained from http://xgboost.ai, for example). Another example framework that may be employed is LightGBM (e.g., as described, for example, in Ke, G., Meng, Q., Finley, T., Wang, T., Chen, W., Ma, W., ... Liu, T.-Y. (2017). Lightgbm: A highly efficient gradient boosting decision tree. Advances in Neural Information Processing Systems, 30, 3146-3154.). The LightGBM software may be obtained from https://lightgbm.readthedocs.io/, for example).

In some embodiments, a neural network classifier may be used. The neural network classifier may be trained using any suitable neural network optimization software. The optimization software may be configured to perform neural network training by gradient descent, stochastic gradient descent, or in any other suitable way. In some embodiments, the Adam optimizer (Kingma, D. and Ba, J. (2015) Adam: A Method for Stochastic Optimization. Proceedings of the 3rd International Conference on Learning Representations (ICLR 2015)) may be used.

*Additional or Alternative Embodiments*

Various embodiments have been described for using a hierarchy of machine learning models to identify one or more cell or particle types for an event. However, it should be appreciated that a multiclass classifier, which does not use information about the hierarchy of cells, may be used to predict a type for the event. For example, such a multiclass classifier may be based on the usage of gradient boosted trees.

However, training such a multiclass classifier may result in greater computational time and random-access memory (RAM) compared to training machine learning models in a hierarchy of machine learning models. Additionally, during model training, all cell populations (e.g., child and parent cell populations) are considered equal. Though this simplifies the learning process, this leads to the loss of information that is captured using the hierarchy of machine learning models.

*Computer Implementation*

An illustrative implementation of a computer system 1000 that may be used in connection with any of the embodiments of the technology described herein (e.g., such as the method of FIGS. 2A-C, 5A-D, and 8) is shown in **FIG. 10.** The computer system 1000 includes one or more processors 1010 and one or more articles of manufacture that comprise non-transitory computer-readable storage media (e.g., memory 1020 and one or more non-volatile storage media 1030). The processor 1010 may control writing data to and reading data from the memory 1020 and the non-volatile storage device 1030 in any suitable manner, as the aspects of the technology described herein are not limited to any particular techniques for writing or reading data. To perform any of the functionality described herein, the processor 1010 may execute one or more processor-executable instructions stored in one or more non-transitory computer-readable storage media (e.g., the memory 1020), which may serve as non-transitory computer-readable storage media storing processor-executable instructions for execution by the processor 1010.

Computing device 1000 may also include a network input/output (I/O) interface 1040 via which the computing device may communicate with other computing devices (e.g., over a network), and may also include one or more user I/O interfaces 1050, via which the computing device may provide output to and receive input from a user. The user I/O interfaces may include devices such as a keyboard, a mouse, a microphone, a display device (e.g., a monitor or touch screen), speakers, a camera, and/or various other types of I/O devices.

The above-described embodiments can be implemented in any of numerous ways. For example, the embodiments may be implemented using hardware, software, or a combination thereof. When implemented in software, the software code can be executed on any suitable processor (e.g., a microprocessor) or collection of processors, whether provided in a single computing device or distributed among multiple computing devices. It should be appreciated that any component or collection of components that perform the functions described above can be generically considered as one or more controllers that control the above-described functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments described herein comprises at least one computer-readable storage medium (e.g., RAM, ROM, EEPROM, flash memory or other memory technology, CD-ROM, digital versatile disks (DVD) or other optical disk storage, magnetic cassettes, magnetic tape, magnetic disk storage or other magnetic storage devices, or other tangible, non-transitory computer-readable storage medium) encoded with a computer program (i.e., a plurality of executable instructions) that, when executed on one or more processors, performs the above-described functions of one or more embodiments. The computer-readable medium may be transportable such that the program stored thereon can be loaded onto any computing device to implement aspects of the techniques described herein. In addition, it should be appreciated that the reference to a computer program which, when executed, performs any of the above-described functions, is not limited to an application program running on a host computer. Rather, the terms computer program and software are used herein in a generic sense to reference any type of computer code (e.g., application software, firmware, microcode, or any other form of computer instruction) that can be employed to program one or more processors to implement aspects of the techniques described herein.

The foregoing description of implementations provides illustration and description but is not intended to be exhaustive or to limit the implementations to the precise form disclosed. Modifications and variations are possible in light of the above teachings or may be acquired from practice of the implementations. In other implementations the methods depicted in these figures may include fewer operations, different operations, differently ordered operations, and/or additional operations. Further, non-dependent blocks may be performed in parallel. It will be apparent that example aspects, as described above, may be implemented in many different forms of software, firmware, and hardware in the implementations illustrated in the figures. Further, certain portions of the implementations may be implemented as a "module" that performs one or more functions. This module may include hardware, such as a processor, an application-specific integrated circuit (ASIC), or a field-programmable gate array (FPGA), or a combination of hardware and software.

*Biological Samples*

Any of the methods, systems, or other claimed elements may use or be used to analyze a biological sample from a subject. In some embodiments, a biological sample has been obtained from a subject having, suspected of having cancer, or at risk of having cancer. The biological sample may be any type of biological sample including, for example, a biological sample of a bodily fluid (e.g., blood, urine or cerebrospinal fluid), one or more cells (e.g., from a scraping or brushing such as a cheek swab or tracheal brushing), a piece of tissue (cheek tissue, muscle tissue, lung tissue, heart tissue, brain tissue, or skin tissue), or some or all of an organ (e.g., brain, lung, liver, bladder, kidney, pancreas, intestines, or muscle), or other types of biological samples (e.g., feces or hair).

In some embodiments, the biological sample is a sample of a tumor from a subject. In some embodiments, the biological sample is a sample of blood from a subject. In some embodiments, the biological sample is a sample of tissue from a subject.

A sample of a tumor, in some embodiments, refers to a sample comprising cells from a tumor. In some embodiments, the sample of the tumor comprises cells from a benign tumor, e.g., non-cancerous cells. In some embodiments, the sample of the tumor comprises cells from a premalignant tumor, e.g., precancerous cells. In some embodiments, the sample of the tumor comprises cells from a malignant tumor, e.g., cancerous cells.

Examples of tumors include, but are not limited to, adenomas, fibromas, hemangiomas, lipomas, cervical dysplasia, metaplasia of the lung, leukoplakia, carcinoma, sarcoma, germ cell tumors, and blastoma.

A sample of blood, in some embodiments, refers to a sample comprising cells, e.g., cells from a blood sample. In some embodiments, the sample of blood comprises non-cancerous cells. In some embodiments, the sample of blood comprises precancerous cells. In some embodiments, the sample of blood comprises cancerous cells. In some embodiments, the sample of blood comprises blood cells. In some embodiments, the sample of blood comprises red blood cells. In some embodiments, the sample of blood comprises white blood cells. In some embodiments, the sample of blood comprises platelets. Examples of cancerous blood cells include, but are not limited to, leukemia, lymphoma, and myeloma. In some embodiments, a sample of blood is collected to obtain the cell-free nucleic acid (e.g., cell-free DNA) in the blood.

A sample of blood may be a sample of whole blood or a sample of fractionated blood. In some embodiments, the sample of blood comprises whole blood. In some embodiments, the sample of blood comprises fractionated blood. In some embodiments, the sample of blood comprises buffy coat. In some embodiments, the sample of blood comprises serum. In some embodiments, the sample of blood comprises plasma. In some embodiments, the sample of blood comprises a blood clot.

A sample of a tissue, in some embodiments, refers to a sample comprising cells from a tissue. In some embodiments, the sample of the tumor comprises non-cancerous cells from a tissue. In some embodiments, the sample of the tumor comprises precancerous cells from a tissue.

Methods of the present disclosure encompass a variety of tissue including organ tissue or non-organ tissue, including but not limited to, muscle tissue, brain tissue, lung tissue, liver tissue, epithelial tissue, connective tissue, and nervous tissue. In some embodiments, the tissue may be normal tissue, or it may be diseased tissue, or it may be tissue suspected of being diseased. In some embodiments, the tissue may be sectioned tissue or whole intact tissue. In some embodiments, the tissue may be animal tissue or human tissue. Animal tissue includes, but is not limited to, tissues obtained from rodents (e.g., rats or mice), primates (e.g., monkeys), dogs, cats, and farm animals.

The biological sample may be from any source in the subject's body including, but not limited to, any fluid [such as blood (e.g., whole blood, blood serum, or blood plasma), saliva, tears, synovial fluid, cerebrospinal fluid, pleural fluid, pericardial fluid, ascitic fluid, and/or urine], hair, skin (including portions of the epidermis, dermis, and/or hypodermis), oropharynx, laryngopharynx, esophagus, stomach, bronchus, salivary gland, tongue, oral cavity, nasal cavity, vaginal cavity, anal cavity, bone, bone marrow, brain, thymus, spleen, small intestine, appendix, colon, rectum, anus, liver, biliary tract, pancreas, kidney, ureter, bladder, urethra, uterus, vagina, vulva, ovary, cervix, scrotum, penis, prostate, testicle, seminal vesicles, and/or any type of tissue (e.g., muscle tissue, epithelial tissue, connective tissue, or nervous tissue).

Any of the biological samples described herein may have been obtained from the subject using any known technique. See, for example, the following publications on collecting, processing, and storing biological samples: Biospecimens and biorepositories: from afterthought to science by Vaught et al. (Cancer Epidemiol Biomarkers Prev. 2012 Feb;21(2):253-5), and Biological sample collection, processing, storage and information management by Vaught and Henderson (IARC Sci Publ. 2011;(163):23-42).

In some embodiments, the biological sample may have been obtained from a surgical procedure (e.g., laparoscopic surgery, microscopically controlled surgery, or endoscopy), bone marrow biopsy, punch biopsy, endoscopic biopsy, or needle biopsy (e.g., a fine-needle aspiration, core needle biopsy, vacuum-assisted biopsy, or image-guided biopsy).

In some embodiments, one or more than one cell (i.e., a cell biological sample) may have been obtained from a subject using a scrape or brush method. The cell biological sample may have been obtained from any area in or from the body of a subject including, for example, from one or more of the following areas: the cervix, esophagus, stomach, bronchus, or oral cavity. In some embodiments, one or more than one piece of tissue (e.g., a tissue biopsy) from a subject may be used. In certain embodiments, the tissue biopsy may comprise one or more than one (e.g., 2, 3, 4, 5, 6, 7, 8, 9, 10, or more than 10) biological samples from one or more tumors or tissues known or suspected of having cancerous cells.

Any of the biological samples from a subject described herein may be stored using any method that preserves stability of the biological sample. In some embodiments, preserving the stability of the biological sample means inhibiting components (e.g., DNA, RNA, protein, or tissue structure or morphology) of the biological sample from degrading until they are measured so that when measured, the measurements represent the state of the sample at the time of obtaining it from the subject. In some embodiments, a biological sample is stored in a composition that is able to penetrate the same and protect components (e.g., DNA, RNA, protein, or tissue structure or morphology) of the biological sample from degrading. As used herein, degradation is the transformation of a component from one from to another such that the first form is no longer detected at the same level as before degradation.

In some embodiments, a biological sample (e.g., tissue sample) is fixed. As used herein, a "fixed" sample relates to a sample that has been treated with one or more agents or processes in order to prevent or reduce decay or degradation, such as autolysis or putrefaction, of the sample. Examples of fixative processes include but are not limited to heat fixation, immersion fixation, and perfusion. In some embodiments a fixed sample is a sample that has been treated with one or more fixative agents. Examples of fixative agents include but are not limited to cross-linking agents (e.g., aldehydes, such as formaldehyde, formalin, glutaraldehyde, etc.), precipitating agents (e.g., alcohols, such as ethanol, methanol, acetone, xylene, etc.), mercurials (e.g., B-5, Zenker's fixative, etc.), picrates, and Hepes-glutamic acid buffer-mediated organic solvent protection effect (HOPE) fixatuve. In some embodiments, a biological sample (e.g., tissue sample) is treated with a cross-linking agent. In some embodiments, the cross-linking agent comprises formalin. In some embodiments, a formalin-fixed biological sample is embedded in a solid substrate, for example paraffin wax. In some embodiments, the biological sample is a formalin-fixed paraffin-embedded (FFPE) sample. Methods of preparing FFPE samples are known, for example as described by Li et al. JCO Precis Oncol. 2018; 2: PO.17.00091.

In some embodiments, the biological sample has been stored using cryopreservation. Non-limiting examples of cryopreservation include, but are not limited to, step-down freezing, blast freezing, direct plunge freezing, snap freezing, slow freezing using a programmable freezer, and vitrification. In some embodiments, the biological sample is stored using lyophilization. In some embodiments, a biological sample is placed into a container that already contains a preservant (e.g., RNALater to preserve RNA) and then frozen (e.g., by snap-freezing), after the collection of the biological sample from the subject. In some embodiments, such storage in frozen state is done immediately after collection of the biological sample. In some embodiments, a biological sample may be kept at either room temperature or 4oC for some time (e.g., up to an hour, up to 8 h, or up to 1 day, or a few days) in a preservant or in a buffer without a preservant, before being frozen.

Non-limiting examples of preservants include formalin solutions, formaldehyde solutions, RNALater or other equivalent solutions, TriZol or other equivalent solutions, DNA/RNA Shield or equivalent solutions, EDTA (e.g., Buffer AE (10 mM Tris·Cl; 0.5 mM EDTA, pH 9.0)) and other coagulants, and Acids Citrate Dextronse (e.g., for blood specimens). In some embodiments, special containers may be used for collecting and/or storing a biological sample. For example, a vacutainer may be used to store blood. In some embodiments, a vacutainer may comprise a preservant (e.g., a coagulant, or an anticoagulant). In some embodiments, a container in which a biological sample is preserved may be contained in a secondary container, for the purpose of better preservation, or for the purpose of avoid contamination.

Any of the biological samples from a subject described herein may be stored under any condition that preserves stability of the biological sample. In some embodiments, the biological sample is stored at a temperature that preserves stability of the biological sample. In some embodiments, the sample is stored at room temperature (e.g., 25 °C). In some embodiments, the sample is stored under refrigeration (e.g., 4 °C). In some embodiments, the sample is stored under freezing conditions (e.g., -20 °C). In some embodiments, the sample is stored under ultralow temperature conditions (e.g., -50 °C to -800 °C). In some embodiments, the sample is stored under liquid nitrogen (e.g., -1700 °C). In some embodiments, a biological sample is stored at -60°C to -80°C (e.g., -70°C) for up to 5 years (e.g., up to 1 month, up to 2 months, up to 3 months, up to 4 months, up to 5 months, up to 6 months, up to 7 months, up to 8 months, up to 9 months, up to 10 months, up to 11 months, up to 1 year, up to 2 years, up to 3 years, up to 4 years, or up to 5 years). In some embodiments, a biological sample is stored as described by any of the methods described herein for up to 20 years (e.g., up to 5 years, up to 10 years, up to 15 years, or up to 20 years).

In some embodiments, one biological sample has been collected from a subject for analysis. In some cases, more than one (e.g., 2, 3, 4, 5, 6, 7, 8, 9, 10, 11, 12, 13, 14, 15, 16, 17, 18, 19, 20, or more) biological samples has been collected from a subject for analysis. In some cases, one biological sample from a subject will be analyzed. In some cases, more than one (e.g., 2, 3, 4, 5, 6, 7, 8, 9, 10, 11, 12, 13, 14, 15, 16, 17, 18, 19, 20, or more) biological samples may be analyzed. If more than one biological sample from a subject is analyzed, the biological samples may have been procured at the same time (e.g., more than one biological sample may be taken in the same procedure), or the biological samples may have been taken at different times (e.g., during a different procedure including a procedure 1, 2, 3, 4, 5, 6, 7, 8, 9, 10 days; 1, 2, 3, 4, 5, 6, 7, 8, 9, 10 weeks; 1, 2, 3, 4, 5, 6, 7, 8, 9, 10 months, 1, 2, 3, 4, 5, 6, 7, 8, 9, 10 years, or 1, 2, 3, 4, 5, 6, 7, 8, 9, 10 decades after a first procedure).

A second or subsequent biological sample may have been taken or obtained from the same region (e.g., from the same tumor or area of tissue) or a different region (including, e.g., a different tumor). A second or subsequent biological sample may have been taken or obtained from the subject after one or more treatments and may be taken from the same region or a different region. As a non-limiting example, the second or subsequent biological sample may be useful in determining whether the cancer in each biological sample has different characteristics (e.g., in the case of biological samples taken from two physically separate tumors in a patient) or whether the cancer has responded to one or more treatments (e.g., in the case of two or more biological samples from the same tumor or different tumors prior to and subsequent to a treatment). In some embodiments, each of the at least one biological sample is a bodily fluid sample, a cell sample, or a tissue biopsy sample.

In some embodiments, one or more biological specimens are combined (e.g., placed in the same container for preservation) before further processing. For example, a first sample of a first tumor obtained from a subject may be combined with a second sample of a second tumor from the subject, wherein the first and second tumors may or may not be the same tumor. In some embodiments, a first tumor and a second tumor are similar but not the same (e.g., two tumors in the brain of a subject). In some embodiments, a first biological sample and a second biological sample from a subject are sample of different types of tumors (e.g., a tumor in muscle tissue and brain tissue).

A sample from which RNA and/or DNA is extracted (e.g., a sample of tumor, or a blood sample) may be sufficiently large such that at least 2 μg (e.g., at least 2 μg, at least 2.5 μg, at least 3 μg, at least 3.5 μg or more) of RNA can be extracted from it. In some embodiments, the sample from which RNA and/or DNA is extracted can be peripheral blood mononuclear cells (PBMCs). A sample from which RNA and/or DNA is extracted can be any type of cell suspension. A sample from which RNA and/or DNA is extracted (e.g., a sample of tumor, or a blood sample) may be sufficiently large such that at least 1.8 μg RNA can be extracted from it. In some cases, at least 50 mg (e.g., at least 1 mg, at least 2 mg, at least 3 mg, at least 4 mg, at least 5 mg, at least 10 mg, at least 12 mg, at least 15 mg, at least 18 mg, at least 20 mg, at least 22 mg, at least 25 mg, at least 30 mg, at least 35 mg, at least 40 mg, at least 45 mg, or at least 50 mg) of tissue sample has been collected from which RNA and/or DNA is extracted. In some cases, at least 20 mg of tissue sample has been collected from which RNA and/or DNA is extracted. In some cases, at least 30 mg of tissue sample has been collected. In some cases, at least 10-50 mg (e.g., 10-50 mg, 10-15 mg, 10-30 mg, 10-40 mg, 20-30 mg, 20-40 mg, 20-50 mg, or 30-50 mg) of tissue sample has been collected from which RNA and/or DNA is extracted. In some cases, at least 30 mg of tissue sample collected. In some cases, at least 20-30 mg of tissue sample has been collected from which RNA and/or DNA is extracted. In some cases, a sample from which RNA and/or DNA is extracted (e.g., a sample of tumor, or a blood sample) is sufficiently large such that at least 0.2 μg (e.g., at least 200 ng, at least 300 ng, at least 400 ng, at least 500 ng, at least 600 ng, at least 700 ng, at least 800 ng, at least 900 ng, at least 1 μg, at least 1.1 μg, at least 1.2 μg, at least 1.3 μg, at least 1.4 μg, at least 1.5 μg, at least 1.6 μg, at least 1.7 μg, at least 1.8 μg, at least 1.9 μg, or at least 2 μg) of RNA can be extracted from it. In some cases, a sample from which RNA and/or DNA is extracted (e.g., a sample of tumor, or a blood sample) is sufficiently large such that at least 0.1 μg (e.g., at least 100 ng, at least 200 ng, at least 300 ng, at least 400 ng, at least 500 ng, at least 600 ng, at least 700 ng, at least 800 ng, at least 900 ng, at least 1 μg, at least 1.1 μg, at least 1.2 μg, at least 1.3 μg, at least 1.4 μg, at least 1.5 μg, at least 1.6 μg, at least 1.7 μg, at least 1.8 μg, at least 1.9 μg, or at least 2 μg) of RNA can be extracted from it.

*Subjects*

Aspects of this disclosure relate to a biological sample that has been obtained from a subject. In some embodiments, a subject is a mammal (e.g., a human, a mouse, a cat, a dog, a horse, a hamster, a cow, a pig, or other domesticated animal). In some embodiments, a subject is a human. In some embodiments, a subject is an adult human (e.g., of 18 years of age or older). In some embodiments, a subject is a child (e.g., less than 18 years of age). In some embodiments, a human subject is one who has or has been diagnosed with at least one form of cancer. In some embodiments, a cancer from which a subject suffers is a carcinoma, a sarcoma, a myeloma, a leukemia, a lymphoma, or a mixed type of cancer that comprises more than one of a carcinoma, a sarcoma, a myeloma, a leukemia, and a lymphoma. Carcinoma refers to a malignant neoplasm of epithelial origin or cancer of the internal or external lining of the body. Sarcoma refers to cancer that originates in supportive and connective tissues such as bones, tendons, cartilage, muscle, and fat. Myeloma is cancer that originates in the plasma cells of bone marrow. Leukemias ("liquid cancers" or "blood cancers") are cancers of the bone marrow (the site of blood cell production). Lymphomas develop in the glands or nodes of the lymphatic system, a network of vessels, nodes, and organs (specifically the spleen, tonsils, and thymus) that purify bodily fluids and produce infection-fighting white blood cells, or lymphocytes. Non-limiting examples of a mixed type of cancer include adenosquamous carcinoma, mixed mesodermal tumor, carcinosarcoma, and teratocarcinoma. In some embodiments, a subject has a tumor. A tumor may be benign or malignant. In some embodiments, a cancer is any one of the following: skin cancer, lung cancer, breast cancer, prostate cancer, colon cancer, rectal cancer, cervical cancer, and cancer of the uterus. In some embodiments, a subject is at risk for developing cancer, e.g., because the subject has one or more genetic risk factors, or has been exposed to or is being exposed to one or more carcinogens (e.g., cigarette smoke, or chewing tobacco).

*Flow Cytometry*

In some embodiments, a flow cytometry platform may be used to perform flow cytometry investigation of a fluid sample. The fluid sample may include target particles with particular particle attributes. The flow cytometry investigation of the fluid sample may provide a flow cytometry result for the fluid sample.

In some embodiments, the fluid sample may be exposed to a stain or dye that provides response radiation when exposed to investigation excitation radiation that may be measured by the radiation detection system of the flow cytometry platform. In some embodiments, a multiplicity of photodetectors is included in the flow cytometry platform. When a particle passes through the laser beam, time correlated pulses on forward scatter (FSC) and side scatter (SSC) detectors, and possibly also fluorescent emission detectors will occur. This is an "event," and for each event the magnitude of the detector output for each detector, FSC, SSC and fluorescence detectors is stored. The data obtained comprise the signals measured for each of the light scatter parameters and the fluorescence emissions.

Flow cytometry platforms may further comprise components for storing the detector outputs and analyzing the data. For example, data storage and analysis may be carried out using a computer connected to the detection electronics. For example, the data can be stored logically in tabular form, where each row corresponds to data for one particle (or one event), and the columns correspond to each of the measured parameters. The use of standard file formats, such as an "FCS" file format, for storing data from a flow cytometer facilitates analyzing data using separate programs and/or machines. In some embodiments, the data may be displayed in 2-dimensional (2D) plots for ease of visualization, but other methods may be used to visualize multidimensional data.

In some embodiments, the parameters measured using a flow cytometer may include FSC, which refers to the excitation light that is scattered by the particle along a generally forward direction, SSC, which refers to the excitation light that is scattered by the particle in a generally sideways direction, and the light emitted from fluorescent molecules in one or more channels (frequency bands) of the spectrum, referred to as FL1, FL2, etc., or by the name of the fluorescent dye that emits primarily in that channel.

Both flow and scanning cytometers are commercially available from, for example, BD Biosciences (San Jose, Calif.). Flow cytometry is described in, for example, Landy et al. (eds.), Clinical Flow Cytometry, Annals of the New York Academy of Sciences Volume 677 (1993); Bauer et al. (eds.), Clinical Flow Cytometry: Principles and Applications, Williams & Wilkins (1993); Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); and Practical Shapiro, Flow Cytometry, 4th ed., Wiley-Liss (2003). Fluorescence imaging microscopy is described in, for example, Pawley (ed.), Handbook of Biological Confocal Microscopy, 2nd Edition, Plenum Press (1989).

*Mass Cytometry*

In some embodiments, a mass cytometry platform may be used to perform mass cytometry investigation of a fluid sample. The fluid sample may include target particles with particular particle attributes. The mass cytometry investigation of the fluid sample may provide a mass cytometry result for the fluid sample.

In some embodiments, the fluid sample may be exposed to target-specific antibodies labeled with metal isotopes. In some embodiments, elemental mass spectrometry (e.g., inductively coupled plasma mass spectrometry (ICP-MS) and time of flight mass spectrometry (TOF-MS)) is used to detect the conjugated antibodies. For example, elemental mass spectrometry can discriminate isotopes of different atomic weights and measure electrical signals for isotopes associated with each particle or cell. Data obtained for a single cell or particle is considered an "event."

Mass cytometry platforms may further comprise components for storing the detector outputs and analyzing the data. For example, data storage and analysis may be carried out using a computer connected to the detection elements. The use of standard file formats, such as an "FCS" file format, for storing data from a mass cytometry platform facilitates analyzing data using separate programs and/or machines.

Mass cytometry platforms are commercially available from, for example, Fluidigm (San Francisco, CA). Mass cytometry is described in, for example, Bendall et al., A deep profiler's guide to cytometry, Trends in Immunology, 33(7), 323-332 (2012) and Spitzer et al., Mass Cytometry: Single Cells, Many Features, Cell, 165(4), 780-791 (2016).

*Methods of Treatment*

In certain methods described herein, an effective amount of anti-cancer therapy described herein may be recommended for administration to a subject (e.g., a human) in need of the treatment via a suitable route (e.g., intravenous administration).

The subject to be treated by the methods described herein may be a human patient having, suspected of having, or at risk for a cancer. Examples of a cancer include, but are not limited to, melanoma, lung cancer, brain cancer, breast cancer, colorectal cancer, pancreatic cancer, liver cancer, prostate cancer, skin cancer, kidney cancer, bladder cancer, or prostate cancer. At the time of diagnosis, the cancer may be cancer of unknown primary. The subject to be treated by the methods described herein may be a mammal (e.g., may be a human). Mammals include but are not limited to: farm animals (e.g., livestock), sport animals, laboratory animals, pets, primates, horses, dogs, cats, mice, and rats.

A subject having a cancer may be identified by routine medical examination, e.g., laboratory tests, biopsy, PET scans, CT scans, or ultrasounds. A subject suspected of having a cancer might show one or more symptoms of the disorder, e.g., unexplained weight loss, fever, fatigue, cough, pain, skin changes, unusual bleeding or discharge, and/or thickening or lumps in parts of the body. A subject at risk for a cancer may be a subject having one or more of the risk factors for that disorder. For example, risk factors associated with cancer include, but are not limited to, (a) viral infection (e.g., herpes virus infection), (b) age, (c) family history, (d) heavy alcohol consumption, (e) obesity, and (f) tobacco use.

"An effective amount" as used herein refers to the amount of each active agent required to confer therapeutic effect on the subject, either alone or in combination with one or more other active agents. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons, or for virtually any other reasons.

Empirical considerations, such as the half-life of a therapeutic compound, generally contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy and is generally (but not necessarily) based on treatment, and/or suppression, and/or amelioration, and/or delay of a cancer. Alternatively, sustained continuous release formulations of an anti-cancer therapeutic agent may be appropriate. Various formulations and devices for achieving sustained release are known in the art.

Dosages for an anti-cancer therapeutic agent as described herein may be determined empirically in individuals who have been administered one or more doses of the anti-cancer therapeutic agent. Individuals may be administered incremental dosages of the anti-cancer therapeutic agent. To assess efficacy of an administered anti-cancer therapeutic agent, one or more aspects of a cancer (e.g., tumor formation, tumor growth, molecular category identified for the cancer using the techniques described herein) may be analyzed.

Generally, for administration of any of the anti-cancer antibodies described herein, an initial candidate dosage may be about 2 mg/kg. For the purpose of the present disclosure, a typical daily dosage might range from about any of 0.1 $\mu$g/kg to 3 $\mu$g/kg to 30 $\mu$g/kg to 300 $\mu$g/kg to 3 mg/kg, to 30 mg/kg to 100 mg/kg or more, depending on the factors mentioned above. For repeated administrations over several days or longer, depending on the condition, the treatment is sustained until a desired suppression or amelioration of symptoms occurs or until sufficient therapeutic levels are achieved to alleviate a cancer, or one or more symptoms thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner (e.g., a medical doctor) wishes to achieve. For example, dosing from one-four times a week is contemplated. In some embodiments, dosing ranging from about 3 $\mu$g/mg to about 2 mg/kg (such as about 3 $\mu$g/mg, about 10 $\mu$g/mg, about 30 $\mu$g/mg, about 100 $\mu$g/mg, about 300 $\mu$g/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy may be monitored by conventional techniques and assays. The dosing regimen (including the therapeutic used) may vary over time.

When the anti-cancer therapeutic agent is not an antibody, it may be administered at the rate of about 0.1 to 300 mg/kg of the weight of the patient divided into one to three doses, or as disclosed herein. For an adult patient of normal weight, doses ranging from about 0.3 to 5.00 mg/kg may be administered. The particular dosage regimen, e.g.., dose, timing, and/or repetition, will depend on the particular subject and that individual's medical history, as well as the properties of the individual agents (such as the half-life of the agent, and other considerations well known in the art).

For the purpose of the present disclosure, the appropriate dosage of an anti-cancer therapeutic agent will depend on the specific anti-cancer therapeutic agent(s) (or compositions thereof) employed, the type and severity of cancer, whether the anti-cancer therapeutic agent is administered for preventive or therapeutic purposes, previous therapy, the patient's clinical history and response to the anti-cancer therapeutic agent, and the discretion of the attending physician. Typically, the clinician will administer an anti-cancer therapeutic agent, such as an antibody, until a dosage is reached that achieves the desired result.

Administration of an anti-cancer therapeutic agent can be continuous or intermittent, depending, for example, upon the recipient's physiological condition, whether the purpose of the administration is therapeutic or prophylactic, and other factors known to skilled practitioners. The administration of an anti-cancer therapeutic agent (e.g., an anti-cancer antibody) may be essentially continuous over a preselected period of time or may be in a series of spaced dose, e.g., either before, during, or after developing cancer.

As used herein, the term "treating" refers to the application or administration of a composition including one or more active agents to a subject, who has a cancer, a symptom of a cancer, or a predisposition toward a cancer, with the purpose to cure, heal, alleviate, relieve, alter, remedy, ameliorate, improve, or affect the cancer or one or more symptoms of the cancer, or the predisposition toward a cancer.

Alleviating a cancer includes delaying the development or progression of the disease or reducing disease severity. Alleviating the disease does not necessarily require curative results. As used therein, "delaying" the development of a disease (e.g., a cancer) means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given period and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detected and assessed using clinical techniques known in the art. However, development also refers to progression that may be undetectable. For purpose of this disclosure, development or progression refers to the biological course of the symptoms. "Development" includes occurrence, recurrence, and onset. As used herein "onset" or "occurrence" of a cancer includes initial onset and/or recurrence.

The anti-cancer therapeutic agent (e.g., an antibody) described herein may be administered to a subject in need of the treatment at an amount sufficient to reduce cancer (e.g., tumor) growth by at least 10% (e.g., 20%, 30%, 40%, 50%, 60%, 70%, 80%, 90% or greater). The anti-cancer therapeutic agent (e.g., an antibody) described herein may be administered to a subject in need of the treatment at an amount sufficient to reduce cancer cell number or tumor size by at least 10% (e.g., 20%, 30%, 40%, 50%, 60%, 70%, 80%, 90% or more). The anti-cancer therapeutic agent may be administered in an amount effective in altering cancer type. Alternatively, the anti-cancer therapeutic agent is administered in an amount effective in reducing tumor formation or metastasis.

Conventional methods, known to those of ordinary skill in the art of medicine, may be used to administer the anti-cancer therapeutic agent to the subject, depending upon the type of disease to be treated or the site of the disease. The anti-cancer therapeutic agent can also be administered via other conventional routes, e.g., administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, an anti-cancer therapeutic agent may be administered to the subject via injectable depot routes of administration such as using 1-, 3-, or 6-month depot injectable or biodegradable materials and methods.

Injectable compositions may contain various carriers such as vegetable oils, dimethylactamide, dimethyformamide, ethyl lactate, ethyl carbonate, isopropyl myristate, ethanol, and polyols (e.g., glycerol, propylene glycol, liquid polyethylene glycol, and the like). For intravenous injection, water soluble anti-cancer therapeutic agents can be administered by the drip method, whereby a pharmaceutical formulation containing the antibody and a physiologically acceptable excipients is infused. Physiologically acceptable excipients may include, for example, 5% dextrose, 0.9% saline, Ringer's solution, and/or other suitable excipients. Intramuscular preparations, e.g., a sterile formulation of a suitable soluble salt form of the anti-cancer therapeutic agent, can be dissolved and administered in a pharmaceutical excipient such as Water-for-Injection, 0.9% saline, and/or 5% glucose solution.

An anti-cancer therapeutic agent can be administered via site-specific or targeted local delivery techniques. Examples of site-specific or targeted local delivery techniques include various implantable depot sources of the agent or local delivery catheters, such as infusion catheters, an indwelling catheter, or a needle catheter, synthetic grafts, adventitial wraps, shunts and stents or other implantable devices, site specific carriers, direct injection, or direct application. See, e.g., PCT Publication No. WO 00/53211 and U.S. Pat. No. 5,981,568.

Targeted delivery of therapeutic compositions containing an antisense polynucleotide, expression vector, or subgenomic polynucleotides can also be used. Receptor-mediated DNA delivery techniques are described in, for example, Findeis et al., Trends Biotechnol. (1993) 11:202; Chiou et al., Gene Therapeutics: Methods and Applications of Direct Gene Transfer (J. A. Wolff, ed.) (1994); Wu et al., J. Biol. Chem. (1988) 263:621; Wu et al., J. Biol. Chem. (1994) 269:542; Zenke et al., Proc. Natl. Acad. Sci. USA (1990) 87:3655; Wu et al., J. Biol. Chem. (1991) 266:338.

Therapeutic compositions containing a polynucleotide may be administered in a range of about 100 ng to about 200 mg of DNA for local administration in a gene therapy protocol. In some embodiments, concentration ranges of about 500 ng to about 50 mg, about 1 μg to about 2 mg, about 5 μg to about 500 μg, and about 20 μg to about 100 μg of DNA or more can also be used during a gene therapy protocol.

Therapeutic polynucleotides and polypeptides can be delivered using gene delivery vehicles. The gene delivery vehicle can be of viral or non-viral origin (e.g., Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters and/or enhancers. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., PCT Publication Nos. WO 90/07936; WO 94/03622; WO 93/25698; WO 93/25234; WO 93/11230; WO 93/10218; WO 91/02805; U.S. Pat. Nos. 5,219,740 and 4,777,127; GB Patent No. 2,200,651; and EP Patent No. 0 345 242), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, e.g., PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., U.S. Pat. No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Pat. No. 5,580,859. Liposomes that can act as gene delivery vehicles are described in U.S. Pat. No. 5,422,120; PCT Publication Nos. WO 95/13796; WO 94/23697; WO 91/14445; and EP Patent No. 0524968. Additional approaches are described in Philip, Mol. Cell. Biol. (1994) 14:2411, and in Woffendin, Proc. Natl. Acad. Sci. (1994) 91:1581.

It is also apparent that an expression vector can be used to direct expression of any of the protein-based anti-cancer therapeutic agents (e.g., anti-cancer antibody). For example, peptide inhibitors that are capable of blocking (from partial to complete blocking) a cancer-causing biological activity are known in the art.

More than one anti-cancer therapeutic agent, such as an antibody and a small molecule inhibitory compound, may be administered to a subject in need of the treatment. The agents may be of the same type or different types from each other. At least one, at least two, at least three, at least four, or at least five different agents may be co-administered. Generally anti-cancer agents for administration have complementary activities that do not adversely affect each other. Anti-cancer therapeutic agents may also be used in conjunction with other agents that serve to enhance and/or complement the effectiveness of the agents.

Treatment efficacy can be assessed by methods well-known in the art, e.g., monitoring tumor growth or formation in a patient subjected to the treatment. Alternatively, or in addition to, treatment efficacy can be assessed by monitoring tumor type over the course of treatment (e.g., before, during, and after treatment).

A subject having cancer may be treated using any combination of anti-cancer therapeutic agents or one or more anti-cancer therapeutic agents and one or more additional therapies (e.g., surgery and/or radiotherapy). The term combination therapy, as used herein, embraces administration of more than one treatment (e.g., an antibody and a small molecule or an antibody and radiotherapy) in a sequential manner, that is, wherein each therapeutic agent is administered at a different time, as well as administration of these therapeutic agents, or at least two of the agents or therapies, in a substantially simultaneous manner.

Sequential or substantially simultaneous administration of each agent or therapy can be affected by any appropriate route including, but not limited to, oral routes, intravenous routes, intramuscular, subcutaneous routes, and direct absorption through mucous membrane tissues. The agents or therapies can be administered by the same route or by different routes. For example, a first agent (e.g., a small molecule) can be administered orally, and a second agent (e.g., an antibody) can be administered intravenously.

As used herein, the term "sequential" means, unless otherwise specified, characterized by a regular sequence or order, e.g., if a dosage regimen includes the administration of an antibody and a small molecule, a sequential dosage regimen could include administration of the antibody before, simultaneously, substantially simultaneously, or after administration of the small molecule, but both agents will be administered in a regular sequence or order. The term "separate" means, unless otherwise specified, to keep apart one from the other. The term "simultaneously" means, unless otherwise specified, happening or done at the same time, i.e., the agents are administered at the same time. The term "substantially simultaneously" means that the agents are administered within minutes of each other (e.g., within 10 minutes of each other) and intends to embrace joint administration as well as consecutive administration, but if the administration is consecutive it is separated in time for only a short period (e.g., the time it would take a medical practitioner to administer two agents separately). As used herein, concurrent administration and substantially simultaneous administration are used interchangeably. Sequential administration refers to temporally separated administration of the agents or therapies described herein.

Combination therapy can also embrace the administration of the anti-cancer therapeutic agent (e.g., an antibody) in further combination with other biologically active ingredients (e.g., a vitamin) and non-drug therapies (e.g., surgery or radiotherapy).

It should be appreciated that any combination of anti-cancer therapeutic agents may be used in any sequence for treating a cancer. The combinations described herein may be selected on the basis of a number of factors, which include but are not limited to reducing tumor formation or tumor growth, and/or alleviating at least one symptom associated with the cancer, or the effectiveness for mitigating the side effects of another agent of the combination. For example, a combined therapy as provided herein may reduce any of the side effects associated with each individual members of the combination, for example, a side effect associated with an administered anti-cancer agent.

An anti-cancer therapeutic agent may be an antibody, an immunotherapy, a radiation therapy, a surgical therapy, and/or a chemotherapy.

Examples of the antibody anti-cancer agents include, but are not limited to, alemtuzumab (Campath), trastuzumab (Herceptin), Ibritumomab tiuxetan (Zevalin), Brentuximab vedotin (Adcetris), Ado-trastuzumab emtansine (Kadcyla), blinatumomab (Blincyto), Bevacizumab (Avastin), Cetuximab (Erbitux), ipilimumab (Yervoy), nivolumab (Opdivo), pembrolizumab (Keytruda), atezolizumab (Tecentriq), avelumab (Bavencio), durvalumab (Imfinzi), and panitumumab (Vectibix).

Examples of an immunotherapy include, but are not limited to, a PD-1 inhibitor or a PD-L1 inhibitor, a CTLA-4 inhibitor, adoptive cell transfer, therapeutic cancer vaccines, oncolytic virus therapy, T-cell therapy, and immune checkpoint inhibitors.

Examples of radiation therapy include, but are not limited to, ionizing radiation, gamma-radiation, neutron beam radiotherapy, electron beam radiotherapy, proton therapy, brachytherapy, systemic radioactive isotopes, and radiosensitizers.

Examples of a surgical therapy include, but are not limited to, a curative surgery (e.g., tumor removal surgery), a preventive surgery, a laparoscopic surgery, and a laser surgery.

Examples of the chemotherapeutic agents include, but are not limited to, Carboplatin or Cisplatin, Docetaxel, Gemcitabine, Nab-Paclitaxel, Paclitaxel, Pemetrexed, and Vinorelbine.

Additional examples of chemotherapy include, but are not limited to, Platinating agents, such as Carboplatin, Oxaliplatin, Cisplatin, Nedaplatin, Satraplatin, Lobaplatin, Triplatin, Tetranitrate, Picoplatin, Prolindac, Aroplatin and other derivatives; Topoisomerase I inhibitors, such as Camptothecin, Topotecan, irinotecan/SN38, rubitecan, Belotecan, and other derivatives; Topoisomerase II inhibitors, such as Etoposide (VP-16), Daunorubicin, a doxorubicin agent (e.g., doxorubicin, doxorubicin hydrochloride, doxorubicin analogs, or doxorubicin and salts or analogs thereof in liposomes), Mitoxantrone, Aclarubicin, Epirubicin, Idarubicin, Amrubicin, Amsacrine, Pirarubicin, Valrubicin, Zorubicin, Teniposide and other derivatives; Antimetabolites, such as Folic family (Methotrexate, Pemetrexed, Raltitrexed, Aminopterin, and relatives or derivatives thereof); Purine antagonists (Thioguanine, Fludarabine, Cladribine, 6-Mercaptopurine, Pentostatin, clofarabine, and relatives or derivatives thereof) and Pyrimidine antagonists (Cytarabine, Floxuridine, Azacitidine, Tegafur, Carmofur, Capacitabine, Gemcitabine, hydroxyurea, 5-Fluorouracil (5FU), and relatives or derivatives thereof); Alkylating agents, such as Nitrogen mustards (e.g., Cyclophosphamide, Melphalan, Chlorambucil, mechlorethamine, Ifosfamide, mechlorethamine, Trofosfamide, Prednimustine, Bendamustine, Uramustine, Estramustine, and relatives or derivatives thereof); nitrosoureas (e.g., Carmustine, Lomustine, Semustine, Fotemustine, Nimustine, Ranimustine, Streptozocin, and relatives or derivatives thereof); Triazenes (e.g., Dacarbazine, Altretamine, Temozolomide, and relatives or derivatives thereof); Alkyl sulphonates (e.g., Busulfan, Mannosulfan, Treosulfan, and relatives or derivatives thereof); Procarbazine; Mitobronitol, and Aziridines (e.g., Carboquone, Triaziquone, ThioTEPA, triethylenemalamine, and relatives or derivatives thereof); Antibiotics, such as Hydroxyurea, Anthracyclines (e.g., doxorubicin agent, daunorubicin, epirubicin and relatives or derivatives thereof); Anthracenediones (e.g., Mitoxantrone and relatives or derivatives thereof); Streptomyces family antibiotics (e.g., Bleomycin, Mitomycin C, Actinomycin, and Plicamycin); and ultraviolet light.

Having thus described several aspects of the technology set forth in the disclosure, it is to be appreciated that various alterations, modifications, and improvements will readily occur to those skilled in the art. In addition, any combination of two or more features, systems, articles, materials, kits, and/or methods described herein, if such features, systems, articles, materials, kits, and/or methods are not mutually inconsistent, is included within the scope of the present disclosure.

The above-described disclosures can be implemented in any of numerous ways. One or more aspects and embodiments of the present disclosure involving the performance of processes or methods may utilize program instructions executable by a device (e.g., a computer, a processor, or other device) to perform, or control performance of, the processes or methods. In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other tangible computer storage medium) encoded with one or more programs that, when executed on one or more computers or other processors, perform methods that implement one or more of the various embodiments described above. The computer readable medium or media can be transportable, such that the program or programs stored thereon can be loaded onto one or more different computers or other processors to implement various ones of the aspects described above. In some embodiments, computer readable media is non-transitory media.

The terms "program" or "software" are used herein in a generic sense to refer to any type of computer code or set of computer-executable instructions that can be employed to program a computer or other processor to implement various aspects as described above. Additionally, it should be appreciated that according to one aspect, one or more computer programs that when executed perform methods of the present disclosure need not reside on a single computer or processor but may be distributed in a modular fashion among a number of different computers or processors to implement various aspects of the present disclosure.

Computer-executable instructions may be in many forms, such as program modules, executed by one or more computers or other devices. Generally, program modules include routines, programs, objects, components, data structures, etc. that perform particular tasks or implement particular abstract data types. Typically, the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

When implemented in software, the software code can be executed on any suitable processor or collection of processors, whether provided in a single computer or distributed among multiple computers.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone, a tablet, or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible formats.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in referenced documents, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The terms "approximately," "substantially," and "about" may be used to mean within $\pm 20\%$ of a target value in some embodiments, within $\pm 10\%$ of a target value in some embodiments, within $\pm 5\%$ of a target value in some embodiments, within $\pm 2\%$ of a target value in some embodiments. The terms "approximately," "substantially," and "about" may include the target value.

## Claims

1. A method for identifying types of cells present in biological samples using flow cytometry or mass cytometry and multiple machine learning models, the method comprising:
   using at least one computer hardware processor to perform:

   obtaining cytometry data (106) for a biological sample (102) previously obtained from a subject, the biological sample (102) comprising a plurality of cells, the cytometry data (106) including cytometry measurements obtained during respective cytometry events, the cytometry events corresponding to particular objects in the biological sample (102) being measured by a flow cytometry platform or a mass cytometry platform (104), the cytometry events including a subset of events corresponding to cells in the biological sample (102) being measured by the cytometry platform (104); and
   identifying types of cells (110) in the plurality of cells using the multiple machine learning models to obtain a respective plurality of cell types (110), the multiple machine learning models including a first machine learning model and a second machine learning model different from the first machine learning model, the identifying comprising, for each particular event in the subset of events,

      obtaining, from the cytometry data (106), cytometry measurements corresponding to the particular event;
      determining an event type (110) for the particular event by processing the cytometry measurements corresponding to the particular event using the first machine learning model, the event type (110) indicating whether the particular event corresponds to a cell being measured by the cytometry platform (104), debris being measured by the cytometry platform (104), or a bead being measured by the cytometry platform (104); and
      when the determined event type (110) indicates that the particular event corresponds to the cell being measured by the cytometry platform (104), determining a type of the cell (110) by processing the cytometry measurements corresponding to the particular event using the second machine learning model.

2. The method of claim 1, wherein the subset of events comprises at least 10,000 events, or at least 100,000 events.

3. The method of claim 1 or claim 2,

   wherein the first machine learning model comprises a first multiclass classifier, and
   wherein the second machine learning model comprises a second multiclass classifier.

4. The method of any preceding claim,

   wherein the first machine learning model comprises a first decision tree classifier, a first gradient boosted decision tree classifier, or a first neural network, and
   wherein the second machine learning model comprises a second decision tree classifier, a second gradient boosted decision tree classifier, or a second neural network.

5. The method of any preceding claim, further comprising:
determining cell composition percentages of different types of cells in the biological sample based on the identified plurality of cell types.

6. The method of claim 5, wherein determining the cell composition percentages comprises:
determining a first cell composition percentage for a first type of cell by determining a ratio between a number of cells in the plurality of cells identified as being of the first type and a total number of the cells in the plurality of cells.

7. The method of claim 5 or claim 6, wherein the subject has, is suspected of having, or is at risk of having cancer, and wherein the method further comprises:
identifying a treatment for the subject based on the determined cell composition percentages.

8. The method of any of claims 5 to 7, further comprising:
comparing a cell composition percentage of the determined cell composition percentages to a range of cell composition percentages associated with a patient cohort; and identifying the subject as a member of the patient cohort based on a result of the comparing, optionally wherein the patient cohort comprises a healthy cohort, a cohort of patients with a disease, or a cohort of patients who have received a treatment.

9. The method of any of claims 5 to 8, further comprising:

comparing a cell composition percentage of the determined cell composition percentages to a range of cell composition percentages associated with a study, wherein the study evaluates effectiveness of one or more treatments in treating a disease; and
identifying a treatment for the subject based on a result of the comparing.

10. The method of any preceding claim,

wherein the subset of events corresponding to the cells in the biological sample being measured by the cytometry platform comprises a first subset of events, and
wherein the cytometry events further include:

a second subset of events corresponding to beads in the biological sample being measured by the cytometry platform, and
a third subset of events corresponding to debris in the biological sample being measured by the cytometry platform.

11. The method of any preceding claim, wherein the cytometry is flow cytometry and the cytometry measurements corresponding to the particular event comprise fluorescence intensity values for at least some of a plurality of markers, or wherein the cytometry is mass cytometry and the cytometry measurements corresponding to the particular event comprise relative intensity of heavy metal ion tags for at least some of a plurality of markers.

12. The method of any preceding claim,

wherein the plurality of events includes a first plurality of events and a second plurality of events, and
wherein the cytometry data comprises first cytometry data for the first plurality of events and second cytometry data for the second plurality of events, the first cytometry data comprising measurements obtained for first markers of a plurality of markers during each of at least some of the first plurality of events and the second cytometry data comprising measurements obtained for second markers of the plurality of markers during each of at least some of the second plurality of events, wherein the first markers of the plurality of markers and the second markers of the plurality of markers are different.

13. The method of claim 12,

wherein the first cytometry data comprises data from a first panel, and
wherein the second cytometry data comprises data from a second panel different from the first panel.

14. At least one non-transitory computer-readable storage medium (1030) storing processor-executable instructions that, when executed by at least one computer hardware processor (1010), cause the at least one computer hardware processor (1010) to perform the method of any one of claims 1-13.

15. A system comprising:

at least one computer hardware processor (1010); and
at least one non-transitory computer-readable storage medium (1030) storing processor-executable instructions that, when executed by the at least one computer hardware processor (1010), cause the at least one computer hardware processor (1010) to perform the method of any one of claims 1-13.

**Patentansprüche**

1. Verfahren zum Identifizieren von Zelltypen, die in biologischen Proben vorliegen, unter Verwendung von Durchflusszytometrie oder Massenzytometrie und mehreren Modellen maschinellen Lernens, wobei das Verfahren Folgendes umfasst:
   Verwenden von zumindest einem Computerhardwareprozessor, um Folgendes durchzuführen:

   Erhalten von Zytometriedaten (106) für eine biologische Probe (102), die zuvor von einem Individuum erhalten wurde, wobei die biologische Probe (102) eine Vielzahl von Zellen umfasst, wobei die Zytometriedaten (106) Zytometriemessungen umfassen, die während entsprechender Zytometrieereignisse erhalten wurden, wobei die Zytometrieereignisse speziellen Objekten in der biologischen Probe (102) entsprechen, die durch eine Zytometrieplattform oder eine Massenzytometrieplattform (104) gemessen wird, wobei die Zytometrieereignisse einen Teilsatz von Ereignissen umfassen, welche Zellen in der biologischen Probe (102) entsprechen, die durch die Zytometrieplattform (104) gemessen wird, und
   Identifizieren von Zelltypen (110) in der Vielzahl von Zellen unter Verwendung der mehreren Modelle maschinellen Lernens, um eine entsprechende Vielzahl von Zelltypen (110) zu erhalten, wobei die mehreren Modelle maschinellen Lernens ein erstes Modell maschinellen Lernens und ein zweites Modell maschinellen Lernens, das sich von dem ersten Modell maschinellen Lernens unterscheidet, umfassen, wobei das Identifizieren für jedes spezielle Ereignis im Teilsatz von Ereignissen Folgendes umfasst:

   Erhalten von Zytometriemessungen, die den spezielle Ereignissen entsprechen, aus den Zytometriedaten (106),
   Bestimmen eines Ereignistyps (110) für das spezielle Ereignis durch Verarbeiten der Zytometriemessungen, die dem speziellen Ereignis entsprechen, unter Verwendung des ersten Modells maschinellen Lernens, wobei der Ereignistyp (110) anzeigt, ob das spezielle Ereignis einer Zelle, die durch die Zytometrieplattform (104) gemessen wird, Bruchstücken, die durch die Zytometrieplattform (104) gemessen werden, oder einem Kügelchen, das durch die Zytometrieplattform (104) gemessen wird, entspricht; und
   wenn der bestimmte Ereignistyp (110) anzeigt, dass das spezielle Ereignis der Zelle entspricht, die durch die Zytometrieplattform (104) gemessen wird, Bestimmen des Zelltyps (110) durch Verarbeiten der Zytometriemessungen, die dem speziellen Ereignis entsprechen, unter Verwendung des zweiten Modells maschinellen Lernens.

2. Verfahren nach Anspruch 1, wobei der Teilsatz von Ereignissen zumindest 10.000 Ereignisse oder zumindest 100.000 Ereignisse umfasst.

3. Verfahren nach Anspruch 1 oder Anspruch 2,

   wobei das erste Modell maschinellen Lernens einen ersten Mehrklassenklassifikator umfasst und
   wobei das zweite Modell maschinellen Lernens einen zweiten Mehrklassenklassifikator umfasst.

4. Verfahren nach einem der vorangegangenen Ansprüche,

   wobei das erste Modell maschinellen Lernens einen ersten Entscheidungsbaumklassifikator, einen ersten Gradient-Boosting-Entscheidungsbaumklassifikator oder ein erstes neuronales Netz umfasst und
   wobei das zweite Modell maschinellen Lernens einen zweiten Entscheidungsbaumklassifikator, einen zweiten Gradient-Boosting-Entscheidungsbaumklassifikator oder ein zweites neuronales Netz umfasst.

5. Verfahren nach einem der vorangegangenen Ansprüche, das ferner Folgendes umfasst:

Bestimmen von Zellzusammensetzungsprozentsätzen von verschiedenen Zelltypen in der biologischen Probe beruhend auf der identifizierten Vielzahl von Zelltypen.

6. Verfahren nach Anspruch 5, wobei das Bestimmen der Zellzusammensetzungsprozentsätze Folgendes umfasst: Bestimmen eines ersten Zellzusammensetzungsprozentsatzes für einen ersten Zelltyp durch Bestimmen eines Verhältnisses zwischen einer Anzahl von Zellen in der Vielzahl von Zellen, die als dem ersten Typ angehörig identifiziert wurden, und einer Gesamtzahl von Zellen in der Vielzahl von Zellen.

7. Verfahren nach Anspruch 5 oder Anspruch 6, wobei das Individuum an Krebs erkrankt ist, beim Individuum Verdacht auf eine Krebserkrankung besteht oder das Individuum ein Risiko trägt, an Krebs zu erkranken, und wobei das Verfahren ferner Folgendes umfasst: Identifizieren einer Behandlung für das Individuum beruhend auf den bestimmten Zellzusammensetzungsprozentsätzen.

8. Verfahren nach einem der Ansprüche 5 bis 7, das ferner Folgendes umfasst:

Vergleichen eines Zellzusammensetzungsprozentsatzes aus den bestimmten Zellzusammensetzungsprozentsätzen mit einem Bereich von Zeilzusammensetzungsprozentsätzen, die einer Patientenkohorte zugeordnet sind; und Identifizieren des Individuums als Mitglied der Patientenkohorte beruhend auf einem Ergebnis des Vergleichs, wobei die Patientenkohorte gegebenenfalls eine gesunde Kohorte, eine Kohorte von Patienten mit einer Erkrankung oder eine Kohorte von Patienten, die eine Behandlung erhalten haben, umfasst.

9. Verfahren nach einem der Ansprüche 5 bis 8, das ferner Folgendes umfasst:

Vergleichen eines Zellzusammensetzungsprozentsatzes aus den bestimmten Zellzusammensetzungsprozentsätzen mit einem Bereich von Zeilzusammensetzungsprozentsätzen, die einer Studie zugeordnet sind, wobei die Studie die Wirksamkeit von einer oder mehreren Behandlungen bei der Behandlung einer Erkrankung bewertet; und Identifizieren einer Behandlung für das Individuum beruhend auf einem Ergebnis des Vergleichs.

10. Verfahren nach einem der vorangegangenen Ansprüche,

wobei der Teilsatz von Ereignissen, die den Zellen in der biologischen Probe entsprechen, die durch die Zytometrieplattform gemessen wird, einen ersten Teilsatz von Ereignissen umfasst und wobei die Zytometrieereignisse ferner Folgendes umfassen:

einen zweiten Teilsatz von Ereignissen, die Kügelchen in der biologischen Probe entsprechen, die durch die Zytometrieplattform gemessen wird, und einen dritten Teilsatz von Ereignissen, die Bruchstücken in der biologischen Probe entsprechen, die durch die Zytometrieplattform gemessen wird.

11. Verfahren nach einem der vorangegangenen Ansprüche, wobei die Zytometrie Durchflusszytometrie ist und die Zytometriemessungen, die dem speziellen Ereignis entsprechen, Fluoreszenzintensitätswerte für zumindest einige aus einer Vielzahl von Markern umfassen oder wobei die Zytometrie Massenzytometrie ist und die Zytometriemessungen, die dem bestimmten Ereignis entsprechen, eine relative Intensität von Schwermetallionenmarkierungen für zumindest einige aus einer Vielzahl von Markern umfassen.

12. Verfahren nach einem der vorangegangenen Ansprüche,

wobei die Vielzahl von Ereignissen eine erste Vielzahl von Ereignissen und eine zweite Vielzahl von Ereignissen umfasst und wobei die Zytometriedaten erste Zytometriedaten für die erste Vielzahl von Ereignissen und zweite Zytometriedaten für die zweite Vielzahl von Ereignissen umfassen, wobei die ersten Zytometriedaten Messungen umfassen, die für erste Marker aus einer Vielzahl von Markern während jedes aus zumindest einigen aus der ersten Vielzahl von Ereignissen erhalten wurden, und die zweiten Zytometriedaten Messungen umfassen, die für zweite Marker aus der Vielzahl von Markern während jedes aus zumindest einigen aus der zweiten Vielzahl von Ereignissen erhalten wurden, wobei die ersten Marker aus der Vielzahl von Markern und die zweiten Marker aus der Vielzahl von Markern sich voneinander unterscheiden.

13. Verfahren nach Anspruch 12,

wobei die ersten Zytometriedaten Daten aus einem ersten Panel umfassen und
wobei die zweiten Zytometriedaten Daten aus einem zweiten Panel, das sich von dem ersten Panel unterscheidet, umfassen.

14. Zumindest ein nichtflüchtiges computerlesbares Speichermedium (1030), auf dem durch einen Prozessor ausführbare Befehle gespeichert sind, die, wenn sie von zumindest einem Computerhardwareprozessor (1010) ausgeführt werden, bewirken, dass der zumindest eine Computerhardwareprozessor (1010) ein Verfahren nach einem der Ansprüche 1 bis 13 durchführt.

15. System, das Folgendes umfasst:

zumindest einen Computerhardwareprozessor (1010); und
zumindest ein nichtflüchtiges computerlesbares Speichermedium (1030), auf dem durch einen Prozessor ausführbare Befehle gespeichert sind, die, wenn sie von dem zumindest einen Computerhardwareprozessor (1010) ausgeführt werden, bewirken, dass der zumindest eine Computerhardwareprozessor (1010) ein Verfahren nach einem der Ansprüche 1 bis 13 durchführt.


**Revendications**

1. Procédé d'identification de types de cellules présentes dans des échantillons biologiques en utilisant de la cytométrie en flux ou de la cytométrie de masse et de multiples modèles d'apprentissage machine, le procédé comprenant l'étape consistant à :
utiliser au moins un matériel processeur informatique pour :

obtenir des données de cytométrie (106) pour un échantillon biologique (102) précédemment obtenu auprès d'un sujet, l'échantillon biologique (102) comprenant une pluralité de cellules, les données de cytométrie (106) incluant des mesures de cytométrie obtenues pendant des événements de cytométrie respectifs, les événements de cytométrie correspondant à des objets particuliers dans l'échantillon biologique (102) étant mesurés par une plate-forme de cytométrie en flux ou une plate-forme de cytométrie de masse (104), les événements de cytométrie incluant un sous-ensemble d'événements correspondant à des cellules dans l'échantillon biologique (102) étant mesurés par la plate-forme de cytométrie (104) ; et
identifier des types de cellules (110) dans la pluralité de cellules en utilisant les multiples modèles d'apprentissage machine pour obtenir une pluralité respective de types de cellules (110), les multiples modèles d'apprentissage machine incluant un premier modèle d'apprentissage machine et un second modèle d'apprentissage machine différent du premier modèle d'apprentissage machine, l'identification comprenant, pour chaque événement particulier dans le sous-ensemble d'événements, les étapes consistant à

obtenir, à partir des données de cytométrie (106), des mesures de cytométrie correspondant à l'événement particulier ;
déterminer un type d'événement (110) pour l'événement particulier en traitant les mesures de cytométrie correspondant à l'événement particulier en utilisant le premier modèle d'apprentissage machine, le type d'événement (110) indiquant si l'événement particulier correspond à une cellule mesurée par la plate-forme de cytométrie (104), des débris étant mesurés par la plate-forme de cytométrie (104), ou une bille étant mesurée par la plate-forme de cytométrie (104) ; et
lorsque le type d'événement déterminé (110) indique que l'événement particulier correspond à la cellule mesurée par la plate-forme de cytométrie (104), déterminer un type de la cellule (110) en traitant les mesures de cytométrie correspondant à l'événement particulier en utilisant le second modèle d'apprentissage machine.

2. Procédé selon la revendication 1, dans lequel le sous-ensemble d'événements comprend au moins 10 000 événements, ou au moins 100 000 événements.

3. Procédé selon la revendication 1 ou 2,

dans lequel le premier modèle d'apprentissage machine comprend un premier classificateur multiclasse, et dans lequel le second modèle d'apprentissage machine comprend un second classificateur multiclasse.

4. Procédé selon l'une quelconque des revendications précédentes,

dans lequel le premier modèle d'apprentissage machine comprend un premier classificateur d'arbre de décision, un premier classificateur d'arbre de décision amplifié par gradient, ou un premier réseau neuronal, et
dans lequel le second modèle d'apprentissage machine comprend un second classificateur d'arbre de décision, un second classificateur d'arbre de décision amplifié par gradient, ou un second réseau neuronal.

5. Procédé selon l'une quelconque des revendications précédentes, comprenant en outre l'étape consistant à :
déterminer des pourcentages de composition de cellule de différents types de cellules dans l'échantillon biologique sur la base de la pluralité identifiée de types de cellule.

6. Procédé selon la revendication 5, dans lequel la détermination des pourcentages de composition de cellule comprend l'étape consistant à :
déterminer un premier pourcentage de composition de cellule pour un premier type de cellule en déterminant un rapport entre un nombre de cellules dans la pluralité de cellules identifiées comme étant du premier type et un nombre total de cellules dans la pluralité de cellules.

7. Procédé selon la revendication 5 ou la revendication 6, dans lequel le sujet présente, est suspecté de présenter ou risque de présenter un cancer, et dans lequel le procédé comprend en outre l'étape consistant à :
identifier un traitement pour le sujet sur la base des pourcentages de composition de cellule déterminés.

8. Procédé selon l'une quelconque des revendications 5 à 7, comprenant en outre les étapes consistant à :

comparer un pourcentage de composition de cellule des pourcentages de composition de cellule déterminés à une plage de pourcentages de composition de cellule associés à une cohorte de patients ; et
identifier le sujet en tant que membre de la cohorte de patients sur la base d'un résultat de la comparaison, facultativement dans lequel la cohorte de patients comprend une cohorte saine, une cohorte de patients atteints d'une maladie, ou une cohorte de patients qui ont reçu un traitement.

9. Procédé selon l'une quelconque des revendications 5 à 8, comprenant en outre les étapes consistant à :

comparer un pourcentage de composition de cellule des pourcentages de composition de cellule déterminés à une plage de pourcentages de composition de cellule associés à une étude, dans lequel l'étude évalue l'efficacité d'un ou plusieurs traitements dans le traitement d'une maladie ; et
identifier un traitement pour le sujet sur la base d'un résultat de la comparaison.

10. Procédé selon l'une quelconque des revendications précédentes,

dans lequel le sous-ensemble d'événements correspondant aux cellules dans l'échantillon biologique mesurés par la plate-forme de cytométrie comprend un premier sous-ensemble d'événements, et
dans lequel les événements de cytométrie incluent en outre :

un deuxième sous-ensemble d'événements correspondant à des billes dans l'échantillon biologique mesuré par la plate-forme de cytométrie, et
un troisième sous-ensemble d'événements correspondant à des débris dans l'échantillon biologique mesuré par la plate-forme de cytométrie.

11. Procédé selon l'une quelconque des revendications précédentes, dans lequel la cytométrie est une cytométrie en flux et les mesures de cytométrie correspondant à l'événement particulier comprennent des valeurs d'intensité de fluorescence pour au moins certains d'une pluralité de marqueurs, ou dans lequel la cytométrie est une cytométrie de masse et les mesures de cytométrie correspondant à l'événement particulier comprennent une intensité relative de marqueurs d'ions de métal lourd pour au moins certains d'une pluralité de marqueurs.

12. Procédé selon l'une quelconque des revendications précédentes,

dans lequel la pluralité d'événements inclut une première pluralité d'événements et une seconde pluralité d'événements, et

dans lequel les données de cytométrie comprennent des premières données de cytométrie pour la première pluralité d'événements et des secondes données de cytométrie pour la seconde pluralité d'événements, les premières données de cytométrie comprenant des mesures obtenues pour des premiers marqueurs d'une pluralité de marqueurs pendant chacun d'au moins certains de la première pluralité d'événements, et les secondes données de cytométrie comprenant des mesures obtenues pour des seconds marqueurs de la pluralité de marqueurs pendant chacun d'au moins certains de la seconde pluralité d'événements, dans lequel les premiers marqueurs de la pluralité de marqueurs et les seconds marqueurs de la pluralité de marqueurs sont différents.

13. Procédé selon la revendication 12,

dans lequel les premières données de cytométrie comprennent des données provenant d'un premier panneau, et dans lequel les secondes données de cytométrie comprennent des données provenant d'un second panneau différent du premier panneau.

14. Au moins un support de stockage non transitoire lisible par ordinateur (1030) stockant des instructions exécutables par processeur qui, lorsqu'elles sont exécutées par au moins un processeur matériel informatique (1010), amènent le au moins un processeur matériel informatiques (1010) à exécuter le procédé selon l'une quelconque des revendications 1 à 13.

15. Système, comprenant :

au moins un processeur matériel informatique (1010); et
au moins un support de stockage non transitoire lisible par ordinateur (1030) stockant des instructions exécutables par processeur qui, lorsqu'elles sont exécutées par le au moins un processeur matériel informatique (1010), amènent le au moins un processeur matériel informatique (1010) à exécuter le procédé selon l'une quelconque des revendications 1 à 13.

100

102

104

Cytometry
Platform

Biological Sample

108

106

Computing Device

Cytometry Data

110

Cell/Particle Types

# FIG. 1A

106

| Event: | Cytometry Data: | |
|--------|-----------------|--|
| Event 1 | Cytometry Data 1 | 132-1 |
| Event 2 | Cytometry Data 2 | 132-2 |
| Event 3 | Cytometry Data 3 | 132-3 |
| . . . | . . . | |
| Event N | Cytometry Data N | 132-N |

# FIG. 1B

106

| Event: | CD3 | CD62L | CD27 | CD45+ | IgA+ | |
|--------|-----|-------|------|-------|------|--|
| Event 1 | 1.58 | 5.43 | -1.69 | -2.13 | 2.96 | 132-1 |
| Event 2 | 1.56 | 1.49 | 0.67 | 3.97 | 1.45 | 132-2 |
| Event 3 | 0.87 | -1.42 | 0.89 | 4.14 | 0.97 | 132-3 |
| . . . | . . . | . . . | . . . | . . . | . . . | |
| Event N | 0.65 | 1.66 | 4.32 | 0.24 | -3.43 | 132-N |

# FIG. 1C

FIG. 1D

FIG. 1E

200

START

202

Obtain cytometry data for a biological sample from a subject, the biological sample comprising a plurality of cells including a first cell, the cytometry data including first cytometry data for the first cell

204

Process the cytometry data using one or more data processing techniques

206

Determine a respective type for each of at least some of the plurality of cells using a hierarchy of machine learning models, the determining comprising:

206a

Determine a first type for the first cell by processing the first cytometry data using a first subset of the hierarchy of machine learning models.

206b

Another cell of the plurality of cells?    Yes

No

END

FIG. 2A

206*a*

222

```
┌─────────────┐
│    Start    │
└─────────────┘
```

Process the first cytometry data for the first cell using one or more machine learning models at a first level of the hierarchy of machine learning models.

224

No ◆ At least one output > threshod? ◆

Yes

226

Determine a first type for the first cell based on outputs of the one or more machine learning models at the first level of the hierarchy of machine learning models.

228

No ◆ Apply another machine learning model of the hierarchy of machine learning models? ◆

Yes

230

Identify one or more machine learning models at a second level of the hierarchy of machine learning models based on the first type determined for the first cell.

```
┌─────────────┐
│     End     │
└─────────────┘
```

# FIG. 2B

START

250

252

Obtain cytometry data for a biological sample previously-obtained from a subject, the biological sample comprising a plurality of cells, the cytometry data including cytometry measurements obtained during respective cytometry events, the cytometry events including a subset of events corresponding to cells in the biological sample being measured by the cytometry platform.

254

Identify types of cells in the plurality of cells using multiple machine learning models to obtain a respective plurality of cell types, the identifying comprising, for each particular event in the subset of events:

254-1

Obtain, from the cytometry data, cytometry measurements corresponding to the particular event.

254-2

Determine an event type for the particular event by processing the cytometry measurements using a first machine learning model, the event type indicating whether the particular event corresponds to a cell, debris, or a bead being measured by the cytometry platform.

254-3

When the determined event type indicates that the particular event corresponds to the cell being measured by the cytometry platform, determine a type for the cell by processing the cytometry measurements using a second machine learning model.

254-4

Another cytometry event in the subset of cytometry events?

YES

NO

END

FIG. 2C

FIG. 3A

EP 4 473 288 B1

350

352 — Event is of Type A

354 — Machine Learning Model

356 — Is event of Type A1 or Type A2?

358 — Type A1
Predicted Probability > Threshold & Predicted Probability of Type A2

360 — Type A2
Predicted Probability > Threshold & Predicted Probability of Type A1

362 — Neither
Predicted Probabilities < Threshold

FIG. 3B

FIG. 4

500

502

Obtain cytometry data for a biological sample from a subject, the biological sample comprising a plurality of cells.

504

Determine a respective type for each of at least some of the plurality of cells

506

Determine a respective cell composition percentage for each of at least some of the determined types.

508

Normalize the cell composition percentages with respect to hierarchical relationships between the cell types.

510

Identify the subject as a member of a patient cohort based on the determined cell composition percentages.

512

Identify a treatment for the subject based on the determined cell composition percentages

514

Administer, to the subject, the identified treatment.

FIG. 5A

FIG. 5B

506

542

Determine a number of beads included in a subsample of the biological sample.

544

Estimate a cell composition percentage for each of at least some of the cell types included in the subsample based on the number of beads.

544a

Determine a number of cells of a first type.

544b

Normalize the number of cells of the first type with respect to the number of beads.

544c

Estimate the cell composition percentage for the first cell type.

546

Another subsample?

548

Determine a single cell composition per cell type.

FIG. 5C

508

START

552

Identify sets of one or more subtypes of a first cell type for which one or more cell composition percentages have been estimated.

554

For a first set of the identified sets, determine a sum of the one or more cell composition percentages estimated for subtypes in the first set.

556

Sum > cell composition percentage of the first cell type?

No

Yes

558

Additional subtypes of the first cell type?

No

Yes

560

Determine a normalization coefficient.

562

Apply the normalization coefficient to the one or more cell composition percentages in the first set.

564

Another set?

Yes

No

566

Another cell type?

Yes

No

END

FIG. 5D

602

**Cytometry Data**

| Cell # | CD3 | CD62L | Type |
|--------|------|-------|------|
| 1 | 1.58 | 5.43 | C |
| 2 | 1.56 | 1.49 | B |
| 3 | 0.87 | -1.42 | A |
| 4 | 0.45 | 4.54 | B |
| 5 | 0.65 | 1.66 | A |

604

Cell Type A%   Cell Type B%   Cell Type C%

# FIG. 6A

**PANEL A** 606

| Cell # | Type |
| --- | --- |
| 1 | A |
| 2 | B |
| 3 | B |
| 4 | A |
| 5 | E |

**PANEL B** 610

| Cell # | Type |
| --- | --- |
| 1 | D |
| 2 | A |
| 3 | E |
| 4 | A |
| 5 | A |

608
Cell Type A1%  Cell Type B1%  Cell Type E1%

612
Cell Type A2%  Cell Type D2%  Cell Type E2%

614
Cell Type A%  Cell Type B%  Cell Type C%  Cell Type D%  Cell Type E%

FIG. 6B

FIG. 6C

FIG. 7A

FIG. 7B

FIG. 7C

FIG. 7D

| Cellular Family | Population name | Patient sample, percent | Reference range, percent |
|---|---|---|---|
| Granulocytes | Neutrophils | 71.5 | 54.3 - 77.1 |
| Granulocytes | Basophils | 0.50 | 0.30 - 1.59 |
| Granulocytes | Eosinophils | 1.74 | 1.82 - 6.43 |
| Monocytes | CD16- (Classical) Monocytes | 4.99 | 0.28 - 6.91 |
| Monocytes | CD16+ (Non-classical) Monocytes | 0.52 | 0.064 - 1.74 |
| Dendritic Cells | CD141+ CLEC9A+ DC1 | 0.023 | 0.011 - 0.052 |
| Dendritic Cells | CD1c+ FceRI+ DC2 | 0.18 | 0.13 - 0.60 |
| Dendritic Cells | CD123+ plasmacytoid DC | 0.029 | 0.033 - 0.27 |
| Natural Killer Cells | CD56+ CD16- NK cells | 0.016 | 0 - 1.32 |
| Natural Killer Cells | CD56+ CD16+ NK cells | 2.82 | 0 - 3.63 |

FIG. 7E

Summary: changes blood cell types MAIT_cells

| MAIT cells | x 2.2 |
| MAIT CD8+ | x 2.4 |
| MAIT CD8+ CD27+ | x 2.3 |
| MAIT CD8+ CD27+ CD56+ CD57+ | x 11.7 |
| MAIT CD8+ CD27+ CD45RA- CD56+ CD57+ | x 11.7 |
| MAIT CD8+ CD27+ CD56- CD57- | x 1.4 |
| MAIT CD8+ CD27+ CD45RA- CD56- CD57- | x 1.4 |
| MAIT CD8+ CD27+ CD45RA- CD56+ CD57- | x 2.2 |
| MAIT CD8+ CD27- CD45RA- CD56+ CD57- | x 3.2 |
| MAIT CD8- | x 1.2 |
| MAIT CD8- CD27+ | x 1.1 |
| MAIT CD8- CD27+ CD45RA- CD56+ CD57- | x 1.8 |

FIG. 7F

Summary: changes blood cell types CD4_T_helpers

| CD4 T helpers | |
| CD4 Central Memory CXCR3- CCR4- CCR6- | x 1.1 |
| CD4 Central Memory CCR4- CCR6- CXCR3- CXCR5- | x 1.5 |
| CD4 Transitional Memory CCR4+ CCR6- CXCR3- CXCR5- | x 2.0 |
| CD4 Effector Memory CCR4- CCR6- CXCR3- CXCR5- | x 1.4 |

FIG. 7G

EP 4 473 288 B1

800

START

802

Obtain cytometry training data for each of a plurality of cells

804

Obtain corresponding cell type data for each of at least some of the plurality of cells.

804a

Obtain a first type for a first cell for which cytometry data was obtained.

804b

Extract, from a hierarchy of cell types, one or more cell types related to the first type.

806

Using the cytometry data and the cell type data, train a machine learning model of a plurality of machine learning models to determine whether a cell is of a particular type.

806a

Using first cytometry data and first cell type data, train a first machine learning model to determine whether the cell is of a first type

806b

Another machine learning model?          Yes

No

END

# FIG. 8

FIG. 9A

FIG. 9A CONT.

FIG. 9B

FIG. 9B CONT.

FIG. 9C

FIG. 9D

FIG. 9C CONT.

FIG. 9D CONT.

WBC_KP62174_CP10_F0624.fcs Viability

FIG. 9E

WBC_KP62174_CP10_F0624 = 600967
Number of cells per million WBC

FIG. 9F

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63304990
- WO 2021247868 A1
- WO 2020081582 A1
- WO 0053211 A
- US 5981568 A
- WO 9007936 A
- WO 9403622 A
- WO 9325698 A
- WO 9325234 A
- WO 9311230 A
- WO 9310218 A
- WO 9102805 A
- US 5219740 A
- US 4777127 A
- GB 2200651 A
- EP 0345242 A
- WO 9412649 A
- WO 9303769 A
- WO 9319191 A
- WO 9428938 A
- WO 9511984 A
- WO 9500655 A
- US 5814482 A
- WO 9507994 A
- WO 9617072 A
- WO 9530763 A
- WO 9742338 A
- WO 9011092 A
- US 5580859 A
- US 5422120 A
- WO 9513796 A
- WO 9423697 A
- WO 9114445 A
- EP 0524968 A

**Non-patent literature cited in the description**

- **M. HUBERT** ; **E. VANDERVIEREN**. An adjusted boxplot for skewed distributions. *Computational Statistics & Data Analysis*, 2008, vol. 52 (12), 5186-5201
- **FLOWJO**. FlowJo™ Software. Ashland, OR: Beckton. Dickinson and Company, 2021
- **VAN GASSEN et al.** FlowSOM: Using self-organizing maps for visualization and interpretation of cytometry data. *Journal of Quantitative Cell Science*, 2015, vol. 87 (7), 636-645
- **QUINLAN, J. R**. *Induction of Decision Trees. Mach. Learn*, March 2018, vol. 1 (1), 81-106
- **QUINLAN, J. R**. C4.5: Programs for Machine Learning. Morgan Kaufmann Publishers, 1993
- **BREIMAN** ; **LEO** ; **FRIEDMAN, J. H.** ; **OLSHEN, R. A.** ; **STONE, C. J**. Classification and regression trees. *Monterey, CA: Wadsworth & Brooks/Cole Advanced Books & Software*, 1984
- 10. Boosting and Additive Trees. **HASTIE, T.** ; **TIBSHIRANI, R.** ; **FRIEDMAN, J. H**. The Elements of Statistical Learning. Springer, 2009, 337-384
- **CHEN, T.** ; **GUESTRIN, C**. XGBoost: A Scalable Tree Boosting System. *In Proceedings of the 22nd ACM SIGKDD International Conference on Knowledge Discovery and Data Mining*, 2016, 785-794
- **KE, G.** ; **MENG, Q** ; **FINLEY, T** ; **WANG, T** ; **CHEN, W** ; **MA, W** ; **LIU, T.-Y**. Lightgbm: A highly efficient gradient boosting decision tree. *Advances in Neural Information Processing Systems*, 2017, vol. 30, 3146-3154
- **KINGMA, D** ; **BA, J**. Adam: A Method for Stochastic Optimization. *Proceedings of the 3rd International Conference on Learning Representations (ICLR 2015)*, 2015
- **VAUGHT et al.** *Cancer Epidemiol Biomarkers Prev*, February 2012, vol. 21 (2), 253-5
- **VAUGHT** ; **HENDERSON**. Biological sample collection, processing, storage and information management. *IARC Sci Publ*, 2011 (163), 23-42
- **LI et al.** *JCO Precis Oncol*, 2018, vol. 2
- Clinical Flow Cytometry. Annals of the New York Academy of Sciences, 1993, vol. 677
- Clinical Flow Cytometry: Principles and Applications. Williams & Wilkins, 1993
- Flow Cytometry: A Practical Approach. Oxford Univ. Press, 1997
- Flow Cytometry Protocols, Methods in Molecular Biology. Humana Press, 1997
- Practical Shapiro, Flow Cytometry. Wiley-Liss, 2003
- Handbook of Biological Confocal Microscopy. Plenum Press, 1989

- **BENDALL et al.** A deep profiler's guide to cytometry. *Trends in Immunology*, 2012, vol. 33 (7), 323-332
- **SPITZER et al.** Mass Cytometry: Single Cells, Many Features. *Cell*, 2016, vol. 165 (4), 780-791
- **FINDEIS et al.** *Trends Biotechnol*, 1993, vol. 11, 202
- **CHIOU et al.** Gene Therapeutics: Methods and Applications of Direct Gene Transfer. 1994
- **WU et al.** *J. Biol. Chem.*, 1988, vol. 263, 621
- **WU et al.** *J. Biol. Chem.*, 1994, vol. 269, 542
- **ZENKE et al.** *Proc. Natl. Acad. Sci. USA*, 1990, vol. 87, 3655
- **WU et al.** *J. Biol. Chem.*, 1991, vol. 266, 338
- **JOLLY.** *Cancer Gene Therapy*, 1994, vol. 1, 51
- **KIMURA.** *Human Gene Therapy*, 1994, vol. 5, 845
- **CONNELLY.** *Human Gene Therapy*, 1995, vol. 1, 185
- **KAPLITT.** *Nature Genetics*, 1994, vol. 6, 148
- **CURIEL.** *Hum. Gene Ther*, 1992, vol. 3, 147
- **WU.** *J. Biol. Chem.*, 1989, vol. 264, 16985
- **PHILIP.** *Mol. Cell. Biol.*, 1994, vol. 14, 2411
- **WOFFENDIN.** *Proc. Natl. Acad. Sci.*, 1994, vol. 91, 1581